# EUROPEAN PATENT SPECIFICATION

(11) **EP 2 064 316 B1**
(45) Date of publication and mention of the grant of the patent: **25.01.2012**
(21) Application number: 07837970.8
(22) Date of filing: 07.09.2007
(51) Int. Cl.: C12N 5/02

(54) **SITE SPECIFIC INCORPORATION OF NON-NATURAL AMINO ACIDS BY VERTEBRATE CELLS**
STANDORTSPEZIFISCHE INKORPORATION NICHT NATÜRLICHER AMINOSÄUREN IN WIRBELTIERZELLEN
INTEGRATION SITE-SPECIFIQUE D'ACIDES AMINES NON NATURELS DANS DES CELLULES DE VERTEBRES

(30) Priority: 08.09.2006 US 843473 P
(43) Date of publication of application: 03.06.2009
(73) Proprietor: Ambrx, Inc., La Jolla, CA 92037 (US)
(72) Inventor: TIAN, Feng, San Diego, CA 92129 (US); NORMAN, Thea, San Diego, CA 92102 (US); CHU, Stephanie, San Diego, CA 92122 (US)
(74) Representative: Mercer, Christopher Paul
(86) International application number: PCT/US2007/019654
(87) International publication number: WO 2008/030612

(56) References cited:
- EP-A- 1 557 469
- WO-A-2006/068802
- WO-A2-2005/003294
- US-B2- 7 045 337
- CHIN J W ET AL: "An Expanded Eukaryotic Genetic Code" SCIENCE, AMERICAN ASSOCIATION FOR THE ADVANCEMENT OF SCIENCE, US, WASHINGTON, DC, vol. 301, 15 August 2003 (2003-08-15), pages 964-967, XP002996001 ISSN: 0036-8075
- WANG L ET AL: "Addition of the keto functional group to the genetic code of Escherichia coli" PROCEEDINGS OF THE NATIONAL ACADEMY OF SCIENCES OF USA, NATIONAL ACADEMY OF SCIENCE, WASHINGTON, DC., US, vol. 100, no. 1, 7 January 2003 (2003-01-07), pages 56-61, XP003019022 ISSN: 0027-8424
- YE SHIXIN ET AL: "Site-specific incorporation of keto amino acids into functional G protein-coupled receptors using unnatural amino acid mutagenesis" JOURNAL OF BIOLOGICAL CHEMISTRY, vol. 283, no. 3, January 2008 (2008-01), pages 1525-1533, XP002541446 ISSN: 0021-9258

## Description

### FIELD OF THE INVENTION

The invention pertains to the field of translation biochemistry in vertebrate cells. The invention relates to methods for producing and compositions of orthogonal tRNA's, orthogonal synthetases and pairs thereof, in vertebrate cells. The invention also relates to compositions of unnatural amino acids, proteins and methods of producing proteins in vertebrate cells that include unnatural amino acids.

### BACKGROUND OF THE INVENTION

The genetic code of every known organism, from bacteria to humans, encodes the same twenty common amino acids. Different combinations of the same twenty natural amino acids form proteins that carry out virtually all the complex processes of life, from photosynthesis to signal transduction and the immune response. In order to study and modify protein structure and function, scientists have attempted to manipulate both the genetic code and the amino acid sequence of proteins. However, it has been difficult to remove the constraints imposed by the genetic code that limit proteins to twenty genetically encoded standard building blocks (with the rare exception of selenocysteine *(see, e.g.,* A. Bock et al., (1991), Molecular Microbiology 5:515-20) and pyrrolysine *(see, e.g.,* G. Srinivasan, et al., (2002), Science 296:1459-62).

Some progress has been made to remove these constraints, although this progress has been limited and the ability to rationally control protein structure and function is still in its infancy. For example, chemists have developed methods and strategies to synthesize and manipulate the structures of small molecules (*see, e.g.,* E. J. Corey, & X.-M. Cheng, The Logic of Chemical Synthesis (Wiley-Interscience, New York, 1995)). Total synthesis *(see, e.g.,* B. Merrifield, (1986), Science 232:341-7 (1986)), and semi-synthetic methodologies *(see, e.g.,* D. Y. Jackson et al., (1994) Science 266:243-7; and, P. E. Dawson, & S. B. Kent, (2000), Annual Review of Biochemistry 69:923-60), have made it possible to synthesize peptides and small proteins, but these methodologies have limited utility with proteins over 10 kilo Daltons (kDa). Mutagenesis methods, though powerful, are restricted to a limited number of structural changes. In a number of cases, it has been possible to competitively incorporate close structural analogues of common amino acids throughout proteins. *See, e.g.,* R. Furter, (1998). Protein Science 7:419-26; K. Kirshenbaum, et al., (2002), ChemBioChem 3:235-7; and, V. Doring et al., (2001), Science 292:501-4.

In an attempt to expand the ability to manipulate protein structure and function, *in vitro* methods using chemically acylated orthogonal tRNA's were developed that allowed unnatural amino acids to be selectively incorporated in response to a nonsense codon, *in vitro (see, e.g.,* J. A. Ellman, et al., (1992), Science 255:197-200). Amino acids with novel structures and physical properties were selectively incorporated into proteins to study protein folding and stability and biomolecular recognition and catalysis. *See, e.g.,* D. Mendel, et al., (1995), Annual Review of Biophysics and Biomolecular Structure 24:435-462; and, V. W. Cornish, et al. (Mar. 31, 1995), Angewandte Chemie-international Edition in English 34:621-633. However, the stoichiometric nature of this process severely limited the amount of protein that could be generated.

Unnatural amino acids have been microinjected into cells. For example, unnatural amino acids were introduced into the nicotinic acetylcholine receptor in Xenopus oocytes (e.g., M.W. Nowak, et al. (1998), In vivo incorporation of unnatural amino acids into ion channels in Xenopus oocyte expression system, Method Enzymol. 293:504-529) by microinjection of a chemically misacylated Tetrahymena thermophila tRNA (e.g., M.E. Saks, et al. (1996), An engineered Tetrahymena tRNAGln for in vivo incorporation of unnatural amino acids into proteins by nonsense suppression, J. Biol. Chem. 271:23169-23175), and the relevant mRNA. This has allowed detailed biophysical studies of the receptor in oocytes by the introduction of amino acids containing side chains with unique physical or chemical properties. *See, e.g.,* D.A. Dougherty (2000), Unnatural amino acids as probes of protein structure and function, Curr. Opin. Chem. Biol. 4:645-652. Unfortunately, this methodology is limited to proteins in cells that can be microinjected, and because the relevant tRNA is chemically acylated in vitro, and cannot be re-acylated, the yields of protein are very low.

To overcome these limitations, new components were added to the protein biosynthetic machinery of the prokaryote *Escherichia coli* (*E. coli*) (e.g., L. Wang, et al., (2001), Science 292:498-500), which allowed genetic encoding of unnatural amino acids in *vivo.* A number of new amino acids with novel chemical, physical or biological properties, including photoaffinity labels and photoisomerizable amino acids, keto amino acids, and glycosylated amino acids have been incorporated efficiently and with high fidelity into proteins in *E*. *coli* in response to the amber codon, TAG, using this methodology. *See, e.g.,* J. W. Chin et al., (2002), Journal of the American Chemical Society 124:9026-9027; J. W. Chin, & P. G. Schultz, (2002), ChemBioChem 11:1135-1137; J. W. Chin, et al., (2002), PNAS United States of America 99:11020-11024: and, L. Wang, & P. G. Schultz, (2002), Chem. Comm., 1-10. However, the translational machinery of prokaryotes and eukaryotes are not highly conserved; thus, components of the biosynthetic machinery added to *E.coli* cannot often be used to site-specifically incorporate unnatural amino acids into proteins in vertebrate cells. For example, the *Methanococcus jannaschii* tyrosyl-tRNA synthetase/tRNA pair that was used in *E.coli* is not orthogonal in vertebrate cells. In addition, the transcription oftRNA in eukaryotes, but not in prokaryotes, is carried out by RNA Polymerase III and this places restrictions on the primary sequence of the tRNA structural genes that can be transcribed in vertebrate cells. Moreover, in contrast to prokaryotic cells, tRNA's in vertebrate cells need to be exported from the nucleus, where they are transcribed, to the cytoplasm, to function in translation. Finally, the vertebrate 80S ribosome is distinct from the 70S prokaryotic ribosome.

WO2005003294 and EP1557469 describe compositions and methods for producing translational components that expand the number of genetically encoded amino acids in eukaryotic cells. The components include orthogonal tRNAs, orthogonal aminoacyl-tRNA synthetases, pairs of tRNAs/synthetases and unnatural amino acids. Proteins and methods of producing proteins with unnatural amino acids in eukaryotic cells are also described.

However, there is a need to develop improved components of the biosynthetic machinery to expand the vertebrate genetic code. This invention fulfills these and other needs, as will be apparent upon review of the following disclosure.

### SUMMARY OF THE INVENTION

The invention provides a vertebrate cell or cell line comprising an orthogonal aminoacyl-tRNA synthetase (O-RS), wherein the O-RS preferentially aminoacylates an orthogonal tRNA (O-tRNA) with para-acetyl-phenylalanine (PAF) in the vertebrate cell and wherein the O-tRNA comprises the nucleotide sequence as set forth in SEQ ID NO: 88 and wherein the O-RS comprises an amino acid sequence as set forth in SEQ ID NO: 45.

Compositions of the invention include a vertebrate cell (e.g., a mammalian cell, an avian cell, a fish cell, a reptile cell, an amphibian cell, cells derived from non-mammalian animals, etc.) comprising an orthogonal aminoacyl-tRNA synthetase (O-RS) (e.g., derived from a non-vertebrate organism, such as *Escherichia coli, Bacillus stearothermophilus,* etc.), where the O-RS preferentially aminoacylates an orthogonal tRNA (O-tRNA) with at least one unnatural amino acid in the vertebrate cell. Optionally, two or more OtRNA's can be aminoacylated in a given vertebrate cell. In one embodiment, an O-RS of the invention aminoacylates the O-tRNA with the unnatural amino acid, e.g., at least 10-fold, at least 20-fold, at least 30-fold, etc., more efficiently than the O-RS aminoacylates the O-tRNA with a natural amino acid.

In one embodiment, the O-RS or a portion thereof is encoded by a polynucleotide sequence as set forth in any one of SEQ ID NO.: 3-35, or a complementary polynucleotide sequence thereof. In another embodiment, the O-RS comprises an amino acid sequence as set forth in any one of SEQ ID NO.: 36-63, and/or 86, or a conservative variation thereof. In yet another embodiment, the O-RS comprises an amino acid sequence that is, e.g., at least 90%, at least 95%, at least 98%, at least 99%, or at least 99.5 % or more, identical to that of a naturally occurring tyrosyl aminoacyl-tRNA synthetase (TyrRS) and comprises two or more amino acids from groups A-E. Group A includes valine, isoleucine, leucine, glycine, serine, alanine, or threonine at a position corresponding to Tyr37 of an *E. coli* TyrRS. Group B includes aspartate at a position corresponding to Asn126 of an *E. coli* TyrRS. Group C includes threonine, serine, arginine, asparagine or glycine at a position corresponding to Asp182 of an *E. coli* TyrRS. Group D includes methionine, alanine, valine, or tyrosine at a position corresponding to Phe183 of an *E. coli* TyrRS; and, group E includes serine, methionine, valine, cysteine, threonine, or alanine at a position corresponding to Leu 186 of an *E*. *coli* TyrRS.

In another embodiment, the O-RS has one or more improved or enhanced enzymatic properties for the unnatural amino acid as compared to a natural amino acid. For example, the improved or enhanced properties for the unnatural amino acid as compared to a natural amino acid include any of, e.g., a higher Km, a lower Km, a higher kcat, a lower kcat, a lower kcat/km, a higher kcat/km, etc.

In another embodiment, the vertebrate cell comprises a nucleic acid that comprises a polynucleotide that encodes a polypeptide of interest, where the polynucleotide comprises a selector codon that is recognized by the O-tRNA.

In certain embodiments, the cells further comprise a polynudeotide that comprises at least one selector codon. In one embodiment, the selector codon is an amber stop codon, e.g., where the amber stop codon is located in or substantially near a portion of the polynucleotide that encodes a DNA binding domain of the transcriptional activator protein.

Examples of a protein (or polypeptide of interest) include, but are not limited to, e.g., a cytokine, a growth factor, a growth factor receptor, an interferon, an interleukin, an inflammatory molecule, an oncogene product, a peptide hormone, a signal transduction molecule, a steroid hormone receptor, erythropoietin (EPO), insulin, human growth hormone, an Alpha-1 antitrypsin, an Angiostatin, an Antihemolytic factor, an antibody, an Apolipoprotein, an Apoprotein, an Atrial natriuretic factor, an Atrial natriuretic polypeptide, an Atrial peptide, a C-X-C chemokine, T39765, NAP-2, ENA-78, a Gro-a, a Gro-b, a Gro-c, an IP-10, a GCP-2, an NAP-4, an SDF-1, a PF4, a MIG, a Calcitonin, a c-kit ligand, a cytokine, a CC chemokine, a Monocyte chemoattractant protein-1, a Monocyte chemoattractant protein-2, a Monocyte chemoattractant protein-3, a Monocyte inflammatory protein-1 alpha, a Monocyte inflammatory protein-1 beta, RANTES, I309, R83915, R91733, HCC1, T58847, D31065, T64262, a CD40, a CD40 ligand, a C-kit Ligand, a Collagen, a Colony stimulating factor (CSF), a Complement factor 5a, a Complement inhibitor, a Complement receptor 1, a cytokine, DHFR, an epithelial Neutrophil Activating Peptide-78, a GROα/MGSA, a CROβ, a GROy a MIP-1α, a MIP-1δ, a MCP-1, an Epidermal Growth Factor (EGF), an epithelial Neutrophil Activating Peptide, an Erythropoietin (EPO), an Exfoliating toxin, a Factor IX, a Factor VII, a Factor VIII, a Factor X, a Fibroblast Growth Factor (FGF), a Fibrinogen, a Fibronectin, a G-CSF, a GM-CSF, a Glucocerebrosidase, a Gonadotropin, a growth factor, a growth factor receptor, a Hedgehog protein, a Hemoglobin, a Hepatocyte Growth Factor (HGF), a Hirudin, a Human serum albumin, an ICAM-1, an ICAM-1 receptor, an LFA-1, an LFA-1 receptor, an Insulin, an Insulin-like Growth Factor (IGF), an IGF-I, an IGF-II, an interferon, an IFN-α, an IFN-β, an IFN-γ, an interleukin, an IL-1, an IL-2, an IL-3, an IL-4, an IL-5, an IL-6, an IL-7, an IL-8, an IL-9, an IL-10, an IL-11, an IL-12, a Keratinocyte Growth Factor (KGF), a Lactoferrin, a leukemia inhibitory factor, a Luciferase, a Neurturin, a Neutrophil inhibitory factor (NIF), an oncostatin M, an Osteogenic protein, an oncogene product, a Parathyroid hormone, a PD-ECSF, a PDGF, a peptide hormone, a Human Growth Hormone, a Pleiotropin, a Protein A, a Protein G, a Pyrogenic exotoxins A, B, or C, a Relaxin, a Renin, an SCF, a Soluble complement receptor I, a Soluble I-CAM 1, a Soluble interleukin receptors, a Soluble TNF receptor, a Somatomedin, a Somatostatin, a Somatotropin, a Streptokinase, a Superantigens, a Staphylococcal enterotoxins, an SEA, an SEB, an SEC1, an SEC2, an SEC3, an SED, an SEE, a steroid hormone receptor, a Superoxide dismutase (SOD), a Toxic shock syndrome toxin, a Thymosin alpha 1, a Tissue plasminogen activator, a tumor growth factor (TGF), a TGF-α, a TGF-β, a Tumor Necrosis Factor, a Tumor Necrosis Factor alpha, a Tumor necrosis factor beta, a Tumor necrosis factor receptor (TNFR), a VLA-4 protein, a VCAM-1 protein, a Vascular Endothelial Growth Factor (VEGEF), a Urokinase, a Mos, a Ras, a Raf, a Met; a p53, a Tat, a Fos, a Myc, a Jun, a Myb, a Rel, an estrogen receptor, a progesterone receptor, a testosterone receptor, an aldosterone receptor, an LDL receptor, a SCF/c-Kit, a CD40L/CD40, a VLA-4/VCAM-1, an ICAM-1/LFA-1, a hyalurin/CD44, a corticosterone.

In certain embodiments, the encoded protein comprises a therapeutic protein, a diagnostic protein, an industrial enzyme, or portion thereof.

In certain embodiments, the compositions and the methods of the invention include vertebrate cells. A vertebrate cell of the invention includes any of, e.g., a mammalian cell, a yeast cell, a fungus cell, a plant cell, an insect cell, etc. The translation components of the invention can be derived from a variety of organisms, e.g., non-vertebrate organisms, such as a prokaryotic organism (e.g., *E. coli, Bacillus stearothermophilus,* or the like), or an archaebacterium, or e.g., a vertebrate organism.

A selector codon of the invention expands the genetic codon framework of vertebrate protein biosynthetic machinery. Any of a variety of selector codons can be used in the invention, including stop codons (e.g., an amber codon, an ochre codon, or an opal stop codon), nonsense codons, rare codons, four (or more) base codons, and/or the like.

Examples of unnatural amino acids that can be used in the compositions and methods described herein include (but are not limited to): a *p*-acetyl-L-phenylalanine, a *p-*iodo-L-phenylalanine, an O-methyl-L-tyrosine, a *p*-propargyloxyphenylalanine, a *p-*propargyl-phenylalanine, an L-3-(2-naphthyl)alanine, a 3-methyl-phenylalanine, an O-4-allyl-L-tyrosine, a 4-propyl-L-tyrosine, a tri-O-acetyl-GlcNAcβ-serine, an L-Dopa, a fluorinated phenylalanine, an isopropyl-L-phenylalanine, a p-azido-L-phenylalanine, ap-acyl-L-phenylalanine, a *p*-benzoyl-L-phenylalanine, an L-phosphoserine, a phosphonoserine, a phosphonotyrosine, a *p*-bromophenylalanine, a *p*-amino-L-phenylalanine, an isopropyl-L-phenylalanine, an unnatural analogue of a tyrosine amino acid; an unnatural analogue of a glutamine amino acid; an unnatural analogue of a phenylalanine amino acid; an unnatural analogue of a serine amino acid; an unnatural analogue of a threonine amino acid; an alkyl, aryl, acyl, azido, cyano, halo, hydrazine, hydrazide, hydroxyl, alkenyl, alkynl, ether, thiol, sulfonyl, seleno, ester, thioacid, borate, boronate, phospho, phosphono, phosphine, heterocyclic, enone, imine, aldehyde, hydroxylamine, keto, or amino substituted amino acid, or any combination thereof; an amino acid with a photoactivatable cross-linker; a spin-labeled amino acid; a fluorescent amino acid; a metal binding amino acid; a metal-containing amino acid; a radioactive amino acid; a photocaged and/or photoisomerizable amino acid; a biotin or biotin-analogue containing amino acid; a keto containing amino acid; an amino acid comprising polyethylene glycol or polyether; a heavy atom substituted amino acid; a chemically cleavable or photocleavable amino acid; an amino acid with an elongated side chain; an amino acid containing a toxic group; a sugar substituted amino acid; a carbon-linked sugar-containing amino acid; a redox-active amino acid; an α-hydroxy containing acid; an amino thio acid; an a,a disubstituted amino acid; a β-amino acid; a cyclic amino acid other than proline or histidine, an aromatic amino acid other than phenylalanine, tyrosine or tryptophan, and/or the like.

Kits are also a feature of the invention. For example, a kit for producing a protein that comprises at least one unnatural amino acid in a cell is provided, where the kit includes a container containing a polynucleotide sequence encoding an O-tRNA or an O-tRNA, and a polynucleotide sequence encoding an O-RS or an O-RS. In one embodiment, the kit further includes at least one unnatural amino acid. In another embodiment, the kit further comprises instructional materials for producing the protein.

BRIEF DESCRIPTION OF THE DRAWINGS

Figure 1 shows incorporation of para-acetyl-phenylalanine into hGH.

Figure 2 shows incorporation of para-acetyl-phenylalanine at various concentrations into hGH.

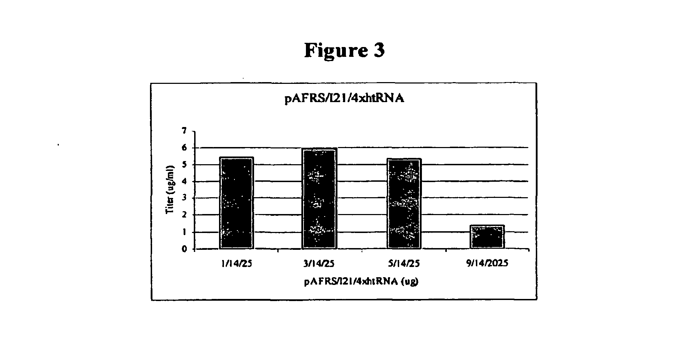

Figure 3: A bar graph of the results from Example 3 showing the Fc titer in micrograms per mL along the Y-axis from CHO cells with different ratios of pAFRS/I21/4xhtRNA shown along the x-axis.

DETAILED DESCRIPTION

Before describing the present invention in detail, it is to be understood that this invention is not limited to particular devices or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a cell" includes a combination of two or more cells; reference to "bacteria" includes mixtures of bacteria, and the like.

Unless otherwise defined herein or below in the remainder of the specification, all technical and scientific terms used herein have the same meaning as commonly understood by those of ordinary skill in the art to which the invention belongs.

Homologous: Proteins and/or protein sequences are "homologous" when they are derived, naturally or artificially, from a common ancestral protein or protein sequence. Similarly, nucleic acids and/or nucleic acid sequences are homologous when they are derived, naturally or artificially, from a common ancestral nucleic acid or nucleic acid sequence. For example, any naturally occurring nucleic acid can be modified by any available mutagenesis method to include one or more selector codon. When expressed, this mutagenized nucleic acid encodes a polypeptide comprising one or more unnatural amino acid. The mutation process can, of course, additionally alter one or more standard codon, thereby changing one or more standard amino acid in the resulting mutant protein, as well. Homology is generally inferred from sequence similarity between two or more nucleic acids or proteins (or sequences thereof). The precise percentage of similarity between sequences that is useful in establishing homology varies with the nucleic acid and protein at issue, but as little as 25% sequence similarity is routinely used to establish homology. Higher levels of sequence similarity, e.g., 30%, 40%, 50%, 60%, 70%, 80%, 90%, 95%, or 99% or more, can also be used to establish homology. Methods for determining sequence similarity percentages (e.g., BLASTP and BLASTN using default parameters) are described herein and are generally available.

Orthogonal: As used herein, the term "orthogonal" refers to a molecule (e.g., an orthogonal tRNA (O-tRNA) and/or an orthogonal aminoacyl tRNA synthetase (O-RS)) that functions with endogenous components of a cell with reduced efficiency as compared to a corresponding molecule that is endogenous to the cell or translation system, or that fails to function with endogenous components of the cell. In the context of tRNA's and aminoacyl-tRNA synthetases, orthogonal refers to an inability or reduced efficiency, e.g., less than 20 % efficient, less than 10 % efficient, less than 5 % efficient, or less than 1% efficient, of an orthogonal tRNA to function with an endogenous tRNA synthetase compared to an endogenous tRNA to function with the endogenous tRNA synthetase, or of an orthogonal aminoacyl-tRNA synthetase to function with an endogenous tRNA compared to an endogenous tRNA synthetase to function with the endogenous tRNA. The orthogonal molecule lacks a functional endogenous complementary molecule in the cell. For example, an orthogonal tRNA in a cell is aminoacylated by any endogenous RS of the cell with reduced or even zero efficiency, when compared to aminoacylation of an endogenous tRNA by the endogenous RS. In another example, an orthogonal RS aminoacylates any endogenous tRNA in a cell of interest with reduced or even zero efficiency, as compared to aminoacylation of the endogenous tRNA by an endogenous RS. A second orthogonal molecule can be introduced into the cell that functions with the first orthogonal molecule. For example, an orthogonal tRNA/RS pair includes introduced complementary components that function together in the cell with an efficiency (e.g., 50% efficiency, 60% efficiency, 70% efficiency, 75% efficiency, 80% efficiency, 90% efficiency, 95% efficiency, or 99% or more efficiency) to that of a corresponding tRNA/RS endogenous pair.

Complementary: The term "complementary" refers to components of an orthogonal pair, O-tRNA and O-RS that can function together, e.g., where the O-RS aminoacylates the O-tRNA.

Preferentially aminoacylates: The term "preferentially aminoacylates" refers to an efficiency, e.g., 70 % efficient, 75 % efficient, 85% efficient, 90% efficient, 95 % efficient, or 99% or more efficient, at which an O-RS aminoacylates an O-tRNA with an unnatural amino acid as compared to the O-RS aminoacylating a naturally occurring tRNA or a starting material used to generate the O-tRNA. The unnatural amino acid is incorporated into a growing polypeptide chain with high fidelity, e.g., at greater than 75% efficiency for a given selector codon, at greater than about 80% efficiency for a given selector codon, at greater than about 90% efficiency for a given selector codon, at greater than about 95% efficiency for a given selector codon, or at greater than about 99% or more efficiency for a given selector codon.

Selector codon: The term "selector codon" refers to codons recognized by the O-tRNA in the translation process and not recognized by an endogenous tRNA. The O-tRNA anticodon loop recognizes the selector codon on the mRNA and incorporates its amino acid, e.g., an unnatural amino acid, at this site in the polypeptide. Selector codons can include, e.g., nonsense codons, such as, stop codons, e.g., amber, ochre, and opal codons; four or more base codons; rare codons; codons derived from natural or unnatural base pairs and/or the like.

Suppressor tRNA: A suppressor tRNA is a tRNA that alters the reading of a messenger RNA (mRNA) in a given translation system, e.g., by providing a mechanism for incorporating an amino acid into a polypeptide chain in response to a selector codon. For example, a suppressor tRNA can read through, e.g., a stop codon, a four base codon, a rare codon, and/or the like.

Recyclable tRNA: The term "recyclable tRNA" refers to a tRNA that is aminoacylated and can be repeatedly reaminoacylated with an amino acid (e.g., an unnatural amino acid) for the incorporation of the amino acid (e.g., the unnatural amino acid) into one or more polypeptide chains during translation.

Translation system: The term "translation system" refers to the collective set of components that incorporate a naturally occurring amino acid into a growing polypeptide chain (protein). Components of a translation system can include, e.g., ribosomes, tRNA's, synthetases, mRNA, amino acids, and the like. The components of the invention (e.g., ORS, OtRNA's, unnatural amino acids, etc.) can be added to an in vitro or in vivo translation system, e.g., a vertebrate cell, e.g., a yeast cell, a mammalian cell, a plant cell, an algae cell, a fungus cell, an insect cell, and/or the like.

Unnatural amino acid: As used herein, the term "unnatural amino acid" refers to any amino acid, modified amino acid, and/or amino acid analogue that is not one of the 20 common naturally occurring amino acids, seleno cysteine or pyrrolysine.

Derived from: As used herein, the term "derived from" refers to a component that is isolated from or made using information from a specified molecule or organism.

Inactive RS: As used herein, the term "inactive RS" refers to a synthetase that has been mutated so that it no longer can aminoacylate its natural cognate tRNA with an amino acid.

Positive selection or screening marker: As used herein, the term "positive selection or screening marker" refers to a marker that when present, e.g., expressed, activated or the like, results in identification of a cell with the positive selection marker from those without the positive selection marker.

Negative selection or screening marker: As used herein, the term "negative selection or screening marker" refers to a marker that when present, e.g., expressed, activated or the like, allows identification of a cell that does not possess the desired property (e.g., as compared to a cell that does possess the desired property).

Reporter: As used herein, the term "reporter" refers to a component that can be used to select target components of a system of interest. For example, a reporter can include a fluorescent screening marker (e.g., green fluorescent protein), a luminescent marker (e.g., a firefly luciferase protein), an affinity based screening marker, or selectable marker genes such as his3, ura3, leu2, lys2, lacZ, β-gal/lacZ (β-galactosidase), Adh (alcohol dehydrogenase), or the like.

Vertebrate: As used herein, the term "vertebrate" refers to organisms belonging to the phylogenetic domain Eucarya such as animals e.g., mammals, reptiles, birds, etc.

Non-eukaryote: As used herein, the term "non-eukaryote" refers to non-vertebrate organisms. For example, a non-vertebrate organism can belong to the Eubacteria (e.g., *Escherichia coli, Thermus thermophilus, Bacillus stearothermophilus,* etc.) phylogenetic domain, or the Archaea (e.g., *Methanococcusjannaschii, Methanobacterium thermoautotrophicum, Halobacterium* such as *Haloferax vo*/*canii* and *Halobacterium* species *NRC-1, Archaeoglobus fulgidus, Pyrococcus furiosus, Pyrococcus horikoshii, Aeuropyrum pernix,* etc.) phylogenetic domain.

Antibody: The term "antibody," as used herein, includes, but is not limited to a polypeptide substantially encoded by an immunoglobulin gene or immunoglobulin genes, or fragments thereof, which specifically bind and recognize an analyte (antigen). Examples include polyclonal, monoclonal, chimeric, and single chain antibodies, and the like. Fragments of immunoglobulins, including Fab fragments and fragments produced by an expression library, including phage display, are also included in the term "antibody" as used herein. *See, e.g.,* Paul, Fundamental Immunology, 4th Ed., 1999, Raven Press, New York, for antibody structure and terminology.

Conservative variant: The term "conservative variant" refers to a translation component, e.g., a conservative variant O-tRNA or a conservative variant O-RS, that functionally performs like the component from which the conservative variant is based, e.g., an O-tRNA or O-RS, but has variations in the sequence. For example, an O-RS will aminoacylate a complementary O-tRNA or a conservative variant O-tRNA with an unnatural amino acid, although the O-tRNA and the conservative variant O-tRNA do not have the same sequence. The conservative variant can have, e.g., one variation, two variations, three variations, four variations, or five or more variations in sequence, as long as the conservative variant is complementary to the corresponding O-tRNA or O-RS.

Selection or screening_agent: As used herein, the term "selection or screening agent" refers to an agent that, when present, allows for a selection/screening of certain components from a population. For example, a selection or screening agent includes, but is not limited to, e.g., a nutrient, an antibiotic, a wavelength of light, an antibody, an expressed polynucleotide (e.g., a transcriptional modulator protein), or the like. The selection agent can be varied, e.g., by concentration, intensity, etc.

Detectable substance: The term "detectable substance," as used herein, refers to an agent that, when activated, altered, expressed or the like, allows for the selection/screening of certain components from a population. For example, the detectable substance can be a chemical agent, e.g., 5-fluroorotic acid (5-FOA), which under certain conditions, e.g., expression of a URA3 reporter, becomes detectable, e.g., a toxic product that kills cells that express the URA3 reporter.

The ability to genetically modify the structures of proteins directly in vertebrate cells, beyond the chemical constraints imposed by the genetic code, would provide a powerful molecular tool to both probe and manipulate cellular processes. The invention provides translational components that expand the number of genetically encoded amino acids in vertebrate cells. These include tRNA's (e.g., orthogonal tRNA's (O-tRNA's)), aminoacyl-tRNA synthetases (e.g., orthogonal synthetase (O-RS)), pairs of O-tRNA/O-RSs, and unnatural amino acids.

Typically, O-tRNA's of the invention are expressed and processed efficiently, and function in translation in a vertebrate cell, but are not significantly aminoacylated by the host's aminoacyl-tRNA synthetases. In response to a selector codon, an O-tRNA of the invention delivers an unnatural amino acid, which does not encode any of the common twenty amino acids, to a growing polypeptide chain during mRNA translation.

An O-RS of the invention preferentially aminoacylates an O-tRNA of the invention with an unnatural amino acid in a vertebrate cell, but does not aminoacylate any of the cytoplasmic host's tRNA's. Moreover, the specificity of an aminoacyl-tRNA synthetase of the invention provides acceptance of an unnatural amino acid while excluding any endogenous amino acids. Polypeptides that include amino acid sequences of example O-RSs, or portions thereof, are also a feature of the invention. In addition, polynucleotides that encode translational components, O-tRNA's, O-RSs and portions thereof, are features of the invention.

The disclosure further provides methods for producing a protein in a vertebrate cell, where the protein comprises an unnatural amino acid. The protein is produced using the translation components of the invention. The disclosure also provides proteins (and proteins produced by the methods of the invention), which include unnatural amino acids. The protein or polypeptide of interest can also include a post-translational modification, e.g., that is added through a [3+2] cycloaddition, or a nucleophilic-electrophilic reaction, that is not made by a prokaryotic cell, etc. In certain aspects methods of producing a transcriptional modulator protein with an unnatural amino acid (and proteins produced by such methods) are also included in the disclosure. Compositions, which include proteins that include an unnatural amino acid are also provided herein.

Kits for producing a protein or polypeptide with an unnatural amino acid are also a feature of the invention.

Orthogonal aminoacyl-TRNA synthetases (O-RS)

In order to specifically incorporate an unnatural amino acid in to a protein or polypeptide of interest, in a vertebrate cell, the substrate specificity of the synthetase is altered so that only the desired unnatural amino acid, but not any of the common 20 amino acids are charged to the tRNA. If the orthogonal synthetase is promiscuous, it will result in mutant proteins with a mixture of natural and unnatural amino acids at the target position.

A vertebrate cell that includes an orthogonal aminoacyl-tRNA synthetase (O-RS) is a feature of the invention. The O-RS preferentially aminoacylates an orthogonal tRNA (O-tRNA) with an unnatural amino acid in the vertebrate cell. In certain embodiments, the O-RS utilizes more than one unnatural amino acid, e.g., two or more, three or more, etc. Thus, an O-RS of the invention can have the capability to preferentially aminoacylate an O-tRNA with different unnatural amino acids. This allows an additional level of control by selecting which unnatural amino acid or combination of unnatural amino acids are put with the cell and/or by selecting the different amounts of unnatural amino acids that are put with the cell for their incorporation.

An O-RS of the invention optionally has one or more improved or enhanced enzymatic properties for the unnatural amino acid as compared to a natural amino acid. These properties include, e.g., higher Km, lower Km, higher kcat, lower kcat, lower kcat/km, higher kcat/km, etc., for the unnatural amino acid, as compared to a naturally occurring amino acid, e.g., one of the 20 known common amino acids.

Optionally, the O-RS can be provided to the vertebrate cell by a polypeptide that includes an O-RS and/or by a polynucleotide that encodes an O-RS or a portion thereof. For example, an O-RS, or a portion thereof, is encoded by a polynucleotide sequence as set forth in any one of SEQ ID NO.: 3-35, or a complementary polynucleotide sequence thereof. In another example, an O-RS comprises an amino acid sequence as set forth in any one of SEQ ID NO.: 36-63, and/or 86, or a conservative variation thereof. *See. e.g.,* Tables 5, 6 and 8, and Example 6 herein for sequences of exemplary O-RS molecules.

An O-RS can also comprise an amino acid sequence that is, e.g., at least 90%, at least 95%, at least 98%, at least 99%, or even at least 99.5 % identical to that of a naturally occurring tyrosyl aminoacyl-tRNA synthetase (TyrRS) (e.g., as set forth in SEQ ID NO.:2) and comprises two or more amino acids of group A-E. Group A includes valine, isoleucine, leucine, glycine, serine, alanine, or threonine at a position corresponding to Tyr37 of *E*. *coli* TyrRS; group B includes aspartate at a position corresponding to Asn126 of *E*. *coli* TyrRS; group C includes threonine, serine, arginine, asparagine or glycine at a position corresponding to Asp182 of *E*. *coli* TyrRS; group D includes methionine, alanine, valine, or tyrosine at a position corresponding to Phe183 of *E*. *coli* TyrRS; and, group E includes serine, methionine, valine, cysteine, threonine, or alanine at a position corresponding to Leu186 of *E*. *coli* TyrRS. *See also, e.g.,* Table 4, Table 6 and Table 8, herein.

Besides the O-RS, a vertebrate cell of the invention can include additional components, e.g., an unnatural amino acid(s). The vertebrate cell also includes an orthogonal tRNA (O-tRNA) (e.g., derived from a non-vertebrate organism, such as *Escherichia coli, Bacillus stearothermophilus,* and/or the like), where the O-tRNA recognizes a selector codon and is preferentially aminoacylated with the unnatural amino acid by the O-RS. A nucleic acid that comprises a polynucleotide that encodes a polypeptide of interest, wherein the polynucleotide comprises a selector codon that is recognized by the O-tRNA, or a combination of one or more of these, can also be present in the cell.

In one example, a vertebrate cell comprises an orthogonal aminoacyl-tRNA synthetase (O-RS), an orthogonal tRNA (O-tRNA), an unnatural amino acid, and a nucleic acid that comprises a polynucleotide that encodes a polypeptide of interest, which polynucleotide comprises a selector codon that is recognized by the O-tRNA. The O-RS preferentially aminoacylates the orthogonal tRNA (O-tRNA) with the unnatural amino acid in the vertebrate cell, and the cell produces the polypeptide of interest in the absence of the unnatural amino acid with a yield that is, e.g., less than 30%, less than 20%, less than 15%, less than 10%, less than 5%, less than 2.5%, etc., of the yield of the polypeptide in the presence of the unnatural amino acid.

Methods for producing an O-RS, optionally include generating a pool of mutant synthetases from the framework of a wild-type synthetase, and then selecting for mutated RSs based on their specificity for an unnatural amino acid relative to the common twenty amino acids. To isolate such a synthetase, the selection methods of the are: (i) sensitive, as the activity of desired synthetases from the initial rounds can be low and the population small; (ii) "tunable", since it is desirable to vary the selection stringency at different selection rounds; and, (iii) general, so that the methods can be used for different unnatural amino acids.

Methods of producing an orthogonal aminoacyl-tRNA synthetase (O-RS) that preferentially aminoacylates an orthogonal tRNA with an unnatural amino acid in a vertebrate cell typically include applying a combination of a positive selection followed by a negative selection. In the positive selection, suppression of the selector codon introduced at nonessential position(s) of a positive marker allows the vertebrate cells to survive under positive selection pressure. In the presence of unnatural amino acids, survivors thus encode active synthetases charging the orthogonal suppressor tRNA with an unnatural amino acid. In the negative selection, suppression of a selector codon introduced at nonessential position(s) of a negative marker removes synthetases with natural amino acid specificities. Survivors of the negative and positive selection encode synthetases that aminoacylate (charge) the orthogonal suppressor tRNA with unnatural amino acids only (or at least preferentially).

For example, the method includes: (a) subjecting to positive selection, in the presence of an unnatural amino acid, a population of vertebrate cells of a first species, where the vertebrate cells each comprise: i) a member of a library of aminoacyl-tRNA synthetases (RSs), ii) an orthogonal tRNA (O-tRNA), iii) a polynucleotide that encodes a positive selection marker, and iv) a polynucleotide that encodes a negative selection marker; wherein cells that survive the positive selection comprise an active RS that aminoacylates the orthogonal tRNA (O-tRNA) in the presence of an unnatural amino acid; and, (b) subjecting the cells that survive the positive selection to negative selection in the absence of the unnatural amino acid to eliminate active RSs that aminoacylate the O-tRNA with a natural amino acid, thereby providing the O-RS that preferentially aminoacylates the O-tRNA with the unnatural amino acid.

The positive selection marker can be any of a variety of molecules. In one embodiment, the positive selection marker is a product that provides a nutritional supplement for growth and the selection is performed on a medium that lacks the nutritional supplement. Examples of polynucleotides that encode positive selection markers include, but are not limited to, e.g., a reporter gene based on complementing the amino acid auxotrophy of a cell, a his3 gene (e.g., where the his3 gene encodes an imidazole glycerol phosphate dehydratase, detected by providing 3-aminotriazole (3-AT), ura3 gene, leu2 gene, lys2 gene, lacZ gene, adh gene, etc. *See, e.g.,* G.M. Kishore, & D.M. Shah, (1988), Amino acid biosynthesis inhibitors as herbicides, Annual Review of Biochemistry 57:627-663. In one embodiment, lacz production is detected by ortho-nitrophenyl-β-D-galactopyranoside (ONPG) hydrolysis. *See, e.g.,* I.G. Serebriiskii, & E.A. Golemis, (2000), Uses of lacZ to study gene function: evaluation of beta-galactosidase assays employed in the yeast two-hybrid system, Analytical Biochemistry 285:1-15. Additional positive selection markers include, e.g., luciferase, green fluorescent protein (GFP), YFP, EGFP, RFP, the product of an antibiotic resistant gene (e.g., chloramphenicol acetyltransferase (CAT)), a transcriptional modulator protein (e.g., GAL4), etc. Optionally, a polynucleotide that encodes a positive selection marker comprises a selector codon.

A polynucleotide that encodes the positive selection marker can be operably linked to a response element. An additional polynucleotide that encodes a transcriptional modulator protein that modulates transcription from the response element, and comprises at least one selector codon, can also be present. The incorporation of the unnatural amino acid into the transcriptional modulator protein by the O-tRNA aminoacylated with the unnatural amino acid results in transcription of the polynucleotide (e.g., reporter gene) encoding the positive selection marker. Optionally, the selector codon is located in or substantially near a portion of the polynucleotide that encodes a DNA binding domain of the transcriptional modulator protein.

A polynucleotide that encodes the negative selection marker can also be operably linked to a response element from which transcription is mediated by the transcriptional modulator protein. *See, e.g.,* A.J. DeMaggio, et al., (2000), The yeast split-hybrid system, Method Enzymol. 328:128-137; H.M. Shih, et al., (1996), A positive genetic selection for disrupting protein-protein interactions: identification of CREB mutations that prevent association with the coactivator CBP, Proc. Natl. Acad. Sci. U. S. A. 93:13896-13901; M. Vidal, et al., (1996), Genetic characterization of a mammalian protein-protein interaction domain by using a yeast reverse two-hybrid system. [comment], Proc. Natl. Acad. Sci. U.S.A. 93:10321-10326; and, M. Vidal, et al., (1996), Reverse two-hybrid and one-hybrid systems to detect dissociation of protein-protein and DNA-protein interactions.[comment], Proc.Natl. Acad. Sci. U. S. A. 93:10315-10320. The incorporation of a natural amino acid into the transcriptional modulator protein by the O-tRNA aminoacylated with a natural amino acid results in transcription of the negative selection marker. Optionally, the negative selection marker comprises a selector codon. In one embodiment, the positive selection marker and/or negative selection marker of the invention can comprise at least two selector codons, which each or both can comprise at least two different selector codons or at least two of the same selector codons.

The transcriptional modulator protein is a molecule that binds (directly or indirectly) to a nucleic acid sequence (e.g., a response element) and modulates transcription of a sequence that is operably linked to the response element. A transcriptional modulator protein can be a transcriptional activator protein (e.g., GAL4, nuclear hormone receptors, AP1, CREB, LEF/tcf family members, SMADs, VP16, SP1, etc.), a transcriptional repressor protein (e.g., nuclear hormone receptors, Groucho/tle family, Engrailed family, etc), or a protein that can have both activities depending on the environment (e.g., LEF/tcf, homobox proteins, etc.). A response element is typically a nucleic acid sequence that is recognized by the transcriptional modulator protein or an additional agent that acts in concert with the transcriptional modulator protein.

Another example of a transcriptional modulator protein is the transcriptional activator protein, GAL4. *See, e.g.,* A. Laughon, et al., (1984), Identification oftwo proteins encoded by the Saccharomyces cerevisiae GAL4 gene, Molecular & Cellular Biology 4:268-275; A. Laughon, & R.F. Gesteland, (1984), Primary structure of the Saccharomyces cerevisiae GAL4 gene, Molecular & Cellular Biology 4:260-267; L. Keegan, et al., (1986). Separation of DNA binding from the transcription-activating function ofa vertebrate regulatory protein, Science 231:699-704; and, M. Ptashne, (1988), How vertebrate transcriptional activators work, Nature 335:683-689. The N-terminal **147** amino acids of this **881** amino acid protein form a DNA binding domain (DBD) that binds DNA sequence specifically, *See, e.g.,* M. Carey, et al., (1989), An amino-terminal fragment of GAL4 binds DNA as a dimer, J. Mol. Biol. 209:423-432; and, E. Giniger, et al., (1985), Specific DNA binding of GAL4, a positive regulatory protein of yeast, Cell 40:767-774. The DBD is linked, by an intervening protein sequence, to a C-terminal 113 amino acid activation domain (AD) that can activate transcription when bound to DNA. *See, e.g.,* J. Ma, & M. Ptashne, (1987), Deletion analysis of GAL4 defines two transcriptional activating segments, Cell 48:847-853: and, J. Ma, & M. Ptashne, (1987), The carboxy-terminal 30 amino acids of GAL4 are recognized by GAL80, Cell 50:137-142. By placing amber codons towards, e.g., the N-terminal DBD of a single polypeptide that contains both the N-terminal DBD of GAL4 and its C-terminal AD, amber suppression by the O-tRNA/O-RS pair can be linked to transcriptional activation by GAL4. GAL4 activated reporter genes can be used to perform both positive and negative selections with the gene.

The medium used for negative selection can comprise a selecting or screening agent that is converted to a detectable substance by the negative selection marker. In one aspect of the invention, the detectable substance is a toxic substance. A polynucleotide that encodes a negative selection marker can be, e.g., an ura3 gene. For example, the URA3 reporter can be placed under control of a promoter that contains GAL4 DNA binding sites. When the negative selection marker is produced, e.g., by translation of a polynucleotide encoding the GAL4 with selector codons, GAL4 activates transcription of URA3. The negative selection is accomplished on a medium that comprises 5-fluoroorotic acid (5-FOA), which is converted into a detectable substance (e.g., a toxic substance which kills the cell) by the gene product of the ura3 gene. *See, e.g.,* J.D. Boeke, et al., (1984), A positive selection for mutants lacking orotidine-5'-phosphate decarboxylase activity in yeast: 5-fluoroorotic acid resistance, Molecular & General Genetics 197:345-346); M. Vidal, et al., (1996), Genetic characterization ofa mammalian protein-protein interaction domain by using a yeast reverse two-hybrid system.[comment], Proc. Natl. Acad. Sci. U. S. A. 93:10321-10326; and, M. Vidal, et al., (1996), Reverse two-hybrid and one-hybrid systems to detect dissociation of protein-protein and DNA-protein interactions.[comment], Proc. Natl. Acad. Sci. U. S. A. 93:10315-10320.

As with the positive selection marker, the negative selection marker can also be any of a variety of molecules. In one embodiment, the positive selection marker and/or the negative selection marker is a polypeptide that fluoresces or catalyzes a luminescent reaction in the presence of a suitable reactant. For example, negative selection markers include, but are not limited to, e.g., luciferase, green fluorescent protein (GFP), YFP, EGFP, RFP, the product of an antibiotic resistant gene (e.g., chloramphenicol acetyltransferase (CAT)), the product of a lacZ gene, transcriptional modulator protein, etc. In one aspect of the invention, the positive selection marker and/or the negative selection marker is detected by fluorescence-activated cell sorting (FACS) or by luminescence. In another example, the positive selection marker and/or negative selection marker comprise an affinity based screening marker. The same polynucleotide can encode both the positive selection marker and the negative selection marker.

Additional levels of selection/screening stringency can also be used in the methods of the invention. The selection or screening stringency can be varied on one or both steps of the method to produce an O-RS. This could include, e.g., varying the amount of response elements in a polynucleotide that encodes the positive and/or negative selection marker, adding a varying amount of an inactive synthetase to one or both of the steps, varying the amount of selection/screening agent that is used, etc. Additional rounds of positive and/or negative selections can also be performed.

Selecting or screening can also comprise one or more positive or negative selection or screening that includes, e.g., a change in amino acid permeability, a change in translation efficiency, a change in translational fidelity, etc. Typically, the one or more change is based upon a mutation in one or more polynucleotides that comprise or encode components of an orthogonal tRNA-tRNA synthetase pair that are used to produce protein.

Model enrichment studies can also be used to rapidly select an active synthetase from an excess of inactive synthetases. Positive and/or negative model selection studies can be done. For example, vertebrate cells that comprise potential active aminoacyl-tRNA synthetases are mixed with a varying fold excess of inactive aminoacyl-tRNA synthetases. A ratio comparison is made between cells grown in a nonselective media and assayed by, e.g., X-GAL overlay, and those grown and able to survive in a selective media (e.g., in the absence of histidine and/or uracil) and assayed by, e.g., an X-GAL assay. For a negative model selection, potential active aminoacyl-tRNA synthetases are mixed with a varying fold excess of inactive aminoacyl-tRNA synthetases and selection is performed with a negative selection substance, e.g., 5-FOA.

Typically, the library of RSs (e.g., a library of mutant RSs) comprises RSs derived from at least one aminoacyl-tRNA synthetase (RS), e.g., from a non-vertebrate organism. In one embodiment, the library of RSs is derived from an inactive RS, e.g., where the inactive RS is generated by mutating an active RS, e.g., at the active site in the synthetase, at the editing mechanism site in the synthetase, at different sites by combining different domains of synthetases, or the like. For example, residues in the active site of the RS are mutated to, e.g., alanine residues. The polynucleotide that encodes the alanine mutated RS is used as a template to mutagenize the alanine residues to all 20 amino acids. The library of mutant RSs is selected/screened to produce the O-RS. In another embodiment, the inactive RS comprises an amino acid binding pocket and one or more amino acids that comprise the binding pocket are substituted with one or more different amino acids. In one example, the substituted amino acids are substituted with alanines. Optionally, the polynucleotide that encodes the alanine mutated RS is used as a template to mutagenize the alanine residues to all 20 amino acids and screened/selected.

The method of producing an O-RS can further include producing the library of RSs by using various mutagenesis techniques known in the art. For example, the mutant RSs can be generated by site-specific mutations, random point mutations, homologous recombination, DNA shuffling or other recursive mutagenesis methods, chimeric construction or any combination thereof. For example, a library of mutant RSs can be produced from two or more other, e.g., smaller, less diverse "sub-libraries." Once the synthetases are subjected to the positive and negative selection/screening strategy, these synthetases can then be subjected to further mutagenesis. For example, a nucleic acid that encodes the O-RS can be isolated; a set of polynucleotides that encode mutated O-RSs (e.g., by random mutagenesis, site-specific mutagenesis, recombination or any combination thereof) can be generated from the nucleic acid; and, these individual steps or a combination of these steps can be repeated until a mutated O-RS is obtained that preferentially aminoacylates the O-tRNA with the unnatural amino acid. In one aspect of the invention, the steps are performed at least two times.

Additional details for producing O-RS can be found in WO 2002/086075 entitled "Methods and compositions for the production of orthogonal tRNA-aminoacyltRNA synthetase pairs." *See also,* Hamano-Takaku et al., (2000) A mutant Escherichia coli Tyrosyl-tRNA Synthetase Utilizes the Unnatural Amino Acid Azatyrosine More Efficiently than Tyrosine, Journal of Biological Chemistry, 275(1):40324-40328; Kiga et al. (2002), An engineered Escherichia coli tyrosyl-tRNA synthetase for site-specific incorporation of an unnatural amino acid into proteins in vertebrate translation and its application in a wheat germ cell-free system, PNAS 99(15): 9715-9723; and, Francklyn et al., (2002), Aminoacyl-tRNA synthetases: Versatile players in the changing theater of translation; RNA, 8:1363-1372.

Orthogonal tRNA's

Eukaryotic cells that include an orthogonal tRNA (O-tRNA) are provided by the invention. The orthogonal tRNA mediates incorporation of an unnatural amino acid into a protein that is encoded by a polynucleotide that comprises a selector codon that is recognized by the O-tRNA, in vivo. In certain embodiments, an O-tRNA of the invention mediates the incorporation of an unnatural amino acid into a protein with, e.g., at least 40%, at least 45%, at least 50%, at least 60%, at least 75%, at least 80%, or even 90% or more as efficiently as tRNA that comprises or is processed in a cell from a polynucleotide sequence as set forth in SEQ ID NO.: 65. *See,* Table 5, herein.

Typically, an O-tRNA of the invention is a recyclable O-tRNA, because the O-tRNA can be reaminoacylated in vivo to again mediate the incorporation of the unnatural amino acid into a protein that is encoded by a polynucleotide in response to a selector codon.

The transcription of the tRNA in eukaryotes, but not in prokaryotes, is carried out by RNA Polymerase III, which places restrictions on the primary sequence of the tRNA structural genes that can be transcribed in vertebrate cells. In addition, in vertebrate cells, tRNA's need to be exported from the nucleus, where they are transcribed, to the cytoplasm, to function in translation. Nucleic acids that encode an O-tRNA of the invention or a complementary polynucleotide thereof are also a feature of the invention. In one aspect of the disclosure, a nucleic acid that encodes an O-tRNA of the invention includes an internal promoter sequence, e.g., an A box (e.g., TRGCNNAGY) and a B box (e.g., GGTTCGANTCC, SEQ ID NO: 87). Additional examples of A box and B box sequences can be found in Geiduschek, (1988), Transcription By RNA Polymerase III, Ann. Rev. Biochem. 57:873-914. The O-tRNA of the invention can also be post-transcriptionally modified. For example, post-transcriptional modification of tRNA genes in eukaryotes includes removal of the 5'- and 3'- flanking sequences by Rnase P and a 3'- endonuclease, respectively. The addition of a 3'- CCA sequence is also a post-transcriptional modification of a tRNA gene in eukaryotes.

In one aspect, an O-tRNA is obtained by subjecting to negative selection a population of vertebrate cells of a first species, where the vertebrate cells comprise a member of a library of tRNA's. The negative selection eliminates cells that comprise a member of the library of tRNA's that is aminoacylated by an aminoacyl-tRNA synthetase (RS) that is endogenous to the vertebrate cells. This provides a pool of tRNA's that are orthogonal to the vertebrate cell of the first species.

Alternatively, or in combination with others methods described above to incorporate an unnatural amino acid into a polypeptide, a trans-translation system can be used. This system involves a molecule called tmRNA present in *Escherichia coli.* This RNA molecule is structurally related to an alanyl tRNA and is aminoacylated by the alanyl synthetase. The difference between tmRNA and tRNA is that the anticodon loop is replaced with a special large sequence. This sequence allows the ribosome to resume translation on sequences that have stalled using an open reading frame encoded within the tmRNA as template. In the invention, an orthogonal tmRNA can be generated that is preferentially aminoacylated with an orthogonal synthetase and loaded with an unnatural amino acid. By transcribing a gene by the system, the ribosome stalls at a specific site; the unnatural amino acid is introduced at that site, and translation resumes using the sequence encoded within the orthogonal tmRNA.

Additional methods for producing a recombinant orthogonal tRNA's can be found, e.g., in International patent applications WO 2002/086075, entitled "Methods and compositions for the production of orthogonal tRNA-aminoacyltRNA synthetase pairs." *See also,* Forster et al., (2003) Programming peptidomimetic synthetases by translating genetic codes designed de novo PNAS 100(11):6353-6357; and, Feng et al., (2003), Expanding tRNA recognition of a tRNA synthetase by a single amino acid change, PNAS 100(10): 5676-5681.

Orthogonal TRNA and Orthogonal aminoacyl-TRNA synthetase pairs

An orthogonal pair is composed of an O-tRNA, e.g., a suppressor tRNA, a frameshift tRNA, or the like, and an O-RS. The O-tRNA is not acylated by endogenous synthetases and is capable of mediating incorporation of an unnatural amino acid into a protein that is encoded by a polynucleotide that comprises a selector codon that is recognized by the **O-tRNA** in vivo. The O-RS recognizes the **O-tRNA** and preferentially aminoacylates the O-tRNA with an unnatural amino acid in a vertebrate cell. The development of multiple orthogonal tRNA/synthetase pairs can allow the simultaneous incorporation of multiple unnatural amino acids using different codons in a vertebrate cell.

An orthogonal O-tRNA/O-RS pair in a vertebrate cell can be produced by importing a pair, e.g., a nonsense suppressor pair, from a different organism with inefficient cross species aminoacylation. The O-tRNA and O-RS are efficiently expressed and processed in the vertebrate cell and the O-tRNA is efficiently exported from the nucleus to the cytoplasm. For example, one such pair is the tyrosyl-tRNA synthetase/tRNA_{CUA} pair from *E. coli (see. e.g.,* H. M. Goodman, et al., (1968), Nature 217:1019-24; and, D. G. Barker, et al., (1982), FEBS Letters 150:419-23). *E. coli* tyrosyl-tRNA synthetase efficiently aminoacylates its cognate *coli* tRNA_{CUA} when both are expressed in the cytoplasm of *S. cerevisiae,* but does not aminoacylate *S*. *cerevisiae* tRNA's. *See, e.g.,* H. Edwards, & P. Schimmel, (1990), Molecular & Cellular Biology 10:1633-41; and, H. Edwards, et al., (1991), PNAS United States of America 88:1153-6. In addition, *E*. *coli* tyrosyl tRNA_{CUA} is a poor substrate for *S*. *cerevisiae* aminoacyl-tRNA synthetases (*see, e.g.,* V. Trezeguet, et al., (1991), Molecular & Cellular Biology 11:2744-51), but functions efficiently in protein translation in *S. cerevisiae. See, e.g.,* H. Edwards, & P. Schimmel, (1990) Molecular & Cellular Biology 10:1633-41; H. Edwards, et al., (1991), PNAS United States of America 88:1153-6; and, V. Trezeguet, et al., (1991), Molecular & Cellular Biology 11:2744-51. Moreover, *E*. *coli* TyrRS does not have an editing mechanism to proofread an unnatural amino acid ligated to the tRNA.

The O-tRNA and O-RS can be naturally occurring or can be derived by mutation of a naturally occurring tRNA and/or RS, which generates libraries of tRNA's and/or libraries of RSs, from a variety of organism. See the section entitled "Sources and Hosts" herein. In various embodiments, the O-tRNA and O-RS are derived from at least one organism. In another embodiment, the O-tRNA is derived from a naturally occurring or mutated naturally occurring tRNA from a first organism and the O-RS is derived from naturally occurring or mutated naturally occurring RS from a second organism. In one embodiment, the first and second non-vertebrate organisms are the same. Alternatively, the first and second non-vertebrate organisms can be different.

See sections herein entitled "Orthogonal aminoacyl-tRNA synthetases" and "O-tRNA" for methods of producing O-RSs and O-tRNA's. *See also,* International patent application WO 2002/086075, entitled "Methods and compositions for the production of orthogonal tRNA-aminoacyltRNA synthetase pairs."

Fidelity, Efficiency, and Yield

Fidelity refers to the accuracy with which a desired molecule, e.g., an unnatural amino acid or amino acid, is incorporated into a growing polypeptide at a desired position. The translational components of the invention incorporate unnatural amino acids, with high fidelity, into proteins in response to a selector codon. For example, using the components of the invention, the efficiency of incorporation of a desired unnatural amino acid into a growing polypeptide chain at a desired position (e.g., in response to a selector codon) is, e.g., greater than 75%, greater than 85%, greater than 95%, or even greater than 99% or more as efficient as compared to unwanted incorporation a specific natural amino acid being incorporated into the growing polypeptide chain the desired position.

Efficiency can also refer to the degree with which the O-RS aminoacylates the O-tRNA with the unnatural amino acid as compared to a relevant control. O-RSs of the invention can be defined by their efficiency.

Source and Host Organisms

The orthogonal translational components of the invention are typically derived from non-vertebrate organisms for use in vertebrate cells or translation systems. For example, the orthogonal O-tRNA can be derived from a non-vertebrate organism, e.g., a eubacterium, such as *Escherichia coli, Thermus thermophilus, Bacillus stearothermphilus,* or the like, or an archaebacterium, such as *Methanococcus jannaschii, Methanobacterium thermoautotrophicum, Halobacterium* such as *Haloferax volcanii* and *Halobacterium* species *NRC-1, Archaeoglobus fulgidus, Pyrococcus furiosus, Pyrococcus horikoshii, Aeuropyrum pernix,* or the like, while the orthogonal O-RS can be derived from a non-vertebrate organism, e.g., a eubacterium, such as *Escherichia coli, Thermus thermophilus, Bacillus stearothermphilus,* or the like, or an archaebacterium, such as *Methanococcus jannaschii, Methanobacterium thermoautotrophicum, Halobacterium* such as *Haloferax volcanii* and *Halobacterium* species *NRC-1, Archaeoglobus fulgidus, Pyrococcus furiosus, Pyrococcus horikoshii, Aeuropyrum pernix,* or the like. Alternately, vertebrate sources can also be used, e.g., plants, algae, protists, fungi, yeasts, animals (e.g., mammals, insects, arthropods, etc.), or the like, e.g., where the components are orthogonal to a cell or translation system of interest, or where they are modified (e.g., mutated) to be orthogonal to the cell or translation system.

The individual components of an O-tRNA/O-RS pair can be derived from the same organism or different organisms. In one embodiment, the O-tRNA/O-RS pair is from the same organism. For example, the O-tRNA/O-RS pair can be derived from a tyrosyl-tRNA synthetase/tRNA_{CUA} pair from *E*. *coli*. Alternatively, the O-tRNA and the O-RS of the 0-tRNA/O-RS pair are optionally from different organisms.

The orthogonal O-tRNA, O-RS or O-tRNA/O-RS pair can be selected or screened and/or used in a vertebrate cell to produce a polypeptide with an unnatural amino acid. A vertebrate cell can be from a variety of sources, e.g., any vertebrate animal (e.g., a mammal, an amphibian, birds, reptiles, fish, etc.), or the like. Compositions of vertebrate cells with translational components of the invention are also a feature of the invention.

In one example, the method of producing O-tRNA/O-RS in a first species as described herein further includes introducing a nucleic acid that encodes the O-tRNA and a nucleic acid that encodes the O-RS into a vertebrate cell of a second species (e.g., a mammal, an insect, a fungus, an algae, a plant and the like). In another example, a method of producing an orthogonal aminoacyl-tRNA synthetase (O-RS) that preferentially aminoacylates an orthogonal tRNA with an unnatural amino acid in a vertebrate cell includes: (a) subjecting to positive selection, in the presence of an unnatural amino acid, a population of vertebrate cells of a first species (e.g., yeast and the like). Each of the vertebrate cells comprise: i) a member of a library of aminoacyl-tRNA synthetases (RSs), ii) an orthogonal tRNA (O-tRNA), iii) a polynucleotide that encodes a positive selection marker, and iv) a polynucleotide that encodes a negative selection marker. The cells that survive the positive selection comprise an active RS that aminoacylates the orthogonal tRNA (O-tRNA) in the presence of an unnatural amino acid. The cells that survive the positive selection are subjected to negative selection in the absence of the unnatural amino acid to eliminate active RSs that aminoacylate the O-tRNA with a natural amino acid. This provides an O-RS that preferentially aminoacylates the O-tRNA with the unnatural amino acid. A nucleic acid that encodes the O-tRNA and a nucleic acid that encodes the O-RS (or the components O-tRNA and/or O-RS) are introduced into a vertebrate cell of a second species e.g., a mammal, an insect, a fungus, an algae, a plant and/or the like. Typically, the O-tRNA is obtained by subjecting to negative selection a population of vertebrate cells of a first species, where the vertebrate cells comprise a member of a library of tRNA's. The negative selection eliminates cells that comprise a member of the library of tRNA's that is aminoacylated by an aminoacyl-tRNA synthetase (RS) that is endogenous to the vertebrate cells, which provides a pool of tRNA's that are orthogonal to the vertebrate cell of the first species and the second species.

Selector Codons

Selector codons of the invention expand the genetic codon framework of the protein biosynthetic machinery. For example, a selector codon includes, e.g., a unique three base codon, a nonsense codon, such as a stop codon, e.g., an amber codon (UAG), an opal codon (UGA), an unnatural codon, at least a four base codon, a rare codon, or the like. A number of selector codons can be introduced into a desired gene, e.g., one or more, two or more, more than three, etc. Once gene can include multiple copies of a given selector codon, or can include multiple different selector codons, or any combination thereof.

In one aspect, the methods involve the use of a selector codon that is a stop codon for the incorporation of unnatural amino acids in vivo in a vertebrate cell. For example, an O-tRNA is produced that recognizes the stop codon, e.g., UAG, and is aminoacylated by an O-RS with a desired unnatural amino acid. This **O-tRNA** is not recognized by the naturally occurring host's aminoacyl-tRNA synthetases. Conventional site-directed mutagenesis can be used to introduce the stop codon, e.g., TAG, at the site of interest in a polypeptide of interest. *See, e.g.,* Sayers, J.R., et al. (1988), 5',3'Exonuclease in phosphorothioate-based oligonucleotide-directed mutagenesis. Nucleic Acids Res, 791-802. When the O-RS, O-tRNA and the nucleic acid that encodes the polypeptide of interest are combined in vivo, the unnatural amino acid is incorporated in response to the UAG codon to give a polypeptide containing the unnatural amino acid at the specified position.

The incorporation of unnatural amino acids in vivo can be done without significant perturbation of the vertebrate host cell. For example, because the suppression efficiency for the UAG codon depends upon the competition between the O-tRNA, e.g., the amber suppressor tRNA, and a vertebrate release factor (e.g., eRF) (which binds to a stop codon and initiates release of the growing peptide from the ribosome), the suppression efficiency can be modulated by, e.g., increasing the expression level of O-tRNA, e.g., the suppressor tRNA.

Selector codons also comprise extended codons, e.g., four or more base codons, such as, four, five, six or more base codons. Examples of four base codons include, e.g., AGGA, CUAG, UAGA, CCCU and the like. Examples of five base codons include, e.g., AGGAC, CCCCU, CCCUC, CUAGA, CUACU, UAGGC and the like. A feature of the invention includes using extended codons based on frameshift suppression. Four or more base codons can insert, e.g., one or multiple unnatural amino acids into the same protein. For example, in the presence of mutated O-tRNA's, e.g., a special frameshift suppressor tRNA's, with anticodon loops, e.g., with at least 8-10 nt anticodon loops, the four or more base codon is read as single amino acid. In other embodiments, the anticodon loops can decode, e.g., at least a four-base codon, at least a five-base codon, or at least a six-base codon or more. Since there are 256 possible four-base codons, multiple unnatural amino acids can be encoded in the same cell using a four or more base codon. *See,* Anderson et al., (2002) Exploring the Limits of Codon and Anticodon Size, Chemistry and Biology, 9:237-244; Magliery, (2001) Expanding the Genetic Code: Selection of Efficient Suppressors of Four-base Codons and Identification of "Shifty" Four-base Codons with a Library Approach in Escherichia coli, J. Mol. Biol. 307: 755-769.

For example, four-base codons have been used to incorporate unnatural amino acids into proteins using in vitro biosynthetic methods. *See, e.g.,* Ma et al., (1993) Biochemistry, 32:7939; and Hohsaka et al., (1999) J. Am. Chem. Soc., 121:34. CGGG and AGGU were used to simultaneously incorporate 2-naphthylalanine and an NBD derivative of lysine into streptavidin in vitro with two chemically acylated frameshift suppressor tRNA's. *See, e.g.,* Hohsaka et al., (1999) J. Am. Chem. Soc., 121:12194. In an in vivo study, Moore et al. examined the ability of tRNALeu derivatives with NCUA anticodons to suppress UAGN codons (N can be U, A, G, or C), and found that the quadruplet UAGA can be decoded by a tRNALeu with a UCUA anticodon with an efficiency of 13 to 26% with little decoding in the 0 or -1 frame. *See,* Moore et al., (2000) J. Mol. Biol. 298:195. In one embodiment, extended codons based on rare codons or nonsense codons can be used in invention, which can reduce missense readthrough and frameshift suppression at other unwanted sites.

For a given system, a selector codon can also include one of the natural three base codons, where the endogenous system does not use (or rarely uses) the natural base codon. For example, this includes a system that is lacking a tRNA that recognizes the natural three-base codon, and/or a system where the three-base codon is a rare codon.

Selector codons optionally include unnatural base pairs. These unnatural base pairs further expand the existing genetic alphabet. One extra base pair increases the number of triplet codons from 64 to 125. Properties of third base pairs include stable and selective base pairing, efficient enzymatic incorporation into DNA with high fidelity by a polymerase, and the efficient continued primer extension after synthesis of the nascent unnatural base pair. Descriptions of unnatural base pairs which can be adapted for methods and compositions include, e.g., Hirao, et al., (2002) An unnatural base pair four incorporating amino acid analogues into protein, Nature Biotechnology 20:177-182. Other relevant publications are listed below.

For in vivo usage, the unnatural nucleoside is membrane permeable and is phosphorylated to form the corresponding triphosphate. In addition, the increased genetic information is stable and not destroyed by cellular enzymes. Previous efforts by Benner and others took advantage of hydrogen bonding patterns that are different from those in canonical Watson-Crick pairs, the most noteworthy example of which is the iso-C:iso-G pair. *See, e.g.,* Switzer et al., (1989) J. Am. Chem. Soc., 111:8322; and Piccirilli et al., (1990) Nature. 343:33; Kool, (2000) Curr. Opin. Chem. Biol., 4:602. These bases in general mispair to some degree with natural bases and cannot be enzymatically replicated. Kool and co-workers demonstrated that hydrophobic packing interactions between bases can replace hydrogen bonding to drive the formation of base pair. *See,* Kool, (2000) Curr. Opin. Chem. Biol., 4:602; and Guckian and Kool, (1998) Angew. Chem. Int. Ed. Engl., 36, 2825. In an effort to develop an unnatural base pair satisfying all the above requirements, Schultz, Romesberg and co-workers have systematically synthesized and studied a series of unnatural hydrophobic bases. A PICS:PICS self-pair is found to be more stable than natural base pairs, and can be efficiently incorporated into DNA by Klenow fragment of Escherichia coli DNA polymerase I (KF). *See, e.g.,* McMinn et al., (1999) J. Am. Chem. Soc., 121:11586; and Ogawa et al., (2000) J. Am. Chem. Soc., 122:3274. A 3MN:3MN self-pair can be synthesized by KF with efficiency and selectivity sufficient for biological function. *See, e.g.,* Ogawa et al., (2000) J. Am. Chem. Soc., 122:8803. However, both bases act as a chain terminator for further replication. A mutant DNA polymerase has been recently evolved that can be used to replicate the PICS self pair. In addition, a 7AI self pair can be replicated. See, e.g., Tae et al., (2001) J. Am. Chem. Soc., 123:7439. A novel metallobase pair, Dipic:Py, has also been developed, which forms a stable pair upon binding Cu(II). See, Meggers et al., (2000) J. Am. Chem. Soc., 122:10714. Because extended codons and unnatural codons are intrinsically orthogonal to natural codons, the methods of the invention can take advantage of this property to generate orthogonal tRNA's for them.

A translational bypassing system can also be used to incorporate an unnatural amino acid in a desired polypeptide. In a translational bypassing system, a large sequence is inserted into a gene but is not translated into protein. The sequence contains a structure that serves as a cue to induce the ribosome to hop over the sequence and resume translation downstream of the insertion.

**Polypeptides with Unnatural Amino Acids**

Proteins or polypeptides of interest with at least one unnatural amino acid are a feature of the invention. The invention also includes polypeptides or proteins with at least one unnatural amino acid produced using the compositions and methods of the invention. An excipient (e.g., a pharmaceutically acceptable excipient) can also be present with the protein.

By producing proteins or polypeptides of interest with at least one unnatural amino acid in vertebrate cells, proteins or polypeptides will typically include vertebrate posttranslational modifications. In certain embodiments, a protein includes at least one unnatural amino acid and at least one post-translational modification that is made in vivo by a vertebrate cell, where the post-translational modification is not made by a prokaryotic cell. For example, the post-translation modification includes, e.g., acetylation, acylation, lipid-modification, palmitoylation, palmitate addition, phosphorylation, glycolipid-linkage modification, glycosylation, and the like. In one aspect, the post-translational modification includes attachment of an oligosaccharide (e.g., (GlcNAc-Man)₂-Man-GlcNAc-GlcNAc)) to an asparagine by a GIcNAc-asparagine linkage. See also, Table 7, which lists some examples of N-linked oligosaccharides of vertebrate proteins (additional residues can also be present, which are not shown). In another aspect, the post-translational modification includes attachment of an oligosaccharide (e.g., Gal-GalNAc, Gal-GlcNAc, etc.) to a serine or threonine by a GalNAc-serine or GalNAc-threonine linkage, or a GlcNAc-serine or a GlcNAc-threonine linkage.

**TABLE 7: EXAMPLES OF OLIGOSACCHARIDES THROUGH GlcNAc-LINKAGE**

| **Type** | **Base Structure** |
|---|---|
| **High-mannose** | 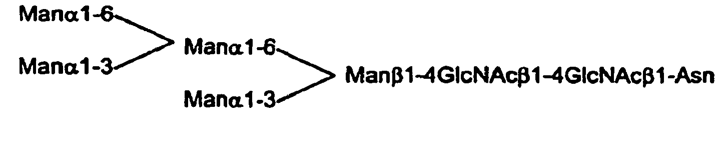 |
| **Hybrid** | 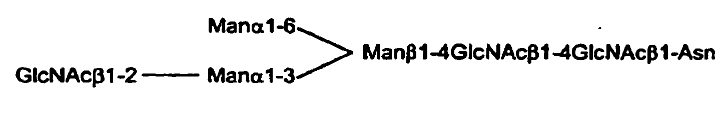 |
| **Complex** | 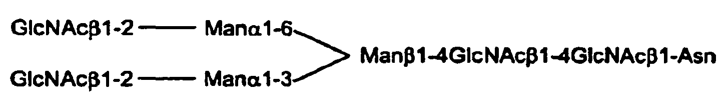 |
| **Xylose** | 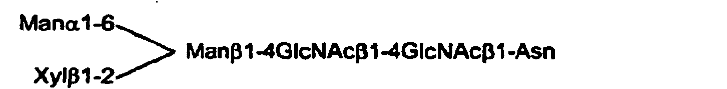 |

In yet another aspect, the post-translation modification includes proteolytic processing of precursors (e.g., calcitonin precursor, calcitonin gene-related peptide precursor, preproparathyroid hormone, preproinsulin, proinsulin, prepro-opiomelanocortin, pro-opiomelanocortin and the like), assembly into a multisubunit protein or macromolecular assembly, translation to another site in the cell (e.g., to organelles, such as the endoplasmic reticulum, the golgi apparatus, the nucleus, lysosomes, peroxisomes, mitochondria, chloroplasts, vacuoles, etc., or through the secretory pathway). In certain embodiments, the protein comprises a secretion or localization sequence, an epitope tag, a FLAG tag, a polyhistidine tag, a GST fusion, or the like.

One advantage of an unnatural amino acid is that it presents additional chemical moieties that can be used to add additional molecules. These modifications can be made in vivo in a vertebrate cell, or in vitro. Thus, in certain embodiments, the post-translational modification is through the unnatural amino acid. For example, the post-translational modification can be through a nucleophilic-electrophilic reaction. Most reactions currently used for the selective modification of proteins involve covalent bond formation between nucleophilic and electrophilic reaction partners, e.g. the reaction of α-haloketones with histidine or cysteine side chains. Selectivity in these cases is determined by the number and accessibility of the nucleophilic residues in the protein. In proteins of the invention, other more selective reactions can be used, such as the reaction of an unnatural keto-amino acid with hydrazides or aminooxy compounds, in vitro and in vivo. *See, e.g.,* Cornish, et al., (1996) Am. Chem. Soc., 118:8150-8151; Mahal, et al., (1997) Science, 276:1125-1128; Wang, et al., (2001) Science 292:498-500; Chin, et al., (2002) Am. Chem. Soc. 124:9026-9027; Chin, et al., (2002) Proc. Natl. Acad. Sci., 99:11020-11024; Wang, et al., (2003) Proc. Natl. Acad. Sci., 100:56-61; Zhang, et al., (2003) Biochemistry, 42:6735-6746; and, Chin, et al., (2003) Science, in press. This allows the selective labeling of virtually any protein with a host of reagents including fluorophores, crosslinking agents, saccharide derivatives and cytotoxic molecules. *See also,* patent application USSN 10/686,944 entitled "Glycoprotein synthesis" filed October 15, 2003. Post-translational modifications, e.g., through an azido amino acid, can also made through the Staudinger ligation (e.g., with triarylphosphine reagents). *See, e.g.,* Kiick et al., (2002) Incorporation of azides into recombinant proteins for chemoselective modification by the Staudinger ligtation, PNAS 99:19-24.

Described herein is another highly efficient method for the selective modification of proteins, which involves the genetic incorporation of unnatural amino acids, e.g., containing an azide or alkynyl moiety into proteins in response to a selector codon. These amino acid side chains can then be modified by, e.g., a Huisgen [3+2] cycloaddition reaction (*see, e.g.,* Padwa, A. in Comprehensive Organic Synthesis, Vol. 4, (1991) Ed. Trost, B. M., Pergamon, Oxford, p. 1069-1109; and, Huisgen, R. in 1,3-Dipolar Cycloaddition Chemistry, (1984) Ed. Padwa, A., Wiley, New York, p. 1-176) with, e.g., alkynyl or azide derivatives, respectively. *See, e.g.,* **Figure 16.** Because this method involves a cycloaddition rather than a nucleophilic substitution, proteins can be modified with extremely high selectivity. This reaction can be carried out at room temperature in aqueous conditions with excellent regioselectivity (1,4 > 1,5) by the addition of catalytic amounts of Cu(I) salts to the reaction mixture. *See, e.g.,* Tomoe, et al., (2002) Org. Chem. 67:3057-3064; and, Rostovtsev, et al., (2002) Angew. Chem. Int. Ed. 41:2596-2599. Another method that can be used is the ligand exchange on a bisarsenic compound with a tetracysteine motif, *see, e.g.,* Griffin, et al., (1998) Science 281:269-272.

A vertebrate cell of the invention provides the ability to synthesize proteins that comprise unnatural amino acids in large useful quantities. The production of large quantities (e.g., greater that that typically possible with other methods, e.g., in vitro translation) of a protein in a vertebrate cell including at least one unnatural amino acid is a feature of the invention.

The incorporation of an unnatural amino acid can be done to, e.g., tailor changes in protein structure and/or function, e.g., to change size, acidity, nucleophilicity, hydrogen bonding, hydrophobicity, accessibility of protease target sites, target to a moiety (e.g., for a protein array), etc. Proteins that include an unnatural amino acid can have enhanced or even entirely new catalytic or physical properties. For example, the following properties are optionally modified by inclusion of an unnatural amino acid into a protein: toxicity, biodistribution, structural properties, spectroscopic properties, chemical and/or photochemical properties, catalytic ability, half-life (e.g., serum half-life), ability to react with other molecules, e.g., covalently or noncovalently, and the like. The compositions including proteins that include at least one unnatural amino acid are useful for, e.g., novel therapeutics, diagnostics, catalytic enzymes, industrial enzymes, binding proteins (e.g., antibodies), and e.g., the study of protein structure and function. See, *e.g.,* Dougherty, (2000) Unnatural Amino Acids as Probes of Protein Structure and Function, Current Opinion in Chemical Biology, 4:645-652.

Essentially any protein (or portion thereof) that includes an unnatural amino acid (and any corresponding coding nucleic acid, e.g., which includes one or more selector codons) can be produced using the compositions and methods herein. No attempt is made to identify the hundreds of thousands of known proteins, any of which can be modified to include one or more unnatural amino acid, e.g., by tailoring any available mutation methods to include one or more appropriate selector codon in a relevant translation system. Common sequence repositories for known proteins include GenBank EMBL, DDBJ and the NCBI. Other repositories can easily be identified by searching the internet.

Typically, the proteins are, e.g., at least 60%, at least 70%, at least 75%, at least 80%, at least 90%, at least 95%, or at least 99% or more identical to any available protein (e.g., a therapeutic protein, a diagnostic protein, an industrial enzyme, or portion thereof, and the like), and they comprise one or more unnatural amino acid. Examples of therapeutic, diagnostic, and other proteins that can be modified to comprise one or more unnatural amino acids include, but are not limited to, e.g., Alpha-1 antitrypsin, Angiostatin, Antihemolytic factor, antibodies (further details on antibodies are found below), Apolipoprotein, Apoprotein, Atrial natriuretic factor, Atrial natriuretic polypeptide, Atrial peptides, C-X-C chemokines (e.g., T39765, NAP-2, ENA-78, Gro-a, Gro-b, Gro-c, IP-10, GCP-2, NAP-4, SDF-1, PF4, MIG), Calcitonin, CC chemokines (e.g., Monocyte chemoattractant protein-1, Monocyte chemoattractant protein-2, Monocyte chemoattractant protein-3, Monocyte inflammatory protein-1 alpha, Monocyte inflammatory protein-1 beta, RANTES, I309, R83915, R91733, HCC1, T58847, D31065, T64262), CD40 ligand, C-kit Ligand, Collagen, Colony stimulating factor (CSF), Complement factor 5a, Complement inhibitor, Complement receptor 1, cytokines, (e.g., epithelial Neutrophil Activating Peptide-78, GROα/MGSA, GROβ, GROγ, MIP-1α, MIP-1δ, MCP-1), Epidermal Growth Factor (EGF), Erythropoietin ("EPO", representing a preferred target for modification by the incorporation of one or more unnatural amino acid), Exfoliating toxins A and B, Factor IX, Factor VII, Factor VIII, Factor X, Fibroblast Growth Factor (FGF), Fibrinogen, Fibronectin, G-CSF, GM-CSF, Glucocerebrosidase, Gonadotropin, growth factors, Hedgehog proteins (e.g., Sonic, Indian, Desert), Hemoglobin, Hepatocyte Growth Factor (HGF), Hirudin, Human serum albumin, Insulin, Insulin-like Growth Factor (IGF), interferons (e.g., IFN-α, IFN-β, IFN-γ), interleukins (e.g., IL-1, IL-2, IL-3, IL-4, IL-5, IL-6, IL-7, IL-8, IL-9, IL-10, IL-11, IL-12, etc.), Keratinocyte Growth Factor (KGF), Lactoferrin, leukemia inhibitory factor, Luciferase, Neurturin, Neutrophil inhibitory factor (NIF), oncostatin M, Osteogenic protein, Parathyroid hormone, PD-ECSF, PDGF, peptide hormones (e.g., Human Growth Hormone), Pleiotropin, Protein A, Protein G, Pyrogenic exotoxins A, B, and C, Relaxin, Renin, SCF, Soluble complement receptor 1, Soluble I-CAM 1, Soluble interleukin receptors (IL-1, 2, 3, 4, 5, 6, 7, 9, 10, 11, 12, 13, 14, 15), Soluble TNF receptor, Somatomedin, Somatostatin, Somatotropin, Streptokinase, Superantigens, i.e., Staphylococcal enterotoxins (SEA, SEB, SEC1, SEC2, SEC3, SED, SEE), Superoxide dismutase (SOD), Toxic shock syndrome toxin (TSST-1), Thymosin alpha 1, Tissue plasminogen activator, Tumor necrosis factor beta (TNF beta), Tumor necrosis factor receptor (TNFR), Tumor necrosis factor-alpha (TNF alpha), Vascular Endothelial Growth Factor (VEGEF), Urokinase, and many others.

One class of proteins that can be made using the compositions and methods for in vivo incorporation of unnatural amino acids described herein includes transcriptional modulators or portions thereof. Example transcriptional modulators include genes and transcriptional modulator proteins that modulate cell growth, differentiation, regulation, or the like. Transcriptional modulators are found in prokaryotes, viruses, and eukaryotes, including fungi, plants, yeasts, insects, and animals, including mammals, providing a wide range of therapeutic targets. It will be appreciated that expression and transcriptional activators regulate transcription by many mechanisms, e.g., by binding to receptors, stimulating a signal transduction cascade, regulating expression of transcription factors, binding to promoters and enhancers, binding to proteins that bind to promoters and enhancers, unwinding DNA, splicing pre-mRNA, polyadenylating RNA, and degrading RNA. For example, compositions of GAL4 protein or portion thereof in a vertebrate cell are also a feature of the invention. Typically, the GAL4 protein or portion thereof comprises at least one unnatural amino acid. See also the section herein entitled "Orthogonal aminoacyl-tRNA synthetases."

One class of proteins of the invention (e.g., proteins with one or more unnatural amino acids) include expression activators such as cytokines, inflammatory molecules, growth factors, their receptors, and oncogene products, e.g., interleukins (e.g., IL-1, IL-2, IL-8, etc.), interferons, FGF, IGF-I, IGF-II, FGF, PDGF, TNF, TGF-α, TGF-β, EGF, KGF, SCF/c-Kit, CD40L/CD40, VLA-4/VCAM-1, ICAM-1/LFA-1, and hyalurin/CD44; signal transduction molecules and corresponding oncogene products, e.g., Mos, Ras, Raf, and Met; and transcriptional activators and suppressors, e.g., p53, Tat, Fos, Myc, Jun, Myb, Rel, and steroid hormone receptors such as those for estrogen, progesterone, testosterone, aldosterone, the LDL receptor ligand and corticosterone.

Enzymes (e.g., industrial enzymes), or portions thereof with at least one unnatural amino acid, are also provided by the invention. Examples of enzymes include, but are not limited to, e.g., amidases, amino acid racemases, acylases, dehalogenases, dioxygenases, diarylpropane peroxidases, epimerases, epoxide hydrolases, esterases, isomerases, kinases, glucose isomerases, glycosidases, glycosyl transferases, haloperoxidases, monooxygenases (e.g., p450s), lipases, lignin peroxidases, nitrile hydratases, nitrilases, proteases, phosphatases, subtilisins, transaminase, and nucleases.

Many of these proteins are commercially available *(See,* e.g., the Sigma BioSciences 2002 catalogue and price list), and the corresponding protein sequences and genes and, typically, many variants thereof, are well-known (*see,* e.g., Genbank). Any of them can be modified by the insertion of one or more unnatural amino acid according to the invention, e.g., to alter the protein with respect to one or more therapeutic, diagnostic or enzymatic properties of interest. Examples of therapeutically relevant properties include serum half-life, shelf half-life, stability, immunogenicity, therapeutic activity, detectability (e.g., by the inclusion of reporter groups (e.g., labels or label binding sites) in the unnatural amino acids), reduction of LD₅₀ or other side effects, ability to enter the body through the gastric tract (e.g., oral availability), or the like. Examples of diagnostic properties include shelf half-life, stability, diagnostic activity, detectability, or the like. Examples of relevant enzymatic properties include shelf half-life, stability, enzymatic activity, production capability, or the like.

A variety of other proteins can also be modified to include one or more unnatural amino acid of the invention. For example, the invention can include substituting one or more natural amino acids in one or more vaccine proteins with an unnatural amino acid, e.g., in proteins from infectious fungi, e.g., *Aspergillus, Candida* species; bacteria, particularly *E. coli,* which serves a model for pathogenic bacteria, as well as medically important bacteria such as *Staphylococci* (e.g., *aureus),* or *Streptococci* (e.g., *pneumoniae);* protozoa such as sporozoa (e.g., *Plasmodia), rhizopods* (e.g., *Entamoeba)* and flagellates (*Trypanosoma, Leishmania, Trichomonas, Giardia,* etc.); viruses such as ( + ) RNA viruses (examples include Poxviruses e.g., vaccinia; Picornaviruses, *e.g. polio;* Togaviruses, e.g., *rubella;* Flaviviruses, e.g., HCV; and Coronaviruses), (-) RNA viruses (e.g., Rhabdoviruses, e.g., VSV; Paramyxovimses, e.g., RSV; Orthomyxovimses, e.g., influenza; Bunyaviruses; and Arenaviruses), dsDNA viruses (Reoviruses, for example), RNA to DNA viruses, i.e., Retroviruses, e.g., HIV and HTLV, and certain DNA to RNA viruses such as Hepatitis B.

Agriculturally related proteins such as insect resistance proteins (e.g., the Cry proteins), starch and lipid production enzymes, plant and insect toxins, toxin-resistance proteins, Mycotoxin detoxification proteins, plant growth enzymes (e.g., Ribulose 1,5-Bisphosphate Carboxylase/Oxygenase, "RUBISCO"), lipoxygenase (LOX), and Phosphoenolpyruvate (PEP) carboxylase are also suitable targets for unnatural amino acid modification.

The invention also provides methods for producing in a vertebrate cell at least one protein comprising at least one unnatural amino acid (and proteins produced by such methods). For example, a method includes: growing, in an appropriate medium, a vertebrate cell that comprises a nucleic acid that comprises at least one selector codon and encodes the protein. The vertebrate cell also comprises: an orthogonal tRNA (O-tRNA) that functions in the-cell and recognizes the selector codon; and an orthogonal aminoacyl tRNA synthetase (O-RS) that preferentially aminoacylates the O-tRNA with the unnatural amino acid, and the medium comprises an unnatural amino acid.

In one embodiment, the O-RS aminoacylates the O-tRNA with the unnatural amino acid at least 50% as efficiently as does an O-RS having an amino acid sequence, e.g., as set forth in SEQ ID NO.: 86 or 45. In another embodiment, the O-tRNA comprises, is processed from, or is encoded by SEQ ID NO.: 65 or 64, or a complementary polynucleotide sequence thereof. In yet another embodiment, the O-RS comprises an amino acid set forth in any one of SEQ ID NO.: 36-63 and/or 86.

The encoded protein can comprise, e.g., a therapeutic protein, a diagnostic protein, an industrial enzyme, or portion thereof. Optionally, the protein that is produced by the method is further modified through the unnatural amino acid. For example, the protein produced by the method is optionally modified by at least one post-translational modification in vivo.

Methods of producing a screening or selecting transcriptional modulator protein are also described (and screening or selecting transcriptional modulator proteins produced by such methods). For example, a method includes: selecting a first polynucleotide sequence, where the polynucleotide sequence encodes a nucleic acid binding domain; and mutating the first polynucleotide sequence to include at least one selector codon. This provides a screening or selecting polynucleotide sequence. The method also includes: selecting a second polynucleotide sequence, where the second polynucleotide sequence encodes a transcriptional activation domain; providing a construct that comprises the screening or selecting polynucleotide sequence operably linked to the second polynucleotide sequence; and, introducing the construct, an unnatural amino acid, an orthogonal tRNA synthetase (O-RS) and an orthogonal tRNA (O-tRNA) into a cell. With these components, the O-RS preferentially aminoacylates the O-tRNA with the unnatural amino acid and the O-tRNA recognizes the selector codon and incorporates the unnatural amino acid into the nucleic acid binding domain, in response to the selector codon in the screening or selecting polynucleotide sequence, thereby providing the screening or selecting transcriptional modulator protein.

In certain embodiments, the protein or polypeptide of interest (or portion thereof) in the methods and/or compositions of the invention is encoded by a nucleic acid. Typically, the nucleic acid comprises at least one selector codon, at least two selector codons, at least three selector codons, at least four selector codons, at least five selector codons, at least six selector codons, at least seven selector codons, at least eight selector codons, at least nine selector codons, ten or more selector codons.

Genes coding for proteins or polypeptides of interest can be mutagenized using methods well-known to one of skill in the art and described herein under "Mutagenesis and Other Molecular Biology Techniques" to include, e.g., one or more selector codon for the incorporation of an unnatural amino acid. For example, a nucleic acid for a protein of interest is mutagenized to include one or more selector codon, providing for the insertion of the one or more unnatural amino acids. The invention includes any such variant, e.g., mutant, versions of any protein, e.g., including at least one unnatural amino acid. Similarly, the invention also includes corresponding nucleic acids, i.e., any nucleic acid with one or more selector codon that encodes one or more unnatural amino acid.

Purifying recombinant proteins comprising unnatural amino acids

Proteins of the invention, e.g., proteins comprising unnatural amino acids, antibodies to proteins comprising unnatural amino acids, etc., can be purified, either partially or substantially to homogeneity, according to standard procedures known to and used by those of skill in the art. Accordingly, polypeptides of the invention can be recovered and purified by any of a number of methods well known in the art, including, e.g., ammonium sulfate or ethanol precipitation, acid or base extraction, column chromatography, affinity column chromatography, anion or cation exchange chromatography, phosphocellulose chromatography, hydrophobic interaction chromatography, hydroxylapatite chromatography, lectin chromatography, gel electrophoresis and the like. Protein refolding steps can be used, as desired, in making correctly folded mature proteins. High performance liquid chromatography (HPLC), affinity chromatography or other suitable methods can be employed in final purification steps where high purity is desired. In one embodiment, antibodies made against unnatural amino acids (or proteins comprising unnatural amino acids) are used as purification reagents, e.g., for affinity-based purification of proteins comprising one or more unnatural amino acid(s). Once purified, partially or to homogeneity, as desired, the polypeptides are optionally used e.g., as assay components, therapeutic reagents or as immunogens for antibody production.

In addition to other references noted herein, a variety of purification/protein folding methods are well known in the art, including, e.g., those set forth in R. Scopes, Protein Purification, Springer-Verlag, N.Y. (1982); Deutscher, Methods in Enzymology Vol. 182: Guide to Protein Purification, Academic Press, Inc. N.Y. (1990); Sandana (1997) Bioseparation of Proteins, Academic Press, Inc.; Bollag et al. (1996) Protein Methods. 2nd Edition Wiley-Liss, NY; Walker (1996) The Protein Protocols Handbook Humana Press, NJ, Harris and Angal (1990) Protein Purification Applications: A Practical Approach IRL Press at Oxford, Oxford, England; Harris and Angal Protein Purification Methods: A Practical Approach III Press at Oxford, Oxford, England; Scopes (1993) Protein Purification: Principles and Practice 3rd Edition Springer Verlag, NY; Janson and Ryden (1998) Protein Purification: Principles, High Resolution Methods and Applications, Second Edition Wiley-VCH, NY; and Walker (1998) Protein Protocols on CD-ROM Humana Press, NJ; and the references cited therein.

One advantage of producing a protein or polypeptide of interest with an unnatural amino acid in a vertebrate cell is that typically the proteins or polypeptides will be folded in their native conformations. However, in certain embodiments of the invention, those of skill in the art will recognize that, after synthesis, expression and/or purification, proteins can possess a conformation different from the desired conformations of the relevant polypeptides. In one aspect of the invention, the expressed protein is optionally denatured and then renatured. This is accomplished, e.g., by adding a chaperonin to the protein or polypeptide of interest, and/or by solubilizing the proteins in a chaotropic agent such as guanidine HCl, etc.

In general, it is occasionally desirable to denature and reduce expressed polypeptides and then to cause the polypeptides to re-fold into the preferred conformation. For example, guanidine, urea, DTT, DTE, and/or a chaperonin can be added to a translation product of interest. Methods of reducing, denaturing and renaturing proteins are well known to those of skill in the art (see, the references above, and Debinski, et al. (1993) J. Biol. Chem., 268: 14065-14070; Kreitman and Pastan (1993) Bioconjug. Chem.,4: 581-585; and Buchner, et al., (1992) Anal. Biochem., 205: 263-270). Debinski, et al., for example, describe the denaturation and reduction of inclusion body proteins in guanidine-DTE. The proteins can be refolded in a redox buffer containing, e.g., oxidized glutathione and L-arginine. Refolding reagents can be flowed or otherwise moved into contact with the one or more polypeptide or other expression product, or vice-versa.

Conservative variations

Owing to the degeneracy of the genetic code, "silent substitutions" (*i.e.,* substitutions in a nucleic acid sequence which do not result in an alteration in an encoded polypeptide) are an implied feature of *every* nucleic acid sequence which encodes an amino acid. Similarly, "conservative amino acid substitutions," in one or a few amino acids in an amino acid sequence are substituted with different amino acids with highly similar properties, are also readily identified as being highly similar to a disclosed construct. Such conservative variations of each disclosed sequence are a feature of the present invention.

"Conservative variations" of a particular nucleic acid sequence refers to those nucleic acids which encode identical or essentially identical amino acid sequences, or, where the nucleic acid does not encode an amino acid sequence, to essentially identical sequences. One of skill will recognize that individual substitutions, deletions or additions which alter, add or delete a single amino acid or a small percentage of amino acids (typically less than 5%, more typically less than 4%, 2% or 1%) in an encoded sequence are "conservatively modified variations" where the alterations result in the deletion of an amino acid, addition of an amino acid, or substitution of an amino acid with a chemically similar amino acid. Thus, "conservative variations" of a listed polypeptide sequence of the present invention include substitutions of a small percentage, typically less than 5%, more typically less than 2% or 1%, of the amino acids of the polypeptide sequence, with a conservatively selected amino acid of the same conservative substitution group. Finally, the addition of sequences that do not alter the encoded activity of a nucleic acid molecule, such as the addition of a non-functional sequence, is a conservative variation of the basic nucleic acid.

Conservative substitution tables providing functionally similar amino acids are well known in the art. The following sets forth example groups which contain natural amino acids that include "conservative substitutions" for one another.

| Conservative Substitution Groups | | | | |
|---|---|---|---|---|
| | | | | |
| 1 | Alanine (A) | Serine (S) | Threonine (T) | |
| 2 | Aspartic acid (D) | Glutamic acid (E) | | |
| 3 | Asparagine (N) | Glutamine (Q) | | |
| 4 | Arginine (R) | Lysine (K) | | |
| 5 | Isoleucine (I) | Leucine (L) | Methionine (M) | Valine (V) |
| 6 | Phenylalanine (F) | Tyrosine (Y) | Tryptophan (W) | |

Nucleic Acid Hybridization

Comparative hybridization can be used to identify nucleic acids of the invention, including conservative variations of nucleic acids of the invention, and this comparative hybridization method is a preferred method of distinguishing nucleic acids of the invention.

A test nucleic acid is said to specifically hybridize to a probe nucleic acid when it hybridizes at least ½ as well to the probe as to the perfectly matched complementary target, i.e., with a signal to noise ratio at lest ½ as high as hybridization of the probe to the target under conditions in which the perfectly matched probe binds to the perfectly matched complementary target with a signal to noise ratio that is at least about 5x-10x as high as that observed for hybridization to any of the unmatched target nucleic acids.

Nucleic acids "hybridize" when they associate, typically in solution. Nucleic acids hybridize due to a variety of well characterized physico-chemical forces, such as hydrogen bonding, solvent exclusion, base stacking and the like. An extensive guide to the hybridization of nucleic acids is found in Tijssen (1993) Laboratory Techniques in Biochemistry and Molecular Biology-Hybridization with Nucleic Acid Probes part I chapter 2, "Overview of principles of hybridization and the strategy of nucleic acid probe assays," (Elsevier, New York), as well as in Ausubel, *supra.* Hames and Higgins (1995) Gene Probes 1 IRL Press at Oxford University Press, Oxford, England, (Hames and Higgins 1) and Hames and Higgins (1995) Gene Probes 2 IRL Press at Oxford University Press, Oxford, England (Hames and Higgins 2) provide details on the synthesis, labeling, detection and quantification of DNA and RNA, including oligonucleotides.

An example of stringent hybridization conditions for hybridization of complementary nucleic acids which have more than 100 complementary residues on a filter in a Southern or northern blot is 50% formalin with 1 mg of heparin at 42°C, with the hybridization being carried out overnight. An example of stringent wash conditions is a 0.2x SSC wash at 65°C for 15 minutes (*see*, Sambrook, *supra* for a description of SSC buffer). Often the high stringency wash is preceded by a low stringency wash to remove background probe signal. An example low stringency wash is 2x SSC at 40°C for 15 minutes. In general, a signal to noise ratio of 5x (or higher) than that observed for an unrelated probe in the particular hybridization assay indicates detection of a specific hybridization.

"Stringent hybridization wash conditions" in the context of nucleic acid hybridization experiments such as Southern and northern hybridizations are sequence dependent, and are different under different environmental parameters. An extensive guide to the hybridization of nucleic acids is found in Tijssen (1993), *supra.* and in Hames and Higgins, 1 and 2. Stringent hybridization and wash conditions can easily be determined empirically for any test nucleic acid. For example, in determining highly stringent hybridization and wash conditions, the hybridization and wash conditions are gradually increased (e.g., by increasing temperature, decreasing salt concentration, increasing detergent concentration and/or increasing the concentration of organic solvents such as formalin in the hybridization or wash), until a selected set of criteria are met. For example, the hybridization and wash conditions are gradually increased until a probe binds to a perfectly matched complementary target with a signal to noise ratio that is at least 5x as high as that observed for hybridization of the probe to an unmatched target.

"Very stringent" conditions are selected to be equal to the thermal melting point (Tₘ) for a particular probe. The Tₘ is the temperature (under defined ionic strength and pH) at which 50% of the test sequence hybridizes to a perfectly matched probe. For the purposes of the present invention, generally, "highly stringent" hybridization and wash conditions are selected to be about 5° C lower than the Tₘ for the specific sequence at a defined ionic strength and pH.

"Ultra high-stringency" hybridization and wash conditions are those in which the stringency of hybridization and wash conditions are increased until the signal to noise ratio for binding of the probe to the perfectly matched complementary target nucleic acid is at least 10x as high as that observed for hybridization to any of the unmatched target nucleic acids. A target nucleic acid which hybridizes to a probe under such conditions, with a signal to noise ratio of at least ½ that of the perfectly matched complementary target nucleic acid is said to bind to the probe under ultra-high stringency conditions.

Similarly, even higher levels of stringency can be determined by gradually increasing the hybridization and/or wash conditions of the relevant hybridization assay. For example, those in which the stringency of hybridization and wash conditions are increased until the signal to noise ratio for binding of the probe to the perfectly matched complementary target nucleic acid is at least 10x, 20X, 50X, 100X, or 500X or more as high as that observed for hybridization to any of the unmatched target nucleic acids. A target nucleic acid which hybridizes to a probe under such conditions, with a signal to noise ratio of at least ½ that of the perfectly matched complementary target nucleic acid is said to bind to the probe under ultra-ultra-high stringency conditions.

Nucleic acids that do not hybridize to each other under stringent conditions are still substantially identical if the polypeptides which they encode are substantially identical. This occurs, e.g., when a copy of a nucleic acid is created using the maximum codon degeneracy permitted by the genetic code.

Unique subsequences

In one aspect, the disclosure provides a nucleic acid that comprises a unique subsequence in a nucleic acid selected from the sequences of O-tRNA's and O-RSs disclosed herein. The unique subsequence is unique as compared to a nucleic acid corresponding to any known O-tRNA or O-RS nucleic acid sequence. Alignment can be performed using, e.g., BLAST set to default parameters. Any unique subsequence is useful, e.g., as a probe to identify the nucleic acids of the invention.

Similarly, the disclosure includes a polypeptide which comprises a unique subsequence in a polypeptide selected from the sequences of O-RSs disclosed herein. Here, the unique subsequence is unique as compared to a polypeptide corresponding to any known polypeptide sequence.

The disclosure also provides for target nucleic acids which hybridizes under stringent conditions to a unique coding oligonucleotide which encodes a unique subsequence in a polypeptide selected from the sequences of O-RSs wherein the unique subsequence is unique as compared to a polypeptide corresponding to any of the control polypeptides (e.g., parental sequences from which synthetases of the invention were derived, e.g., by mutation). Unique sequences are determined as noted above.

Sequence comparison, identity, and homology

The terms "identical" or percent "identity," in the context of two or more nucleic acid or polypeptide sequences, refer to two or more sequences or subsequences that are the same or have a specified percentage of amino acid residues or nucleotides that are the same, when compared and aligned for maximum correspondence, as measured using one of the sequence comparison algorithms described below (or other algorithms available to persons of skill) or by visual inspection.

The phrase "substantially identical," in the context of two nucleic acids or polypeptides (*e.g*., DNAs encoding an O-tRNA or O-RS, or the amino acid sequence of an O-RS) refers to two or more sequences or subsequences that have at least about 60%, preferably 80%, most preferably 90-95% nucleotide or amino acid residue identity, when compared and aligned for maximum correspondence, as measured using a sequence comparison algorithm or by visual inspection. Such "substantially identical" sequences are typically considered to be "homologous," without reference to actual ancestry. Preferably, the "substantial identity" exists over a region of the sequences that is at least about 50 residues in length, more preferably over a region of at least about 100 residues, and most preferably, the sequences are substantially identical over at least about 150 residues, or over the full length of the two sequences to be compared.

For sequence comparison and homology determination, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (*see generally,* Ausubel *et al., infra*).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (www.ncbi.nlm.nih.govn. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff(1989) Proc. Natl. Acad. Sci. USA 89:10915).

In addition to calculating percent sequence identity, the BLAST algorithm also performs a statistical analysis of the similarity between two sequences (*see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

Mutagenesis and Other Molecular Biology Techniques

General texts which describe molecular biological techniques include Berger and Kimmel, Guide to Molecular Cloning Techniques, Methods in Enzymology volume 152 Academic Press, Inc., San Diego, CA (Berger); Sambrook et al., Molecular Cloning - A Laboratory Manual (2nd Ed.). Vol. 1-3. Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 1999) ("Ausubel")). These texts describe mutagenesis, the use of vectors, promoters and many other relevant topics related to, e.g., the generation of genes that include selector codons for production of proteins that include unnatural amino acids, orthogonal tRNA's, orthogonal synthetases, and pairs thereof.

Various types of mutagenesis are described in the disclosure e.g., to produce libraries of tRNA's, to produce libraries of synthetases, to insert selector codons that encode unnatural amino acids in a protein or polypeptide of interest. They include but are not limited to site-directed, random point mutagenesis, homologous recombination, DNA shuffling or other recursive mutagenesis methods, chimeric construction, mutagenesis using uracil containing templates, oligonucleotide-directed mutagenesis, phosphorothioate-modified DNA mutagenesis, mutagenesis using gapped duplex DNA or the like, or any combination thereof. Additional suitable methods include point mismatch repair, mutagenesis using repair-deficient host strains, restriction-selection and restriction-purification, deletion mutagenesis, mutagenesis by total gene synthesis, double-strand break repair, and the like. Mutagenesis, e.g., involving chimeric constructs, are also included in the present invention. In one aspect, mutagenesis can be guided by known information of the naturally occurring molecule or altered or mutated naturally occurring molecule, e.g., sequence, sequence comparisons, physical properties, crystal structure or the like.

The above texts and examples found herein describe these procedures. Additional information is found in the following publications and references cited within: Ling et al., Approaches to DNA mutagenesis: an overview, Anal Biochem. 254(2): 157-178 (1997); Dale et al., Oligonucleolide-directed random mutagenesis using the phosphorothioate method, Methods Mol. Biol. 57:369-374 (1996); Smith, In vitro mutagenesis, Ann. Rev. Genet. 19:423-462(1985); Botstein & Shortle, Strategies and applications ofin vitro mutagenesis, Science 229:1193-1201(1985); Carter, Site-directed mutagenesis, Biochem. J. 237:1-7 (1986); Kunkel, The efficiency of oligonuc/eotide directed mutagenesis, in Nucleic Acids & Molecular Biology (Eckstein, F. and Lilley, D.M.J. eds., Springer Verlag, Berlin)) (1987); Kunkel, Rapid and efficient site-specific mutagenesis without phenotypic selection, Proc. Natl. Acad. Sci. USA 82:488-492 (1985); Kunkel et al., Rapid and efficient site-specific mutagenesis without phenotypic selection, Methods in Enzymol. 154, 367-382 (1987); Bass et al., Mutant Trp repressors with new DNA-binding specificities, Science 242:240-245 (1988); Methods in Enzymol. 100: 468-500 (1983); Methods in Enzymol. 154: 329-350 (1987); Zoller & Smith, Oligonuc/eotide-directed mutagenesis using M13-derived vectors: an efficient and general procedure for the production of point mutations in any DNA fragment, Nucleic Acids Res. 10:6487-6500 (1982); Zoller & Smith, Oligonucleotide-directed mutagenesis of DNA fragments cloned into M13 vectors, Methods in Enzymol. 100:468-500 (1983); Zoller & Smith, Oligonucleoiide-directed mutagenesis: a simple method using two oligonucleotide primers and a single-stranded DNA template, Methods in Enzymol. 154:329-350 (1997); Taylor et al., The use of phosphorothioate-modfied DNA in restriction enzyme reactions to prepare nicked DNA, Nucl. Acids Res. 13: 8749-8764 (1985); Taylor et al., The rapid generation of oligonucleotide-directed mutations at high frequency using phosphorothioate-modified DNA, Nucl. Acids Res. 13: 8165-8787 (1985); Nakamaye & Eckstein, Inhibition of restriction endonuclease Nci I cleavage by phosphorothioare groups and its application to oligonucleotide-directed mutagenesis, Nucl. Acids Res. 14: 9679-9698 (1986); Sayers et al., Y-T Exonucleases in phosphorothioate-based oligonucleotide-directed mutagenesis, Nucl. Acids Res. 16:791-802 (1988); Sayers et al., Strand specific cleavage of phosphorothioate-containing DNA by reaction with restriction endonucleases in the presence of ethidium bromide, (1988) Nucl. Acids Res. 16: 803-814; Kramer et al., The gapped duplex DNA approach to oligonucleotide-directed mutation construction, Nucl. Acids Res. 12: 9441-9456 (1984); Kramer & Fritz Oligonucleotide-directed construction of mutations via gapped duplex DNA, Methods in Enzymol. 154:350-367 (1987); Kramer et al., Improved enzymatic in vitro reactions in the gapped duplex DNA approach to oligonucleotide-directed construction of mutations, Nucl. Acids Res. 16: 7207 (1988); Fritz et al., Oligonucleotide-directed construction of mutations: a gapped duplex DNA procedure without enzymatic reactions in vitro, Nucl. Acids Res, 16: 6987-6999 (1988); Kramer et al., Point Mismatch Repair, Cell 38:879-887 (1984); Carter et al., Improved oligonucleotide site-directed mutagenesis using M13 vectors, Nucl. Acids Res. 13: 4431-4443 (1985); Carter, Improved oligonucleotide-directed mutagenesis using M13 vectors, Methods in Enzymol. 154: 382-403 (1987); Eghtedarzadeh & Henikoff, Use of oligonucleotides to generate large deletions, Nucl. Acids Res. 14: 5115 (1986); Wells et al., Importance of hydrogen-bond formation in stabilizing the transition state of subtilisin, Phil. Trans. R. Soc. Lond. A 317: 415-423 (1986); Nambiar et al., Total synthesis and cloning of a gene coding for the ribonuclease Sprotein, Science 223: 1299-1301 (1984); Sakamar and Khorana, Total synthesis and expression of a gene for the a-subunit of bovine rod outer segment guanine nucleotide-binding protein (transducin), Nucl. Acids Res. 14: 6361-6372 (1988); Wells et al., Cassette mutagenesis: an efficient method jor generation of multiple mutations at defined sites, Gene 34:315-323 (1985); Grundström et al., Oligonucleotide-directed mutagenesis by microscale 'shot-gun'gene synthesis, Nucl. Acids Res. 13: 3305-3316 (1985); Mandecki, Oligonucleotide-directed double-strand break repair in plasmids of Escherichia coli: a method for site-specific mutagenesis, Proc. Natl. Acad. Sci. USA, 83:7177-7181 (1986); Arnold, Protein engineering for unusual environments, Current Opinion in Biotechnology 4:450-455 (1993); Sieber, et al., Nature Biotechnology, 19:456-460 (2001). W. P. C. Stemmer, Nature 370, 389-91 (1994); and, I. A. Lorimer, I. Pastan, Nucleic Acids Res. 23, 3067-8 (1995). Additional details on many of the above methods can be found in Methods in Enzymology Volume 154, which also describes useful controls for trouble-shooting problems with various mutagenesis methods.

The invention also relates to vertebrate host cells and organisms for the in vivo incorporation of an unnatural amino acid via orthogonal tRNA/RS pairs. Host cells are genetically engineered (e.g., transformed, transduced or transfected) with the polynucleotides of the invention or constructs which include a polynucleotide of the disclosure e.g., a vector of the disclosure, which can be, for example, a cloning vector or an expression vector. The vector can be, for example, in the form of a plasmid, a bacterium, a virus, a naked polynucleotide, or a conjugated polynucleotide. The vectors are introduced into cells and/or microorganisms by standard methods including electroporation (From et al., Proc. Natl. Acad. Sci. USA 82, 5824 (1985), infection by viral vectors, high velocity ballistic penetration by small particles with the nucleic acid either within the matrix of small beads or particles, or on the surface (Klein et al., Nature 327, 70-73 (1987)).

The engineered host cells can be cultured in conventional nutrient media modified as appropriate for such activities as, for example, screening steps, activating promoters or selecting transformants. These cells can optionally be cultured into transgenic organisms. Other useful references, e.g. for cell isolation and culture (e.g., for subsequent nucleic acid isolation) include Freshney (1994) Culture of Animal Cells, a Manual of Basic Technique, third edition, Wiley- Liss, New York and the references cited therein; Payne et al. (1992) Plant Cell and Tissue Culture in Liquid Systems John Wiley & Sons, Inc. New York, NY; Gamborg and Phillips (eds) (1995) Plant Cell, Tissue and Organ Culture; Fundamental Methods Springer Lab Manual, Springer-Verlag (Berlin Heidelberg New York) and Atlas and Parks (eds) The Handbook of Microbiological Media (1993) CRC Press, Boca Raton, FL.

The invention also relates to vertebrate cell lines with the ability to incorporate an unnatural amino acid or acids via orthogonal tRNA/RS pairs. These cell lines can be established using cell culture techniques known in the art on host cells which have been transformed, transduced, or transfected with the polynucleotides of the disclosure or constructs which include a polynucleotide of the disclosure. The methods of introducing exogenous nucleic acids into host cells are well known in the art, and will vary with the host cell used. Techniques include, but are not limited to, dextran-mediated transfection, calcium phosphate precipitation, calcium chloride treatment, polybrene mediated transfection, protoplast fusion, electroporation, viral or phage infection, encapsulation of the polynucleotide(s) in liposomes, and direct microinjection.

Cells may be transformed or transfected in a manner to allow either transient or stable incorporation of DNA. For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compounds that interact directly or indirectly with the antibody molecule. Alternatively, other techniques, such as some viral-mediated vector transfection techniques, well known to those in the art, can permit transient transfection of cells.

Several well-known methods of introducing target nucleic acids into cells are available, any of which can be used in the invention. These include: fusion of the recipient cells with bacterial protoplasts containing the DNA, electroporation, projectile bombardment, and infection with viral vectors (discussed further, below), etc. Bacterial cells can be used to amplify the number of plasmids containing DNA constructs of this invention. The bacteria are grown to log phase and the plasmids within the bacteria can be isolated by a variety of methods known in the art (*see*, for instance, Sambrook). In addition, a plethora of kits are commercially available for the purification of plasmids from bacteria, (see, e.g., EasyPrep™, FlexiPrep™, both from Pharmacia Biotech; StrataCleah™, from Stratagene; and, QIAprep™ from Qiagen). The isolated and purified plasmids are then further manipulated to produce other plasmids, used to transfect cells or incorporated into related vectors to infect organisms. Typical vectors contain transcription and translation terminators, transcription and translation initiation sequences, and promoters useful for regulation of the expression of the particular target nucleic acid. The vectors optionally comprise generic expression cassettes containing at least one independent terminator sequence, sequences permitting replication of the cassette in eukaryotes, or prokaryotes, or both, (e.g., shuttle vectors) and selection markers for both prokaryotic and vertebrate systems. Vectors are suitable for replication and integration in prokaryotes, eukaryotes, or preferably both. See, Giliman & Smith, Gene 8:81 (1979); Roberts, et al., Nature, 328:731 (1987); Schneider, B., et al., Protein Expr. Purif. 6435:10 (1995); Ausubel, Sambrook, Berger (*all supra*). A catalogue of Bacteria and Bacteriophages useful for cloning is provided, e.g., by the ATCC, e.g., The ATCC Catalogue of Bacteria and Bacteriogha (1992) Gherna et al. (eds) published by the ATCC. Additional basic procedures for sequencing, cloning and other aspects of molecular biology and underlying theoretical considerations are also found in Watson et al. (1992) Recombinant DNA Second Edition Scientific American Books, NY. In addition, essentially any nucleic acid (and virtually any labeled nucleic acid, whether standard or non-standard) can be custom or standard ordered from any of a variety of commercial sources, such as the Midland Certified Reagent Company (Midland, TX mcrc.com), The Great American Gene Company (Ramona, CA available on the World Wide Web at genco.com), ExpressGen Inc. (Chicago, IL available on the World Wide Web at expressgen.com), Operon Technologies Inc. (Alameda, CA) and many others.

Kits

Kits are also a feature of the invention. For example, a kit for producing a protein that comprises at least one unnatural amino acid in a cell is provided, where the kit includes a container containing a polynucleotide sequence encoding an O-tRNA, and/or an O-tRNA, and/or a polynucleotide sequence encoding an O-RS, and/or an O-RS. In one embodiment, the kit further includes at least one unnatural amino acid. In another embodiment, the kit further comprises instructional materials for producing the protein.

EXAMPLES .

The following examples are offered to illustrate, but not to limit the claimed invention. One of skill will recognize a variety of non-critical parameters that may be altered without departing from the scope of the claimed invention.

Example 1: Methods of producing and compositions of Aminoacyl-tRNA synthetases that Incorporate unnatural amino acids in vertebrate cells

The expansion of the vertebrate genetic code to include unnatural amino acids with novel physical, chemical or biological properties would provide powerful tools for analyzing and controlling protein function in these cells. Towards this goal, a general approach for the isolation of aminoacyl-tRNA synthetases that incorporate unnatural amino acids with high fidelity into proteins in response to an amber codon in Saccharomyces cerevisiae (S. cerevisiae) is described. The method is based on the activation of GAL4 responsive reporter genes, HIS3, URA3 or LacZ, by suppression of amber codons between the DNA binding domain and transcriptional activation domain of GAL4. The optimization of a GAL4 reporter for positive selection of active *Escherichia coli* tyrosyl-tRNA synthetase (EcTyrRS) variants is described. A negative selection of inactive EcTyrRS variants has also been developed with the URA3 reporter by use of a small molecule (5-fluroorotic acid (5-FOA)) added to the growth media as a 'toxic allele.' Importantly both positive and negative selections can be performed in a single yeast strain and with a range of stringencies. This can facilitate the isolation of a range of aminoacyl-tRNA synthetase (aaRS) activities from large libraries of mutant synthetases. The power of the method for isolating desired aaRS phenotypes is demonstrated by model selections.

Example 2

Site specific incorporation of pAF in mammalian cells

Plasmid constructions:

Wild-type human growth hormone (hGH) and hGH amber mutant expression vectors were constructed by ligating a DNA insert encoding hGH that has an N-terminal native secretion signal with pM1-MT vector (Roche) at Sal I and EcoR V restriction sites.

Single copy *B. stearothermophilus* tRNA expression insert which includes 5' restriction sites EcoR I and Bgl II , 5' flanking sequence of human tRNA Tyr *B. stearothermophilus tRNA* amber suppression mutant lacking 3'-CCA, 3' flanking sequence of human tRNA^{Tyr}
(GACAAGTGCGGTTTTTTTCTCCAGCTCCCGATGACTTATGGC (SEQ ID NO: 89)) and 3' restriction sites BamH I and Hind III, was constructed by overlap PCR using primers:
FTam 73: forward primer with EcoR I and Bgl II site
FTam 74: Reverse primer overlap with FTam73
FTam 75: Forward primer overlap with FTam74 and FTam 76
FTam 76: Reverse primer with BamH I and Hind III sites, overlap with FTam 75

To construct a single copy tRNA expression vector, the insert described above was digested with EcoRI/HindIII and ligated to pUC 19 vector cut with the same restriction enzymes. To construct a two-copy tRNA expression vector, the single copy insert was digested with EcoR I and BamH I and ligated to the single copy expression vector cut with EcoR I and Bgl II. The ligated product regenerates a 5' EcoR I and 3' Bgl II sites. A similar strategy can be used in an iterative fashion to construct expression vectors containing tandem copies of tRNA sequence.

The FLAG tag (DYKDDDDK) was added to the C-termini of wild-type E. *coli* Tyr tRNA synthetase and its mutants that charge non-natural amino acids. The RS gene was amplified by PCR and ligated to pcDNA3.1/Zeo(+) (Invitrogen).

Cell culture

One day prior to transfection, approximately 3.5 x10⁵ CHO K1 cells were plated in each well of a 6-well tissue culture plate (BD bioscience) in F-12 + Glutmax medium (Gibco) supplemented with 10% Fetal Bovine Serum (FBS) (Hyclone) and 100 U/ml penicillin G sodium and 100 ug/ml streptomycin sulfate (Gibco). The plates were incubated at 37°C, 5% CO₂. At 95% confluence, transfections were carried out according to the standard transfection protocol for lipofectamine 2000 (Invitrogen). In order to observe transient suppression, 1 µg of each plasmid (ie. tRNA, RS, GOI plasmids) was added to each well. After 4 hours of incubation, the transfection solution was replaced with 2 ml of growth medium (F-12 + Glutmax medium supplemented with 10% FBS serum and 100 U/ml penicillin G sodium and 100 ug/ml streptomycin sulfate). For those wells transfected with a non-natural amino acid tRNA/RS pair, 1 mM of the corresponding non-natural amino acid was added as a supplement. In most cases, experiments were performed in triplicate. The expression ofhGH was assayed after 40 hours using the Active Human Growth Hormone ELISA kit ( Diagnostic Systems Laboratories Inc.).

Results:

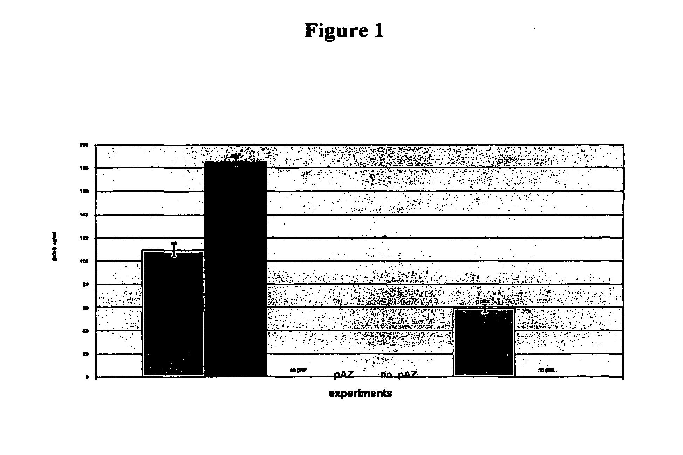

**Site specifc amber suppression with non-natural amino acids in CHO-K1. (****Figure 1****).** The non-natural amino acid dependence of amber suppression was evaluated with the hGH G131 amber mutant. CHO K1 cells were co-transfected with: a plasmid carrying 6 tandem copies of an amber-suppressing *B. stearothermophilus* tRNA mutant; a gene of interest plasmid encoding the hGH-G131 amber mutant; and a plasmid encoding the *E*. *coli* Tyr tRNA synthetase mutant that aminoacylates its cognate tRNA with tyrosine (Tyr), para-acetylphenylalanine (pAF), *para*-azidophenylalanine (pAz) and *para*benzoylphenylalanine (pBz). After a 4 hour incubation with transfection solution, the transfection solutions were replaced with growth medium +/- corresponding 1 mM non-natural amino acids. The expression of hGH was assayed after 24 hours. For pAF and pBz, full length hGH expression was only observed in the presence of the corresponding non-natural amino acid. In the absence of non-natural amino acid, no full length hGH expression were detected. In the case of amber suppression with pAZ, no hGH expression was observed in the presence and absence of pAZ. This is likely due to the inability of expression of pAZ tRNA synthetase, which was observed by anti-FLAG western blot.

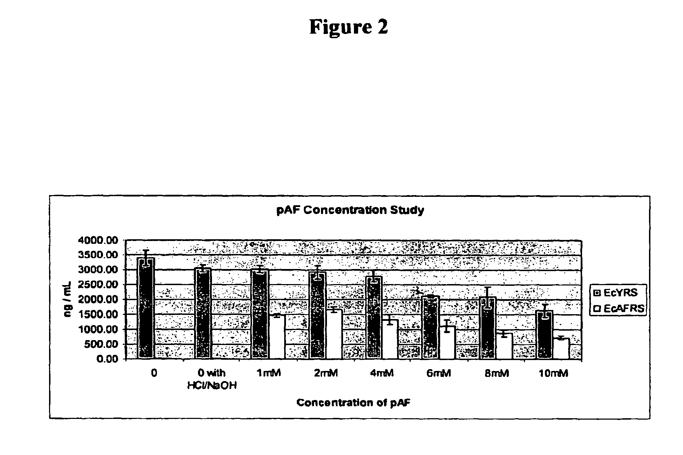

The effect of pAF concentration on amber suppression of Tyr and pAF was evaluated using the hGH E88 amber mutant. Plasmids encoding hGH E88 amber mutant, 6-copy *B* (Figure 2). *Stearothermophilus* tRNA, and the corresponding *E*. *coli* tRNA synthetases that charge Tyr and pAF were co-transfected into CHO K 1 cell. After a 4 hour incubation with transfection solution, the transfection solution was replaced with growth media with or without the addition of 1 M HCl (1:1000 relative to the growth media volume). The resulting media was next supplemented with 1, 2, 4, 6, 8 and 10 mM pAF using 1M pAF stock solution in 1 M HCl, and neutralized with an equal volume of 1 M NaOH. Expression of hGH was assayed after 42 hours. In the case of Tyr suppression, the suppression efficiency decreased slightly with the HCl/NaOH-treatment. In the case of pAF suppression, no hGH expression was detected in the absence of pAF. For both Tyr and pAF-based suppressions, the efficiency was optimum at 1 and 2 mM pAF. Suppression efficiency decreased with the increase of pAF concentration from 4 to 10 mM.

### Example 3

### Amber suppression of human Fc in suspension cells with pAF using hybrid tRNA

Plasmids containing human Fc 121 amber mutant, human tRNA and the *E*. *coli* tRNA synthetase mutant charging pAF were co-transfected into CHO-S FreeStyle suspension cells. Four copies of htRNA were used in the experiment. The expression medium contained 1mM pAF. The expression of human Fc was assayed 72 hours after transfection. Higher suppression of human Fc was detected by deceasing amount of transfected pAF specific synthetase (pAFRS).
hIgG 1-Fc2 DNA sequence:
5' IL2 signal sequence:
   ATGTACAGGATGCAACTCCTGTCTTGCATTGCACTAAGTCT (SEQ ID NO: 95)
hIgG1-Fc2 protein sequence
IL2 signal sequence:
   MYRMQLLSCIALSLALVTNS (SEQ ID NO: 97)

It is understood that the examples and embodiments described herein are for illustrative purposes only and that various modifications or changes in light thereof will be suggested to persons skilled in the art and are to be included within the scope of this application and of the appended claims.

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described herein can be used in various combinations.

**TABL 5:**

| **SEQ ID NO.:** | **Label** | **SEQUENCE** |
|---|---|---|
| SEQ ID NO.:1 | E. coli wild-type TyrRS (synthetase) polynucleotide | |
| SEQ ID NO.: 2 | E. coli wild-type TyrRS (synthetase) Amino acid (aa) | |
| SEQ ID NO.: 3 | pOMe-1 Synthetase polynucleotide | |
| | | |
| SEQ ID NO.: 4 | pOMe-2 Synthetase polynucleotide | |
| SEQ ID NO.: 5 | pOMe-3 Synthetase polynucleotide | |
| | | |
| SEQ ID NO.: 6 | pOMe-4 Synthetase polynucleotide | |
| SEQ ID NO.: 7 | pOMe-5 Synthetase polynucleotide | |
| | | |
| SEQ ID NO.: 8 | pOMe-6 (active site) Synthetase polynucleotide | |
| SEQ ID NO.: 9 | pOMe-7 (active site) Synthetase polynucteotide | |
| SEQ ID NO.: 10 | pOMe-8 (active site) Synthetase polynucleotide | |
| SEQ ID NO.:11 | pOMe-9 (active site) Synthetase polynucleotide | |
| SEQ ID NO.: 12 | pOMe-10 (active site) Synthetase polynucleotide | |
| | | |
| SEQ ID NO.:13 | pOMe-11 (active site) Synthetase polynucleotide | |
| SEQ ID NO.: 14 | pOMe-12 (active site) Synthetase polynucleotide | |
| SEQ ID NO.: 15 | pOMe-13 (active site) Synthetase polynucleotide | |
| SEQ ID NO.: 16 | pOMe-14 (active site) Synthetase polynucleotide | |
| SEQ ID NO.: 17 | p-acetylPhe-1 (active site) Synthetase | |
| | polynucleotide | |
| SEQ ID NO.: 18 | pBenzophenon -1 (active site) Synthetase polynucleotide | |
| SEQ ID NO.: 19 | pBenzophenon e-2 (active site) Synthetase polynucleotide | |
| SEQ ID NO.: 20 | pAzidoPhe-1 (active site) Synthetase polynucleotide | |
| SEQ ID NO.: 21 | pAzidoPhe-2 (active site) Synthetase polynucleotide | |
| | | |
| SEQ ID NO.: 22 | pAzidoPhe-3 (active site) Synthetase polynucleotide | |
| SEQ ID NO.: 23 | pAzidoPhe-4 (active site) Synthetase polynucleotide | |
| SEQ ID NO.: 24 | pAzidoPhe-5 (active site) Synthetase polynucleotide | |
| SEQ ID NO.: 25 | pAzidoPhe-6 (active site) Synthetase polynucleotide | |
| SEQ ID NO.: 26 | pPR-EcRS-1 (propargyloxy phenylalanine synthetase) | |
| | (active site) Synthetase polynucleotide | |
| SEQ ID NO.: 27 | pPR-EcRS -2 (active site) Synthetase polynucleotide | |
| SEQ ID NO.: 28 | pPR-EcRS -3 (active site) Synthetase polynucleotide | |
| SEQ ID NO.: 29 | pPR-EcRS -4 (active site) Synthetase polynucleotide | |
| SEQ ID NO.: 30 | pPR-EcRS-5 (active site) Synthetase polynucleotide | |
| | | |
| SEQ ID NO.:31 | pPR-EcRS-6 (active site) Synthetase polynucleotide | |
| SEQ ID NO.:32 | pPR-EcRS-7 (active site) Synthetase polynucleotide | |
| SEQ ID NO.: 33 | pPR-EcRS-8 (active site) Synthetase polynucleotide | |
| SEQ ID NO.: 34 | pPR-EcRS-9 (active site) Synthetase polynucleotide | |
| SEQ ID NO.: 35 | pPR-EcRS-10 (active site) Synthetase polynucleotide | |
| | | |
| SEQ ID NO.: 36 | p-iodoPheRS-1 Synthetase Amino acid (aa) | |
| SEQ ID NO.: 37 | p-iodoPheRS-2 Synthetase Amino acid (aa) | |
| SEQ ID NO.: 38 | p-iodoPheRS-3 Synthetase Amino acid (aa) | |
| SEQ ID NO.: 39 | OMeTyrRS-1 Synthetase Amino acid (aa) | |
| SEQ ID NO.: 40 | OMeTyrRS-2 Synthetase Amino acid (aa) | |
| SEQ ID NO.: 41 | OMeTyrRS-3 Synthetase Amino acid (aa) | |
| | | |
| SEQ ID NO.: 42 | OMeTyrRS-4 Synthetase Amino acid (aa) | |
| SEQ ID NO.: 43 | OMeTyrRS-5 Synthetase Amino acid (aa) | |
| SEQ ID NO.: 44 | OMeTyrRS-6 Synthetase Amino acid (aa) | |
| SEQ ID NO.: 45 | p-acetylPheRS-1 Synthetase Amino acid (aa) | |
| SEQ ID NO.: 46 | p-benzoylPheRS -1 Synthetase Amino acid (aa) | |
| SEQ ID NO.: 47 | p-benzoylPheRS-2 Synthetase Amino acid (aa) | |
| SEQ ID NO.: 48 | p-azidoPheRS-1 Synthetase Amino acidd (aa) | |
| | | |
| SEQ ID NO.: 49 | p-azidoPheRS-2 Synthetase Amino acid (aa) | |
| SEQ ID NO.: 50 | p-azidoPheRS-3 Synthetase Amino acid (aa) | |
| SEQ ID NO.: 51 | p-azidoPheRS-4 Synthetase Amino acid (aa) | |
| SEQ ID NO.: 52 | p-azidoPheRS-5 Synthetase Amino acid (aa) | |
| SEQ ID NO.: 53 | p-azidoPheRS-6 Synthetase Amino acid (aa) | |
| SEQ ID NO.: 54 | pPR-EcRS-1 Synthetase Amino acid (aa) *p*-*propargyloxyp henylalanine synthetase* | |
| | | |
| SEQ ID NO.: 55 | pPR-EcRS-2 Synthetase Amino acid (aa) | |
| SEQ ID NO.: 56 | pPR-EcRS-3 Synthetase Amino acid (aa) | |
| SEQ ID NO.: 57 | pPR-EcRS-44 Synthetase Amino acid (aa) | |
| SEQ ID NO.: 58 | pPR-EcRS-5 Synthetase Amino acid (aa) | |
| SEQ ID NO.: 59 | pPR-EcRS-6 Synthetase Amino acid (aa) | |
| SEQ ID NO.: 60 | pPR-EcRS-7 Synthetase Amino acid (aa) | |
| SEQ ID NO.: 61 | pPR-EcRS-8 Synthetase Amino acid (aa) | |
| | | |
| SEQ ID NO.: 62 | pPR-EcRS-9 Synthetase Amino acid (aa) | |
| SEQ ID NO.: 63 | pPR-EcRS-10 Synthetase Amino acid (aa) | |
| SEQ ID NO.: 64 | tRNA/Tyrr polynucleotide | |
| SEQ ID NO.: 65 | tRNA/Tyr | |
| SEQ ID NO.: 66 | Amber Mutants L3TAG | 5'-ATGAAGTAGCTGTCTTCTATCGAACAAGCATGCG-3' |
| SEQ ID NO.: 67 | Amber Mutants I13TAG | 5'-CGAACAAGCATGCGATTAGTGCCGACTTAAAAAG-3' |
| SEQ ID NO.: 68 | Amber Mutants T44TAG | 5'-CGCTACTCTCCCAAATAGAAAAGGTCTCCGCTG-3' |
| SEQ ID NO.: 69 | Amber Mutants F68TAG | 5'-CTGGAACAGCTATAGCTACTGATTTTTCCTCG-3' |
| SEQ ID NO.: 70 | Amber Mutants R110TAG | 5'-GCCGTCACAGATTAGTTGGCTTCAGTGGAGACTG-3' |
| SEQ ID NO.: 71 | Amberr Mutants V114TAG | 5'-GATTGGCTTCATAGGAGACTGATATGCTCTAAC-3' |
| SEQ ID NO.:72 | Amber Mutants T121TAG | 5'-GCCTCTATAGTTGAGACAGCATAGAATAATGCG-3' |
| SEQ ID NO.: 73 | Amber Mutants 1127TAG | 5'-GAGACAGCATAGATAGAGTGCGACATCATCATCGG-3' |
| SEQ ID NO.: 74 | Amber Mutants S131TAG | 5'-GAATAAGTGCGACATAGTCATCGGAAGAGAGTAGTAG-3' |
| SEQ ID NO.: 75 | Amber Mutants T145TAG | 5'-GGTCAAAGACAGTTGTAGGTATCGATTGACTCGGC-3' |
| SEQ ID NO.: 76 | Permissive Site Mutants T44F | 5'-CGCTACTCTCCCCAAATTTAAAAGGTCTCCGCTG-3' |
| SEQ ID NO.: 77 | Permissive Site Mutants T44Y | 5'-CGCTACTCTCCCCAAATATAAAAGGTCTCCGCTG-3' |
| SEQ ID NO.: 78 | Permissive Site Mutants T44W | 5'-CGCTACTCTCCCCAAATGGAAAAGGTCTCCGCTG-3' |
| SEQ ID NO.:79 | Permissive Site Mutants T44D | 5'-CGCTACTCTCCCCAAAGATAAAAGGTCTCCGCTG-3' |
| SEQ ID NO.:80 | Permissive Site Mutants T44K | 5'-CGCTACTCTCCCCAAAAAAAAAAGGTCTCCGCTG-3' |
| SEQ ID NO.: 81 | Permissive Site Mutants R110F | 5'-GCCGTCACAGATTTTTTGGCTTCAGTGGAGACTG-3' |
| SEQ ID NO.: 82 | Permissive Site Mutants R110Y | 5'-GCCGTCACAGATTATTTGGCTTCAGTGGAGACTG-3' |
| SEQ ID NO.: 83 | Permissive Site Mutants R110W | 5'-GCCGTCACAGATTGGTTGGCTTCAGTGGAGACTG-3' |
| SEQ ID NO.: 84 | Permissive Site Mutantss R110D | 5'-GCCGTCACAGATGATTTGGCTTCAGTGGAGACTG-3' |
| SEQ ID NO.: 85 | Permissive Site Mutants R110K | 5'-GCCGTCACAGATAAATTGGCT7CAGTGGAGACTG-3' |
| SEQ ID NO.:86 | p-acetylPheRS-1 Synthetase Amino acidd (aa)^{a} | |
| | A box | TRGCNNAGY |
| SEQ ID NO: 87 | B box | GGTTCGANTCC |
| SEQ ID NO: 88 | Single copy *B*. *stearothermop hilus* tRNA expression insert | |
| SEQ ID NO: 89 | *B. stearothermop hilus tRNA* amber suppression mutant | GACAAGTGCGGTTTTTTTCTCCAGCTCCCGATGACTTATGGC |
| SEQ ID NO: 90 | FTam 73: forward primer | |
| SEQ ID NO: 91 | FTam 74: Reversee primer | |
| | | |
| SEQ ID NO: 92 | FTam 75: Forward primer | |
| SEQ ID NO: 93 | FTam 76: Reverse primer | |
| SEQ ID NO: 94 | hIgG1-Fc2 DNA | |
| SEQ ID NO: 95 | 5' IL2 signal sequence | ATGTACAGGATGCAACTCCTGTCTTGCATTGCACTAAGTCT |
| SEQ ID NO: 96 | hIgG1-Fc2 protein | |
| SEQ ID NO:97 | IL 2 signal sequence | MYRMQLLSCIALSLALVTNS |

| | | |
|---|---|---|
| ^{a} These clones also contain a Asp165Gly mutation | | |

## Claims

1. A vertebrate cell or cell line comprising an orthogonal aminoacyl-tRNA synthetase (O-RS), wherein the O-RS preferentially aminoacylates an orthogonal tRNA (O-tRNA) with para-acetyl-phenylalanine (PAF) in the vertebrate cell and wherein the O-tRNA comprises the nucleotide sequence as set forth in SEQ ID NO: 88 and wherein the O-RS comprises an amino acid sequence as set forth in SEQ ID NO: 45.

2. The cell or cell line of claim 1, wherein the O-RS aminoacylates the O-tRNA with the para-amino-phenylalanine or the PAF at least 10-fold more efficiently than the O-RS aminoacylates the O-tRNA with a natural amino acid.

3. The cell or cell line of claim 1, wherein the vertebrate cell or cell line is mammalian.

4. The cell line of claim 1, wherein the vertebrate cell line is a human cell line.

5. The cell or cell line of claim 1, wherein the O-RS has one or more improved or enhanced enzymatic properties for the at least one unnatural amino acid as compared to a natural amino acid, which properties are selected from the group consisting of: higher Kₘ, lower Kₘ, higher k_{cat}, lower k_{cat}, lower k_{cat}/kₘ, and higher k_{cat}/kₘ.

6. The cell or cell line of claim 1, wherein the O-tRNA is derived from a non-vertebrate organism.

7. The cell or cell line of claim 6, wherein the non-vertebrate organism is *Escherichia coli,* or *Bacillus stearothermophilus.*

8. The cell or cell line of claim 1, further comprising a nucleic acid that comprises a polynucleotide that encodes a polypeptide of interest, wherein the polynucleotide comprises a selector codon that is recognized by the O-tRNA.

9. The cell or cell line of claim 8, wherein the selector codon is selected from the group consisting of: amber codon, ochre codon, opal codon, or four or more base codons.

10. The cell or cell line of claim 8, wherein the selector codon is amber codon.

11. The cell or cell line of claim 8, wherein the selector codon is ochre codon.

12. The cell or cell line of claim 8, wherein the selector codon is opal codon.

13. The cell or cell line of claim 8, wherein the selector codon contains four or more base codons.

14. The cell or cell line of claim 8, wherein the polypeptide of interest is a therapeutic protein, a diagnostic protein, an industrial enzyme, or portion thereof.

15. The cell or cell line of claim 8, wherein the polypeptide of interest comprises a protein or a portion of a protein selected from the group consisting of: a cytokine, a growth factor, a growth factor receptor, an interferon, an interleukin, an inflammatory molecule, an oncogene product, a peptide hormone, a signal transduction molecule, a steroid hormone receptor, erythropoietin (EPO), insulin, human growth hormone, an Alpha-1 antitrypsin, an Angiostatin, an Antihemolytic factor, an antibody, an Apolipoprotein, an Apoprotein, an Atrial natriuretic factor, an Atrial natriuretic polypeptide, an Atrial peptide, a C-X-C chemokine, T39765, NAP-2, ENA-78, a Gro-a, a Gro-b, a Gro-c, an TP-10, a GCP-2, an NAP-4, an SDF-1, a PF4, a MIG, a Calcitonin, a c-kit ligand, a cytokine, a CC chemokine, a Monocyte chemoattractant protein-1, a Monocyte chemoattractant protein-2, a Monocyte chemoattractant protein-3, a Monocyte inflammatory protein-1 alpha, a Monocyte inflammatory protein-1 beta, RANTES, 1309, R83915, R91733, HCC1, T58847, D31065, T64262, a CD40, a CD40 ligand, a C-kit Ligand, a Collagen, a Colony stimulating factor (CSF), a Complement factor 5a, a Complement inhibitor, a Complement receptor 1, a cytokine, DHFR, an epithelial Neutrophil Activating Peptide-78, a GROα/MGSA, a GROβ, a GROγ a MIP-1α, a MIP-1δ, a MCP-1, an Epidermal Growth Factor (EGF), an epithelial Neutrophil Activating Peptide, an Erythropoietin (EPO), an Exfoliating toxin, a Factor IX, a Factor VII, a Factor VIII, a Factor X, a Fibroblast Growth Factor (FGF), a Fibrinogen, a Fibronectin, a G-CSF, a GM-CSF, a Glucocerebrosidase, a Gonadotropin, a growth factor, a growth factor receptor, a Hedgehog protein, a Hemoglobin, a Hepatocyte Growth Factor (HGF), a Hirudin, a Human serum albumin, an ICAM-1, an ICAM-1 receptor, an LFA-1, an LFA-1 receptor, an Insulin, an Insulin-like Growth Factor (IGF), an IGF-1, an IGF-II, an interferon, an IFN-α, an IFN-β, an IFN-γ, an interleukin, an IL-1, an IL-2, an IL-3, an IL-4, an IL-5, an IL-6, an IL-7, an IL-8, an IL-9, an IL-10, an IL-11, an IL-12, a Keratinocyte Growth Factor (KGF), a Lactoferrin, a leukemia inhibitory factor, a Luciferase, a Neurturin, a Neutrophil inhibitory factor (NIF), an oncostatin M, an Osteogenic protein, an oncogene product, a Parathyroid hormone, a PD-ECSF, a PDGF, a peptide hormone, a Human Growth Hormone, a Pleiotropin, a Protein A, a Protein G, a Pyrogenic exotoxins A, B, or C, a Relaxin, a Renin, an SCF, a Soluble complement receptor I, a Soluble I-CAM 1, a Soluble intcrleukin receptor, a Soluble TNF receptor, a Somatomedin, a Somatostatin, a Somatotropin, a Streptokinase, a Superantigen, a Staphylococcal enterotoxins, an SEA, an SEB, an SEC1, an SEC2, an SEC3, an SED, an SEE, a steroid hormone receptor, a Superoxide dismutase (SOD), a Toxic shock syndrome toxin, a Thymosin alpha 1, a Tissue plasminogen activator, a tumor growth factor (TGF), a TGF-α, a TGF-β, a Tumor Necrosis Factor, a Tumor Necrosis Factor alpha, a Tumor necrosis factor beta, a Tumor necrosis factor receptor (TNFR), a VLA-4 protein, a VCAM-1 protein, a Vascular Endothelial Growth Factor (VEGEF), a Urokinase, a Mos, a Ras, a Raf, a Met; a p53, a Tat, a Fos, a Myc, a Jun, a Myb, a ReI, an estrogen receptor, a progesterone receptor, a testosterone receptor, an aldosterone receptor, an LDL receptor, a SCF/c-Kit, a CD40L/CD40, a VLA-4/VCAM-1, an ICAM-1/LFA-1, a hyalurin/CD44, and a corticosterone.

16. The vertebrate cell line from claim 1 wherein the cell line has been transiently transfected.

17. The vertebrate cell line from claim 1 wherein the cell line has been stably transfected.

18. A kit for producing a protein that comprises at least one unnatural amino acid in a cell, the kit comprising: a container containing a polynucleotide sequence encoding an O-tRNA, wherein the O-tRNA has a sequence as set forth in SEQ ID NO: 88, and a polynucleotide sequence encoding an O-RS, wherein the O-RS comprises an amino acid sequence as set forth in SEQ ID NO: 45.

19. The kit of claim 18, wherein the kit further comprises at least one unnatural amino acid.

20. The kit of claim 18, wherein the kit further comprises instructional materials for producing the protein.

## Patentansprüche

1. Vertebratenzelle oder Zelllinie, umfassend eine orthogonale Aminoacyl-tRNA-Synthetase (O-RS), wobei die O-RS vorzugsweise eine orthogonale tRNA (O-tRNA) mit para-Acetylphenylalanin (PAF) in der Vertebratenzelle aminoacyliert und wobei die O-tRNA die Nukleotidsequenz nach SEQ ID NO: 88 umfasst und wobei die O-RS eine Aminosäuresequenz nach SEQ ID NO: 45 umfasst.

2. Zelle oder Zelllinie nach Anspruch 1, wobei die O-RS die O-tRNA mit dem para-Acetylphenylalanin oder PAF mindestens 10-fach effizienter aminoacyliert als die O-RS die O-tRNA mit einer natürlichen Aminosäure aminoacyliert.

3. Zelle oder Zelllinie nach Anspruch 1, wobei die Vertebratenzelle oder Zelllinie aus Säugetier stammt.

4. Zelllinie nach Anspruch 1, wobei die Vertebratenzelllinie eine Zelllinie aus Mensch ist.

5. Zelle oder Zelllinie nach Anspruch 1 , wobei die O-RS eine oder mehrere verbesserte oder gesteigerte enzymatische Eigenschaften für die mindestens eine unnatürliche Aminosäure im Vergleich zu einer natürlichen Aminosäure aufweist, wobei die Eigenschaften ausgewählt sind aus der Gruppe, bestehend aus: höherem Kₘ, niedrigerem Kₘ, höherem k_{cat}, niedrigerem k_{cat}, niedrigerem k_{cat}/kₘ, und höherem k_{cat}/kₘ.

6. Zelle oder Zelllinie nach Anspruch 1, wobei die O-tRNA von einem Nicht-Vertebraten-Organismus hergeleitet ist.

7. Zelle oder Zelllinie nach Anspruch 6, wobei der Nicht-Vertebraten-Organismus *Escherichia coli,* oder *Bacillus stearothermophilus* ist.

8. Zelle oder Zelllinie nach Anspruch 1, zudem umfassend eine Nukleinsäure, die ein Polynukleotid umfasst, welches ein interessierendes Polypeptid codiert, wobei das Polynukleotid ein Selektorcodon umfasst, das von der O-tRNA erkannt wird.

9. Zelle oder Zelllinie nach Anspruch 8, wobei das Selektorcodon ausgewählt ist aus der Gruppe, bestehend aus: Amber-Codon, Ochre-Codon, Opal-Codon, oder vier-oder mehrbasigen Codons.

10. Zelle oder Zelllinie nach Anspruch 8, wobei das Selektorcodon ein Amber-Codon ist.

11. Zelle oder Zelllinie nach Anspruch 8, wobei das Selektorcodon ein Ochre-Codon ist.

12. Zelle oder Zelllinie nach Anspruch 8, wobei das Selektorcodon ein Opal-Codon ist.

13. Zelle oder Zelllinie nach Anspruch 8, wobei das Selektorcodon vier- oder mehrbasige Codons enthält.

14. Zelle oder Zelllinie nach Anspruch 8, wobei das interessierende Polypeptid ein therapeutisches Protein, ein diagnostisches Protein, einem industrielles Enzym, oder ein Teil davon ist.

15. Zelle oder Zelllinie nach Anspruch 8, wobei das interessierende Polypeptid ein Protein oder einen Teil eines Proteins umfasst, ausgewählt aus der Gruppe, bestehend aus: einem Cytokin, einem Wachstumsfaktor, einem Wachstumsfaktorrezeptor, einem Interferon, einem Interleukin, einem inflammatorischen Molekül, einem Onkogenprodukt, einem Peptidhormon, einem Signaltransduktionsmolekül, einem Steroidhormonrezeptor, Erythropoietin (EPO), Insulin, humanem Wachstumshormon, einem Alpha-lAntitrypsin, einem Angiostatin, einem Antihämolysefaktor, einem Antikörper, einem Apolipoprotein, einem Apoprotein, einem Atrialen natriuretischen Faktor, einem atrialen natriuretischen Polypeptid, einem atrialen Peptid, einem C-X-C Chemokin, T39765, NAP-2, ENA-78, einem Gro-a, einem Gro-b, einem Gro-c, einem IP-10, einem GCP-2, einem NAP-4, einem SDF-1, einem PF4, einem MIG, einem Calcitonin, einem c-Kit-Ligand, einem Cytokin, einem CC Chemokin, einem Monocyten-Chemoattractant-Protein-1, einem Monocyten-Chemoattractant-Protein-2, einem Monocyten-Chemoattractant-Protein-3, einem Monocyteninflammatorischen Protein-1 alpha, einem Monocyteninflammatorischen Protein-1 beta, RANTES, 1309, R83915, R91733, HCC1, T58847, D31065, T64262, einem CD40, einem CD40-Ligand, einem C-Kit-Ligand, einem Collagen, einem Koloniestimulierenden Faktor (CSF), einem Komplementfaktor 5a, einem Komplementinhibitor, einem Komplementrezeptor 1, einem Cytokin, DHFR, einem epithelialen Neutrophilen-Aktivierungs-Peptid-78, einem GROα/MGSA, einem GROß, einem GROγ einem MIP-1α, einem MIP-1δ, einem MCP-1, einem epidermalen Wachstumsfaktor (EGF), einem epithelialen Neutrophilen-Aktivierungspeptid, einem Erythropoietin (EPO), einem exfoliativen Toxin, einem Faktor IX, einem Faktor VII, einem Faktor VIII, einem Faktor X, einem Fibroblasten-Wachstumsfaktor (FGF), einem Fibrinogen, einem Fibronectin, einem G-CSF, einem GM-CSF, einer Glucocerebrosidase, einem Gonadotropin, einem Wachstumsfaktor, einem Wachstumsfaktorrezeptor, einem Hedgehog-Protein, einem Hämoglobin, einem Hepatocyten-Wachstumsfaktor (HGF), einem Hirudin, einem Human-Serumalbumin, einem ICAM-1, einem ICAM-1-Rezeptor, einem LFA-1, einem LFA-1 Rezeptor, einem Insulin, einem Insulin-artigen Wachstumsfaktor (IGF), einem IGF-I, einem IGF-II, einem Interferon, einem IFN-α, einem IFN-β, einem IFN-γ, einem Interleukin, einem IL-1, einem IL-2, einem IL-3, einem IL-4, einem IL-5, einem IL-6, einem IL-7, einem IL-8, einem IL-9, einem IL-10, einem IL-11, einem IL- 12, einem Keratinocyten-Wachstumsfaktor (KGF), einem Lactoferrin, einem leukämieinhibitorischen Faktor, einer Luciferase, einem Neurturin, einem Neutrophilen-inhibitorischen Faktor (NIF), einem Oncostatin M, einem osteogenen Protein, einem Oncogenproduct, einem Parathyroidhormon, einem PD-ECSF, einem PDGF, einem Peptidhormon, einem Human-Wachstumsfaktor, einem Pleiotropin, einem Protein A, einem Protein G, pyrogenen Exotoxinen A, B, oder C, einem Relaxin, einem Renin, einem SCF, einem löslichen Komplementrezeptor I, einem löslichen I-CAM 1, einem löslichen Interleukinrezeptor, einem löslichen TNF-Rezeptor, einem Somatomedin, einem Somatostatin, einem Somatotropin, einer Streptokinase, einem Superantigen, Staphylococcus-Enterotoxinen, einem SEA, einem SEB, einem SEC1, einem SEC2, einem SEC3, einem SED, einem SEE, einem Steroidhormonrezeptor, einer Superoxiddismutase (SOD), einem Toxic Shock Syndrom-Toxin, einem Thymosin alpha 1, einem Gewebeplasminogenaktivator, einem Tumor-Wachstumsfaktor (TGF), einem TGF-α, einem TGF-β, einem Tumornekrosefaktor, einem Tumornekrosefaktor alpha, einem Tumornekrosefaktor beta, einem Tumornekrosefaktorrezeptor (TNFR), einem VLA-4 Protein, einem VCAM-1 Protein, einem Gefäßendothel-Wachstumsfaktor (VEGEF), einer Urokinase, einem Mos, einem Ras, einem Raf, einem Met; einem p53, einem Tat, einem Fos, einem Myc, einem Jun, einem Myb, einem Rel, einem Östrogenrezeptor, einem Progesteronrezeptor, einem Testosteronrezeptor, einem Aldosteronrezeptor, einem LDL-Rezeptor, einem SCF/c-Kit, einem CD40L/CD40, einem VLA-4/VCAM-1, einem ICAM-1 /LFA-1, einem Hyalurin/CD44, und einem Corticosteron.

16. Vertebratenzelllinie nach Anspruch 1, wobei die Zelllinie transient transfiziert wurde.

17. Vertebratenzelllinie nach Anspruch 1, wobei die Zelllinie stabil transfiziert wurde.

18. Kit zur Herstellung eines Proteins, das mindestens eine unnatürliche Aminosäure in einer Zelle umfasst, wobei das Kit umfasst: einen Behälter mit einer Polynukleotidsequenz, die eine O-tRNA codiert, wobei die O-tRNA eine Sequenz nach SEQ 10 NO: 88 hat, und einer Polynukleotidsequenz, die eine O-RS codiert, wobei die O-RS eine Aminosäuresequenz nach SEQ ID NO: 45 umfasst.

19. Kit nach Anspruch 18, wobei das Kit zudem mindestens eine unnatürliche Aminosäure umfasst.

20. Kit nach Anspruch 18, wobei das Kit zudem Anweisungsmaterialien zum Herstellen des Proteins umfasst.

## Revendications

1. Cellule ou lignée de cellules de vertébré comprenant une aminoacyl-ARNt orthogonal synthétase (ORS), dans laquelle la O-RS aminoacyle préférentiellement un ARNt orthogonal (O-ARNt) avec une para-acétyl-phénylalanine (PAF) dans la cellule de vertébré et dans laquelle le O-ARNt comprend la séquence nucléotidique telle qu'indiquée dans la SEQ ID n° : 88 et dans laquelle la O-RS comprend une séquence d'acides aminés telle qu'indiquée dans la SEQ ID n° : 45.

2. Cellule ou lignée de cellules selon la revendication 1, dans laquelle la O-RS aminoacyle le O-ARNt avec la para-amino-phénylalanine ou la PAF d'une manière au moins 10 fois plus efficace que quand la ORS aminoacyle le O-ARNt avec un acide aminé naturel.

3. Cellule ou lignée de cellules selon la revendication 1, dans laquelle la cellule ou la lignée de cellules de vertébré est mammalienne.

4. Lignée de cellules selon la revendication 1, dans laquelle la lignée de cellules est une lignée de cellules humaines.

5. Cellule ou lignée de cellules selon la revendication 1, dans laquelle la O-RS a une ou plusieurs propriété(s) enzymatique(s) améliorée(s) ou renforcée(s) en ce qui concerne l'acide aminé non naturel étant au nombre de un au moins, en comparaison avec un acide aminé naturel, ces propriétés étant choisies dans le groupe constitué par : une Kₘ plus élevée ; une Kₘ moins élevée ; une K_{cat} plus élevée ; une K_{cat} moins élevée ; un K_{cat}/Kₘ moins élevé ; et un K_{cat}/Kₘ plus élevé.

6. Cellule ou lignée de cellules selon la revendication 1, dans laquelle le O-ARNt est dérivé d'un organisme n'étant pas un vertébré.

7. Cellule ou lignée de cellules selon la revendication 6, dans laquelle l'organisme qui n'est pas un vertébré est *Escherichia coli* ou *Bacillus stearothermophilus.*

8. Cellule ou lignée de cellules, selon la revendication 1, comprenant en outre un acide nucléique, qui comprend un polynucléotide codant pour un polypeptide d'intérêt, dans lequel le polynucléotide comprend un codon sélecteur qui est reconnu par le O-ARNt.

9. Cellule ou lignée de cellules selon la revendication 8, dans laquelle le codon sélecteur est choisi dans le groupe constitué par : un codon ambre ; un codon ocre ; un codon opale ; ou quatre codons de base ou plus.

10. Cellule ou lignée de cellules selon la revendication 8, dans laquelle le codon sélecteur est le codon ambre.

11. Cellule ou lignée de cellules selon la revendication 8, dans laquelle le codon sélecteur est le codon ocre.

12. Cellule ou lignée de cellules selon la revendication 8, dans laquelle le codon sélecteur est le codon opale.

13. Cellule ou lignée de cellules selon la revendication 8, dans laquelle le codon sélecteur contient quatre codons de base ou plus.

14. Cellule ou lignée de cellules selon la revendication 8, dans laquelle le polypeptide d'intérêt est une protéine thérapeutique, une protéine diagnostique, une enzyme industrielle ou une partie de celles-ci.

15. Cellule ou lignée de cellules selon la revendication 8, dans laquelle le polypeptide d'intérêt comprend une protéine ou une partie de protéine choisie dans le groupe constitué par : une cytokine ; un facteur de croissance ; un récepteur d'un facteur de croissance ; un interféron ; une interleukine ; une molécule inflammatoire ; un produit oncogène ; une hormone peptidique ; une molécule de transduction du signal ; un récepteur d'hormone stéroïdienne ; une érythropoïétine (EPO) ; l'insuline ; l'hormone de croissance humaine ; une antitrypsine alpha-1 ; une angiostatine ; un facteur anti-hémolytique ; un anticorps ; une apo-lipoprotéine ; une apoprotéine ; un facteur atrial natriurétique ; un polypeptide atrial natriurétique ; un peptide atrial ; une chimiokine C-X-C ; le T39765 ; une NAP-2 ; une ENA-78 ; un Gro-a ; un Gro-b ; un Gro-c ; une IP-10 ; une GCP-2 ; une NAP-4 ; un SDF-1 ; un PF4 ; un MIG ; une calcitonine ; un ligand c-kit ; une cytokine ; une chimiokine CC ; une protéine-1 chimiotactique des monocytes ; une protéine-2 chimiotactique des monocytes ; une protéine-3 chimiotactique des monocytes ; une protéine-1 alpha inflammatoire des monocytes ; une protéine-1 bêta inflammatoire des monocytes ; une RANTES ; un 1309 ; un R83915 ; un R91733 ; un HCC1 ; un T58847 ; un D31065 ; un T64262 ; un CD40 ; un ligand de CD40 ; un ligand C-kit ; un collagène ; un facteur de stimulation des colonies (CSF) ; un facteur 5a du Complément ; un inhibiteur du Complément ; un récepteur 1 du Complément ; une cytokine ; un DHFR ; un peptide 78 activateur des polynucléaires neutrophiles épithéliaux ; un GROα-MGSA ; un GROβ ; un GROγ ; un MIP-1α ; un MIP-1δ ; un MCP-1 ; un facteur de croissance épidermique (EGF) ; un peptide activateur des polynucléaires neutrophiles épithéliaux ; une érythropoïétine (EPO) ; une toxine exfoliante ; un facteur IX ; un facteur VII ; un facteur VIII ; un facteur X ; un facteur de croissance des fibroblastes (FGF) ; un fibrinogène ; une fibronectine ; un G-CSF ; un GM-CSF ; une gluco-cérébrosidase ; une gonadostimuline ; un facteur de croissance ; un récepteur d'un facteur de croissance ; une protéine hérisson ; une hémoglobine ; un facteur de croissance des hépatocytes (HGF) ; une hirudine ; une sérumalbumine humaine ; un ICAM-1 ; un récepteur d'ICAM-1 ; un LFA-1 ; un récepteur de LFA-1 ; une insuline ; un facteur de croissance du type insuline (IGF) ; un IGF-I ; un IGF-II ; un interféron ; un IFN-α ; un IFN-β ; un IFN-γ ; une interleukine ; une IL-1 ; une IL-2 ; une IL-3 ; une IL-4 ; une IL-5 ; une IL-6 ; une IL-7 ; une IL-8 ; une IL-9 ; une IL-10 ; une IL-11 ; une IL-12 ; un facteur de croissance des kératinocytes (KGF) ; une lactoferrine ; un facteur d'inhibition de la leucémie ; une luciférase ; une neurturine ; un facteur d'inhibition des polynucléaires neutrophiles (NIF) ; une oncostatine M ; une protéine ostéogène ; un produit oncogène ; l'hormone parathyroïdienne ; un PD-ECSF ; un PDGF ; une hormone peptidique ; une hormone de croissance humaine ; une pléiotropine ; une protéine A ; une protéine G ; les exotoxines pyrogènes A, B ou C ; une relaxine ; une rénine ; un SCF ; un récepteur I du Complément soluble ; un I-CAM 1 soluble ; un récepteur d'interleukine soluble ; un récepteur de TNF soluble ; une somatomédine ; une somatostatine ; une somatotropine ; une streptokinase ; un Superantigène ; des entérotoxines staphylococciques ; un SEA ; un SEB ; un SEC1 ; un SEC2 ; un SEC3 ; un SED ; un SEE ; un récepteur d'hormone stéroïdienne ; une superoxyde dismutase (SOD) ; une toxine du syndrome du choc toxique ; une thymosine alpha-1 ; un activateur du plasminogène tissulaire ; un facteur de croissance tumorale (TGF) ; un TGF-α ; un TGF-β ; un facteur de nécrose tumorale ; un facteur alpha de nécrose tumorale ; un facteur bêta de nécrose tumorale ; un récepteur d'un facteur de nécrose tumorale (TNFR) ; une protéine VLA-4 ; une protéine VCAM-1 ; un facteur de croissance endothélial vasculaire (VEGEF) ; une urokinase ; une Mos ; une Ras ; une Raf ; une Met ; un p53 ; un Tat ; un Fos ; un Myc ; un Jun ; un Myb ; un Rel ; un récepteur d'oestrogènes; un récepteur de progestérone ; un récepteur de testostérone ; un récepteur d'aldostérone ; un récepteur de LDL ; un SCF/c-Kit ; un CD40L/CD40 ; une VLA-4/VCAM-1 ; un ICAM-1/LFA-1 ; une hyalurine/CD44 ; et une corticostérone.

16. Lignée de cellules de vertébré provenant de la revendication 1, dans laquelle la lignée de cellules a été transfectée de manière transitoire.

17. Lignée de cellules de vertébré provenant de la revendication 1, dans laquelle la lignée de cellules a été transfectée de manière stable.

18. Trousse de production d'une protéine qui comprend au moins un acide aminé n'étant pas naturel dans une cellule, la trousse comprenant : un conteneur, contenant une séquence de polynucléotides codant pour un O-ARNt, dans laquelle le O-ARNt a une séquence telle qu'indiquée dans la SEQ ID n° : 88, et une séquence de polynucléotides codant pour une O-RS, dans laquelle la O-RS comprend une séquence d'acides aminés telle qu'indiquée dans la SEQ ID n° : 45.

19. Trousse selon la revendication 18, dans laquelle la trousse comprend en outre au moins un acide aminé n'étant pas naturel.

20. Trousse selon la revendication 18, dans laquelle la trousse comprend en outre du matériel d'instruction destiné à produire la protéine.


### SEQUENCE LISTING

<110> Feng, Tian
   Norman, Thea
   Chu, Stephanie
<120> SITE SPECIFIC INCORPORATION OF NON-NATURAL AMINO ACIDS BY VERTEBRATE CELLS
<130> AMBX-0123.00PCT
<150> 60/843,473
   <151> 2006-09-08
<160> 97
<170> PatentIn version 3.4
<210> 1
   <211> 1275
   <212> DNA
   <213> Escherichia coli
<400> 1
   atggcaagca gtaacttgat taaacaattg caagagcggg ggctggtagc ccaggtgacg     60
   gacgaggaag cgttagcaga gcgactggcg caaggcccga tcgcgctcta ttgcggcttc    120
   gatcctaccg ctgacagctt gcatttgggg catcttgttc cattgttatg cctgaaacgc    180
   ttccagcagg cgggccacaa gccggttgcg ctggtaggcg gcgcgacggg tctgattggc    240
   gacccgagct tcaaagctgc cgagcgtaag ctgaacaccg aagaaactgt tcaggagtgg    300
   gtggacaaaa tccgtaagca ggttgccccg ttcctcgatt tcgactgtgg agaaaactct    360
   gctatcgcgg cgaacaacta tgactggttc ggcaatatga atgtgctgac cttcctgcgc    420
   gatattggca aacacttctc cgttaaccag atgatcaaca aagaagcggt taagcagcgt    480
   ctcaaccgtg aagatcaggg gatttcgttc actgagtttt cctacaacct gttgcagggt    540
   tatgacttcg cctgtctgaa caaacagtac ggtgtggtgc tgcaaattgg tggttctgac    600
   cagtggggta acatcacttc tggtatcgac ctgacccgtc gtctgcatca gaatcaggtg    660
   tttggcctga ccgttccgct gatcactaaa gcagatggca ccaaatttgg taaaactgaa    720
   ggcggcgcag tctggttgga tccgaagaaa accagcccgt acaaattcta ccagttctgg    780
   atcaacactg cggatgccga cgtttaccgc ttcctgaagt tcttcacctt tatgagcatt    840
   gaagagatca acgccctgga agaagaagat aaaaacagcg gtaaagcacc gcgcgcccag    900
   tatgtactgg cggagcaggt gactcgtctg gttcacggtg aagaaggttt acaggcggca    960
   aaacgtatta ccgaatgcct gttcagcggt tctttgagtg cgctgagtga agcggacttc   1020
   gaacagctgg cgcaggacgg cgtaccgatg gttgagatgg aaaagggcgc agacctgatg   1080
   caggcactgg tcgattctga actgcaacct tcccgtggtc aggcacgtaa aactatcgcc   1140
   tccaatgcca tcaccattaa cggtgaaaaa cagtccgatc ctgaatactt ctttaaagaa   1200
   gaagatcgtc tgtttggtcg ttttacctta ctgcgtcgcg gtaaaaagaa ttactgtctg   1260
   atttgctgga aataa                                                     1275
<210> 2
   <211> 424
   <212> PRT
   <213> Escherichia coli
<400> 2
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
               20                  25                  30
   Pro Ile Ala Leu Tyr Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Asp Phe Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                 320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
           355                 360                 365
   Gln Pro Ser Arg Gly Gln Ala Arg Lys Thr Ile Ala Ser Asn Ala Ile
       370                 375                 380
   Thr Ile Asn Gly Glu Lys Gln Ser Asp Pro Glu Tyr Phe Phe Lys Glu
   385                 390                 395                 400
   Glu Asp Arg Leu Phe Gly Arg Phe Thr Leu Leu Arg Arg Gly Lys Lys
                   405                 410                 415
   Asn Tyr Cys Leu Ile Cys Trp Lys
               420
<210> 3
   <211> 1275
   <212> DNA
   <213> Artificial
<220>
   <223> artificial synthetase
<400> 3
   atggcaagca gtaacttgat taaacaattg caagagcggg ggctggtagc ccaggtgacg      60
   gacgaggaag cgttagcaga gcgactggcg caaggcccga tcgcactcgt gtgtggcttc     120
   gatcctaccg ctgacagctt gcatttgggg catcttgttc cattgttatg cctgaaacgc     180
   ttccagcagg cgggccacaa gccggttgcg ctggtaggcg gcgcgacggg tctgattggc     240
   gacccgagct tcaaagctgc cgagcgtaag ctgaacaccg aagaaactgt tcaggagtgg 300
   gtggacaaaa tccgtaagca ggttgccccg ttcctcgatt tcgactgtgg agaaaactct 360
   gctatcgcgg ccaataatta tgactggttc ggcaatatga atgtgctgac cttcctgcgc 420
   gatattggca aacacttctc cgttaaccag atgatcaaca aagaagcggt taagcagcgt 480
   ctcaaccgtg aagatcaggg gatttcgttc actgagtttt cctacaacct gctgcagggt 540
   tatagtatgg cctgtttgaa caaacagtac ggtgtggtgc tgcaaattgg tggttctgac 600
   cagtggggta acatcacttc tggtatcgac ctgacccgtc gtctgcatca gaatcaggtg 660
   tttggcctga ccgttccgct gatcactaaa gcagatggca ccaaatttgg taaaactgaa 720
   ggcggcgcag tctggttgga tccgaagaaa accagcccgt acaaattcta ccagttctgg 780
   atcaacactg cggatgccga cgtttaccgc ttcctgaagt tcttcacctt tatgagcatt 840
   gaagagatca acgccctgga agaagaagat aaaaacagcg gtaaagcacc gcgcgcccag 900
   tatgtactgg cggagcaggt gactcgtctg gttcacggtg aagaaggttt acaggcggca 960
   aaacgtatta ccgaatgcct gttcagcggt tctttgagtg cgctgagtga agcggacttc 1020
   gaacagctgg cgcaggacgg cgtaccgatg gttgagatgg aaaagggcgc agacctgatg 1080
   caggcactgg tcgattctga actgcaacct tcccgtggtc aggcacgtaa aactatcgcc 1140
   tccaatgcca tcaccattaa cggtgaaaaa cagtccgatc ctgaatactt ctttaaagaa 1200
   gaagatcgtc tgtttggtcg ttttacctta ctgcgtcgcg gtaaaaagaa ttactgtctg 1260
   atttgctgga aataa 1275
<210> 4
   <211> 1275
   <212> DNA
   <213> Artificial
<220>
   <223> artificial synthetase
<400> 4
   atggcaagca gtaacttgat taaacaattg caagagcggg ggctggtagc ccaggtgacg 60
   gacgaggaag cgttagcaga gcgactggcg caaggcccga tcgcactcac ttgtggcttc 120
   gatcctaccg ctgacagctt gcatttgggg catcttgttc cattgttatg cctgaaacgc 180
   ttccagcagg cgggccacaa gccggttgcg ctggtaggcg gcgcgacggg tctgattggc 240
   gacccgagct tcaaagctgc cgagcgtaag ctgaacaccg aagaaactgt tcaggagtgg 300
   gtggacaaaa tccgtaagca ggttgccccg ttcctcgatt tcgactgtgg agaaaactct 360
   gctatcgcgg ccaataatta tgactggttc agcaatatga atgtgctgac cttcctgcgc 420
   gatattggca aacacttctc cgttaaccag atgatcaaca aagaagcggt taagcagcgt 480
   ctcaaccgtg aagatcaggg gatttcgttc actgagtttt cctacaacct gctgcagggt 540
   tatacgtatg cctgtctgaa caaacagtac ggtgtggtgc tgcaaattgg tggttctgac 600
   cagtggggta acatcacttc tggtatcgac ctgacccgtc gtctgcatca gaatcaggtg 660
   tttggcctga ccgttccgct gatcactaaa gcagatggca ccaaatttgg taaaactgaa 720
   ggcggcgcag tctggttgga tccgaagaaa accagcccgt acaaattcta ccagttctgg 780
   atcaacactg cggatgccga cgtttaccgc ttcctgaagt tcttcacctt tatgagcatt 840
   gaagagatca acgccctgga agaagaagat aaaaacagcg gtaaagcacc gcgcgcccag 900
   tatgtactgg cggagcaggt gactcgtctg gttcacggtg aagaaggttt acaggcggca 960
   aaacgtatta ccgaatgcct gttcagcggt tctttgagtg cgctgagtga agcggacttc 1020
   gaacagctgg cgcaggacgg cgtaccgatg gttgagatgg aaaagggcgc agacctgatg 1080
   caggcactgg tcgattctga actgcaacct tcccgtggtc aggcacgtaa aactatcgcc 1140
   tccaatgcca tcaccattaa cggtgaaaaa cagtccgatc ctgaatactt ctttaaagaa 1200
   gaagatcgtc tgtttggtcg ttttacctta ctgcgtcgcg gtaaaaagaa ttactgtctg 1260
   atttgctgga aataa 1275
<210> 5
   <211> 1275
   <212> DNA
   <213> Artificial
<220>
   <223> artificial synthetase
<400> 5
   atggcaagca gtaacttgat taaacaattg caagagcggg ggctggtagc ccaggtgacg 60
   gacgaggaag cgttagcaga gcgactggcg caaggcccga tcgcactcgt gtgtggcttc 120
   gatcctaccg ctgacagctt gcatttgggg catcttgttc cattgttatg cctgaaacgc 180
   ttccagcagg cgggccacaa gccggttgcg ctggtaggcg gcgcgacggg tctgattggc 240
   gacccgagct tcaaagctgc cgagcgtaag ctgaacaccg aagaaactgt tcaggagtgg 300
   gtggacaaaa tccgtaagca ggttgccccg ttcctcgatt tcgactgtgg agaaaactct 360
   gctatcgcgg ccaataatta tgactggttc ggcaatatga atgtgctgac cttcctgcgc 420
   gatattggca aacacttctc cgttaaccag atgatcaaca aagaagcggt taagcagcgt 480
   ctcaaccgtg aagatcaggg gatttcgttc actgagtttt cctacaacct gctgcagggt 540
   tatagtatgg cctgtttgaa caaacagtac ggtgtggtgc tgcaaattgg tggttctgac 600
   cagtggggta acatcacttc tggtatcgac ctgacccgtc gtctgcatca gaatcaggtg 660
   tttggcctga ccgttccgct gatcactaaa gcagatggca ccaaatttgg taaaactgaa 720
   ggcggcgcag tctggttgga tccgaagaaa accagcccgt acaaattcta ccagttctgg 780
   atcaacactg cggatgccga cgtttaccgc ttcctgaagt tcttcacctt tatgagcatt 840
   gaagagatca acgccctgga agaagaagat aaaaacagcg gtaaagcacc gcgcgcccag 900
   tatgtactgg cggagcaggt gactcgtctg gttcacggtg aagaaggttt acaggcggca 960
   aaacgtatta ccgaatgcct gttcagcggt tctttgagtg cgctgagtga agcggacttc 1020
   gaacagctgg cgcaggacgg cgtaccgatg gttgagatgg aaaagggcgc agacctgatg 1080
   caggcactgg tcgattctga actgcaacct tcccgtggtc aggcacgtaa aactatcgcc 1140
   tccaatgcca tcaccattaa cggtgaaaaa cagtccgatc ctgaatactt ctttaaagaa 1200
   gaagatcgtc tgtttggtcg ttttacctta ctgcgtcgcg gtaaaaagaa ttactgtctg 1260
   atttgctgga aataa 1275
<210> 6
   <211> 1275
   <212> DNA
   <213> Artificial
<220>
   <223> artificial synthetase
<400> 6
   atggcaagca gtaacttgat taaacaattg caagagcggg ggctggtagc ccaggtgacg 60
   gacgaggaag cgttagcaga gcgactggcg caaggcccga tcgcactcgt gtgtggcttc 120
   gatcctaccg ctgacagctt gcatttgggg catcttgttc cattgttatg cctgaaacgc 180
   ttccagcagg cgggccacaa gccggttgcg ctggtaggcg gcgcgacggg tctgattggc 240
   gacccgagct tcaaagctgc cgagcgtaag ctgaacaccg aagaaactgt tcaggagtgg 300
   gtggacaaaa tccgtaagca ggttgccccg ttcctcgatt tcgactgtgg agaaaactct 360
   gctatcgcgg ccaataatta tgactggttc ggcaatatga atgtgctgac cttcctgcgc 420
   gatattggca aacacttctc cgttaaccag atgatcaaca aagaagcggt taagcagcgt 480
   ctcaaccgtg aagatcaggg gatttcgttc actgagtttt cctacaacct gctgcagggt 540
   tatagtatgg cctgtttgaa caaacagtac ggtgtggtgc tgcaaattgg tggttctgac 600
   cagtggggta acatcacttc tggtatcgac ctgacccgtc gtctgcatca gaatcaggtg 660
   tttggcctga ccgttccgct gatcactaaa gcagatggca ccaaatttgg taaaactgaa 720
   ggcggcgcag tctggttgga tccgaagaaa accagcccgt acaaattcta ccagttctgg 780
   atcaacactg cggatgccga cgtttaccgc ttcctgaagt tcttcacctt tatgagcatt 840
   gaagagatca acgccctgga agaagaagat aaaaacagcg gtaaagcacc gcgcgcccag 900
   tatgtactgg cggagcaggt gactcgtctg gttcacggtg aagaaggttt acaggcggca 960
   aaacgtatta ccgaatgcct gttcagcggt tctttgagtg cgctgagtga agcggacttc 1020
   gaacagctgg cgcaggacgg cgtaccgatg gttgagatgg aaaagggcgc agacctgatg 1080
   caggcactgg tcgattctga actgcaacct tcccgtggtc aggcacgtaa aactatcgcc 1140
   tccaatgcca tcaccattaa cggtgaaaaa cagtccgatc ctgaatactt ctttaaagaa 1200
   gaagatcgtc tgtttggtcg ttttacctta ctgcgtcgcg gtaaaaagaa ttactgtctg 1260
   atttgctgga aataa 1275
<210> 7
   <211> 1275
   <212> DNA
   <213> Artificial
<220>
   <223> artificial synthetase
<400> 7
   atggcaagca gtaacttgat taaacaattg caagagcggg ggctggtagc ccaggtgacg      60
   gacgaggaag cgttagcaga gcgactggcg caaggcccga tcgcactcac gtgtggcttc     120
   gatcctaccg ctgacagctt gcatttgggg catcttgttc cattgttatg cctgaaacgc     180
   ttccagcagg cgggccacaa gccggttgcg ctggtaggcg gcgcgacggg tctgattggc     240
   gacccgagct tcaaagctgc cgagcgtaag ctgaacaccg aagaaactgt tcaggagtgg     300
   gtggacaaaa tccgtaagca ggttgccccg ttcctcgatt tcgactgtgg agaaaactct     360
   gctatcgcgg ccaataatta tgactggttc ggcaatatga atgtgctgac cttcctgcgc     420
   gatattggca aacacttctc cgttaaccag atgatcaaca aagaagcggt taagcagcgt     480
   ctcaaccgtg aagatcaggg gatttcgttc actgagtttt cctacagcct gctgcagggt     540
   tatacgatgg cctgtctgaa caaacagtac ggtgtggtgc tgcaaattgg tggttctgac     600
   cagtggggta acatcacttc tggtatcgac ctgacccgtc gtctgcatca gaatcaggtg     660
   tttggcctga ccgttccgct gatcactaaa gcagatggca ccaaatttgg taaaactgaa     720
   ggcggcgcag tctggttgga tccgaagaaa accagcccgt acaaattcta ccagttctgg     780
   atcaacactg cggatgccga cgtttaccgc ttcctgaagt tcttcacctt tatgagcatt     840
   gaagagatca acgccctgga agaagaagat aaaaacagcg gtaaagcacc gcgcgcccag     900
   tatgtactgg cggagcaggt gactcgtctg gttcacggtg aagaaggttt acaggcggca     960
   aaacgtatta ccgaatgcct gttcagcggt tctttgagtg cgctgagtga agcggacttc    1020
   gaacagctgg cgcaggacgg cgtaccgatg gttgagatgg aaaagggcgc agacctgatg    1080
   caggcactgg tcgattctga actgcaacct tcccgtggtc aggcacgtaa aactatcgcc    1140
   tccaatgcca tcaccattaa cggtgaaaaa cagtccgatc ctgaatactt ctttaaagaa    1200
   gaagatcgtc tgtttggtcg ttttacctta ctgcgtcgcg gtaaaaagaa ttactgtctg    1260
   atttgctgga aataa                                                     1275
<210> 8
   <211> 540
   <212> DNA
   <213> Artificial
<220>
   <223> artificial synthetase
<400> 8
   cgggggctgg tagcccaggt gacggacgag gaagcgttag cagagcgact ggcgcaaggc      60
   ccgatcgcac tcacttgtgg cttcgatcct accgctgaca gcttgcattt ggggcatctt     120
   gttccattgt tatgcctgaa acgcttccag caggcgggcc acaagccggt tgcgctggta     180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcagcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttatacg tatgcctgtc tgaacaaaca gtacggtgtg 540
<210> 9
   <211> 540
   <212> DNA
   <213> Artificial
<220>
   <223> artificial synthetase
<400> 9
   cgggggctgg taccccaggt gacggacgag gaagcgttag cagagcgact ggcgcaaggc 60
   ccgatcgcac tcacttgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcagcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttatacg tatgcctgtc tgaacaaaca gtacggtgtg 540
<210> 10
   <211> 540
   <212> DNA
   <213> Artificial
<220>
   <223> artificial synthetase
<400> 10
   cgggggctgg tagcccaggt gacggacgag gaagcgttag cagagcgact ggcgcaaggc 60
   ccgatcgcac tcacttgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcagcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttatacg tatgcctgtc tgaacaaaca gtacggtgtg 540
<210> 11
   <211> 540
   <212> DNA
   <213> Artificial
<220>
   <223> artificial synthetase
<400> 11
   cgggggctgg tagcccaggt gacggacgag gaagcgttag cagagcgact ggcgcaaggc 60
   ccgatcgcac tcacttgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcggcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttattcg tatgcctgtg cgaacaaaca gtacggtgtg 540
<210> 12
   <211> 540
   <212> DNA
   <213> Artificial
<220>
   <223> artificial synthetase
<400> 12
   cgggggctgg tagcccaggt gacggacgag gaagcgttag cagagcgact ggcgcaaggc 60
   ccgatcgcac tcacttgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcagcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttatacg tatgcctgtc tgaacaaaca gtacggtgtg 540
<210> 13
   <211> 540
   <212> DNA
   <213> Artificial
<220>
   <223> artificial synthetase
<400> 13
   cgggggctgg taccccaggt gacggacgag gaagcgttag cagagcgact ggcgcaaggc 60
   ccgatcgcac tcctttgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcggcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttattct attgcctgtt cgaacaaaca gtacggtgtg 540
<210> 14
   <211> 540
   <212> DNA
   <213> Artificial
<220>
   <223> artificial synthetase
<400> 14
   cgggggctgg tagcccaggt gacggacgag gaagcgttag cagagcgact ggcgcaaggc 60
   ccgatcgcac tcgtgtgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcggcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttatagt attgcctgtt tgaacaaaca gtacggtgtg 540
<210> 15
   <211> 540
   <212> DNA
   <213> Artificial
<220>
   <223> artificial synthetase
<400> 15
   cgggggctgg taccccaggt gacggacgag gaagcgttag cagagcgact ggcgcaaggc 60
   ccgatcgcac tcgtgtgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcggcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttatagt attgcctgtt tgaacaaaca gtacggtgtg 540
<210> 16
   <211> 540
   <212> DNA
   <213> Artificial
<220>
   <223> artificial synthetase
<400> 16
   cgggggctgg tagcccaggt gacggacgag gaagcgttag cagagcgact ggcgcaaggc      60
   ccgatcgcac tctggtgtgg cttcgatcct accgctgaca gcttgcattt ggggcatctt     120
   gttccattgt tatgcctgaa acgcttccag caggcgggcc acaagccggt tgcgctggta     180
   ggcggcgcga cgggtctgat tggcgacccg agcttcaagg ctgccgagcg taagctgaac     240
   accgaagaaa ctgttcagga gtgggtggac aaaatccgta agcaggttgc cccgttcctc     300
   gatttcgact gtggagaaaa ctctgctatc gcggccaatt gttatgactg gttcggcaat     360
   atgaatgtgc tgaccttcct gcgcgatatt ggcaaacact tctccgttaa ccagatgatc     420
   aacaaagaag cggttaagca gcgtctcaac cgtgaagatc aggggatttc gttcactgag     480
   ttttcctaca acctgctgca gggttatatg cgtgcctgtg agaacaaaca gtacggtgtg     540
<210> 17
   <211> 624
   <212> DNA
   <213> Artificial
<220>
   <223> artificial synthetase
<400> 17
   cgggggctgg tagcccaggt gacggacgag gaagcgttag cagagcgact ggcgcaaggc      60
   ccgatcgcac tcatttgtgg cttcgatcct accgctgaca gcttgcattt ggggcatctt     120
   gttccattgt tatgcctgaa acgcttccag caggcgggcc acaagccggt tgcgctggta     180
   ggcggcgcga cgggtctgat tggcgacccg agcttcaaag ctgccgagcg taagctgaac     240
   accgaagaaa ctgttcagga gtgggtggac aaaatccgta agcaggttgc cccgttcctc     300
   gatttcgact gtggagaaaa ctctgctatc gcggccaata attatgactg gttcggcaat     360
   atgaatgtgc tgaccttcct gcgcgatatt ggcaaacact tctccgttaa ccagatgatc     420
   aacaaagaag cggttaagca gcgtctcaac cgtgaaggtc aggggatttc gttcactgag     480
   ttttcctaca acctgctgca gggttatggt atggcctgtg ctaacaaaca gtacggtgtg     540
   gtgctgcaaa ttggtggttc tgaccaatgg ggtaacatca cttctggtat cgacctgacc     600
   cgtcgtctgc atcagaatca ggtg                                            624
<210> 18
   <211> 609
   <212> DNA
   <213> Artificial
<220>
   <223> artificial synthetase
<400> 18
   caggtgacgg acgaggaagc gttagcagag cgactggcgc aaggcccgat cgcactcggt 60
   tgtggcttcg atcctaccgc tgacagcttg catttggggc atcttgttcc attgttatgc 120
   ctgaaacgct tccagcaggc gggccacaag ccggttgcgc tggtaggcgg cgcgacgggt 180
   ctgattggcg acccgagctt caaagctgcc gagcgtaagc tgaacaccga agaaactgtt 240
   caggagtggg tggacaaaat ccgtaagcag gttgccccgt tcctcgattt cgactgtgga 300
   gaaaactctg ctatcgcggc caataattat gactggttcg gcaatatgaa tgtgctgacc 360
   ttcctgcgcg atattggcaa acacttctcc gttaaccaga tgatcaacaa agaagcggtt 420
   aagcagcgtc tcaaccgtga agatcagggg atttcgttca ctgagttttc ctacaacctg 480
   ctgcagggtt atggttttgc ctgtttgaac aaacagtacg gtgtggtgct gcaaattggt 540
   ggttctgacc agtggggtaa catcacttct ggtatcgacc tgacccgtcg tctgcatcag 600
   aatcaggtg 609
<210> 19
   <211> 591
   <212> DNA
   <213> Artificial
<220>
   <223> artificial synthetase
<400> 19
   gcgttagcag agcgactggc gcaaggcccg atcgcactcg ggtgtggctt cgatcctacc 60
   gctgacagct tgcatttggg gcatcttgtt ccattgttat gcctgaaacg cttccagcag 120
   gcgggccaca agccggttgc gctggtaggc ggcgcgacgg gtctgattgg cgacccgagc 180
   ttcaaagctg ccgagcgtaa gctgaacacc gaagaaactg ttcaggagtg ggtggacaaa 240
   atccgtaagc aggttgcccc gttcctcgat ttcgactgtg gagaaaactc tgctatcgcg 300
   gccaataatt atgactggtt cggcaatatg aatgtgctga ccttcctgcg cgatattggc 360
   aaacacttct ccgttaacca gatgatcaac aaagaagcgg ttaagcagcg tctcaaccgt 420
   gaagatcagg ggatttcgtt cactgagttt tcctacaacc tgctgcaggg ttatggttat 480
   gcctgtatga acaaacagta cggtgtggtg ctgcaaattg gtggttctga ccagtggggt 540
   aacatcactt ctggtatcga cctgacccgt cgtctgcatc agaatcaggt g 591
<210> 20
   <211> 621
   <212> DNA
   <213> Artificial
<220>
   <223> artificial synthetase
<220>
   <221> misc_feature
   <222> (26)..(26)
   <223> n is a, c, g, or t
<220>
   <221> misc_feature
   <222> (612)..(612)
   <223> n is a, c, g, or t
<220>
   <221> misc_feature
   <222> (618)..(618)
   <223> n is a, c, g, or t
<400> 20
   gggctggtag cccaggtgac ggacgnagaa gcgttagcag agcgactggc gcaaggcccg      60
   atcgcactcc tttgtggctt cgatcctacc gctgacagct tgcatttggg gcatcttgtt     120
   ccattgttat gcctgaaacg cttccagcag gcgggccaca agccggttgc gctggtaggc     180
   ggcgcgacgg gtctgattgg cgacccgagc ttcaaagctg ccgagcgtaa gctgaacacc     240
   gaagaaactg ttcaggagtg ggtggacaaa atccgtaagc aggttgcccc gttcctcgat     300
   ttcgactgtg gagaaaactc tgctatcgcg gccaataatt atgactggtt cggcaatatg     360
   aatgtgctga ccttcctgcg cgatattggc aaacacttct ccgttaacca gatgatcaac     420
   aaagaagcgg ttaagcagcg tctcaaccgt gaagatcagg ggatttcgtt cactgagttt     480
   tcctacaacc tgctgcaggg ttattctatg gcctgtgcga acaaacagta cggtgtggtg     540
   ctgcaaattg gtggttctga ccagtggggt aacatcactt ctggtatcga cctgacccgt     600
   cgtctgcatc anaatcangt g                                               621
<210> 21
   <211> 588
   <212> DNA
   <213> Artificial
<220>
   <223> artificial synthetase
<400> 21
   ttagcagagc gactggcgca aggcccgatc gcactcgttt gtggcttcga tcctaccgct      60
   gacagcttgc atttggggca tcttgttcca ttgttatgcc tgaaacgctt ccagcaggcg     120
   ggccacaagc cggttgcgct ggtaggcggc gcgacgggtc tgattggcga cccgagcttc     180
   aaagctgccg agcgtaagct gaacaccgaa gaaactgttc aggagtgggt ggacaaaatc     240
   cgtaagcagg ttgccccgtt cctcgatttc gactgtggag aaaactctgc tatcgcggcc     300
   aataattatg actggttcgg caatatgaat gtgctgacct tcctgcgcga tattggcaaa     360
   cacttctccg ttaaccagat gatcaacaaa gaagcggtta agcagcgtct caaccgtgaa     420
   gatcagggga tttcgttcac tgagttttcc tacaacctgc tgcagggtta ttctgcggcc     480
   tgtgcgaaca aacagtacgg tgtggtgctg caaattggtg gttctgacca gtggggtaac     540
   atcacttctg gtatcgacct gacccgtcgt ctgcatcaga atcaggtg                 588
<210> 22
   <211> 600
   <212> DNA
   <213> Artificial
<220>
   <223> artificial synthetase
<220>
   <221> misc_feature
   <222> (403)..(403)
   <223> n is a, c, g, or t
<220>
   <221> misc_feature
   <222> (513)..(513)
   <223> n is a, c, g, or t
<220>
   <221> misc_feature
   <222> (515)..(515)
   <223> n is a, c, g, or t
<220>
   <221> misc_feature
   <222> (518)..(518)
   <223> n is a, c, g, or t
<220>
   <221> misc_feature
   <222> (531)..(531)
   <223> n is a, c, g, or t
<400> 22
   gacgaggaag cgttagcaga gcgactggcg caaggcccga tcgcactcct gtgtggcttc 60
   gatcctaccg ctgacagctt gcatttgggg catcttgttc cattgttatg cctgaaacgc 120
   ttccagcagg cgggccacaa gccggttgcg ctggtaggcg gcgcgacggg tctgattggc 180
   gacccgagct tcaaagctgc cgagcgtaag ctgaacaccg aagaaactgt tcaggagtgg 240
   gtggacaaaa tccgtaagca ggttgccccg ttcctcgatt tcgactgtgg agaaaactct 300
   gctatcgcgg ccaataatta tgactggttc ggcaatatga atgtgctgac cttcctgcgc 360
   gatattggca aacacttctc cgttaaccag atgatcaaca aanaagcggt taagcagcgt 420
   ctcaaccgtg aagatcaggg gatttcgttc actgagtttt cctacaacct gctgcagggt 480
   tattcggctg cctgtgcgaa caaacagtac ggngnggngc tgcaaattgg nggttctgac 540
   caggggggta acatcacttc tggtatcgac ctgacccgtc gtctgcatca aaatcaggtg 600
<210> 23
   <211> 591
   <212> DNA
   <213> Artificial
<220>
   <223> artificial synthetase
<220>
   <221> misc_feature
   <222> (588)..(588)
   <223> n is a, c, g, or t
<400> 23
   gcgttagcag agcgactggc gcaaggcccg atcgcactcg tttgtggctt cgatcctacc      60
   gctgacagct tgcatttggg gcatcttgtt ccattgttgt gcctgaaacg cttccagcag     120
   gcgggccaca agccggttgc gctggtaggc ggcgcgacgg gtctgattgg cgacccgagc     180
   ttcaaagctg ccgagcgtaa gctgaacacc gaagaaactg ttcaggagtg ggtggacaaa     240
   atccgtaagc aggttgcccc gttcctcgat ttcgactgtg gagaaaactc tgctatcgcg     300
   gccaataatt atgactggtt cggcaatatg aatgtgctga ccttcctgcg cgatattggc     360
   aaacacttct ccgttaacca gatgatcaac aaagaagcgg ttaagcagcg tctcaaccgt     420
   gaagatcagg ggatttcgtt cactgagttt tcctacaacc tgctgcaggg ttatagtgcg     480
   gcctgtgtta acaaacagta cggtgtggtg ctgcaaattg gtggttctga ccagtggggt     540
   aacatcactt ctggtatcga cctgacccgt cgtctgcatc agaatcangt g              591
<210> 24
   <211> 600
   <212> DNA
   <213> Artificial
<220>
   <223> artificial synthetase
<400> 24
   gacgaggaag cgttagcaga gcgactggcg caaggcccga tcgcactcat ttgtggcttc      60
   gatcctaccg ctgacagctt gcatttgggg catcttgttc cattgttatg cctgaaacgc     120
   ttccagcagg cgggccacaa gccggttgcg ctggtaggcg gcgcgacggg tctgattggc     180
   gacccgagct tcaaagctgc cgagcgtaag ctgaacaccg aagaaactgt tcaggagtgg     240
   gtggacaaaa tccgtaagca ggttgccccg ttcctcgatt tcgactgtgg agaaaactct     300
   gctatcgcgg ccaatgatta tgactggttc ggcaatatga atgtgctgac cttcctgcgc     360
   gatattggca aacacttctc cgttaaccag atgatcaaca aagaagcggt taagcagcgt     420
   ctcaaccgtg aagatcaggg gatttcgttc actgagtttt cctacaacct gctgcagggt     480
   tataattttg cctgtgtgaa caaacagtac ggtgtggtgc tgcaaattgg tggttctgac     540
   cagtggggta acatcacttc tggtatcgac ctgacccgtc gtctgcatca gaatcaggtg     600
<210> 25
   <211> 579
   <212> DNA
   <213> Artificial
<220>
   <223> artificial synthetase
<400> 25
   cgactggcgc aaggcccgat cgcactcacg tgtggcttcg atcctaccgc tgacagcttg      60
   catttggggc atcttgttcc attgttatgc ctgaaacgct tccagcaggc gggccacaag     120
   ccggttgcgc tggtaggcgg cgcgacgggt ctgattggcg acccgagctt caaagctgcc     180
   gagcgtaagc tgaacaccga agaaactgtt caggagtggg tggacaaaat ccgtaagcag     240
   gttgccccgt tcctcgattt cgactgtgga gaaaactctg ctatcgcggc caataattat     300
   gactggttcg gcaatatgaa tgtgctgacc ttcctgcgcg atattggcaa acacttctcc     360
   gttaaccaga tgatcaacaa agaagcggtt aagcagcgtc tcaaccgtga agatcagggg     420
   atttcgttca ctgagttttc ctacaatctg ctgcagggtt attcggctgc ctgtcttaac     480
   aaacagtacg gtgtggtgct gcaaattggt ggttctgacc agtggggtaa catcacttct     540
   ggtatcgacc tgacccgtcg tctgcatcag aatcaggtg                            579
<210> 26
   <211> 624
   <212> DNA
   <213> Artificial
<220>
   <223> artificial synthetase
<220>
   <221> misc_feature
   <222> (13)..(13)
   <223> n is a, c, g, or t
<220>
   <221> misc_feature
   <222> (599)..(599)
   <223> n is a, c, g, or t
<400> 26
   cgggggctgg tancccaggt gacggacgag gaagcgttag cagagcgact ggcgcaaggc 60
   ccgatcgcac tcgggtgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcggcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca acctgctgca gggttattct atggcctgtt tgaacaaaca gtacggtgtg 540
   gtgctgcaaa ttggtggttc tgaccagtgg ggtaacatca cttctggtat cgacctganc 600
   cgtcgtctgc atcagaatca ggtg 624
<210> 27
   <211> 625
   <212> DNA
   <213> Artificial
<220>
   <223> artificial synthetase
<220>
   <221> misc_feature
   <222> (600)..(600)
   <223> n is a, c, g, or t
<400> 27
   cgggggctgg tagcccaggt gacggacgag gaagcgttag cagagcgact ggcgcaaggc 60
   ccgatcgcac tcacgtgtgg cttcgatcct accgctgaca gcttgcattt ggggcatctt 120
   gttccattgt tatgcctgaa acgcttccag caggcgggcc acaagccggt tgcgctggta 180
   ggcggcgcga cgggtctgat tggcgacccg agcttcaaag ctgccgagcg taagctgaac 240
   accgaagaaa ctgttcagga gtgggtggac aaaatccgta agcaggttgc cccgttcctc 300
   gatttcgact gtggagaaaa ctctgctatc gcggccaata attatgactg gttcggcaat 360
   atgaatgtgc tgaccttcct gcgcgatatt ggcaaacact tctccgttaa ccagatgatc 420
   aacaaagaag cggttaagca gcgtctcaac cgtgaagatc aggggatttc gttcactgag 480
   ttttcctaca atctgctgca gggttattcg gctgcctgtc ttaacaaaca gtacggtgtg 540
   gtgctgcaaa ttggtggttc tgaccagtgg ggtaacatca cttctggtat cgaacctgan 600
   ccgtcgtctg catcaaaatc aagtg 625
<210> 28
   <211> 624
   <212> DNA
   <213> Artificial
<220>
   <223> artificial synthetase
<400> 28
   cgggggctgg taccccaagt gacggacgag gaaacgttag cagagcgact ggcgcaaggc      60
   ccgatcgcac tctcttgtgg cttcgatcct accgctgaca gcttgcattt ggggcatctt     120
   gttccattgt tatgcctgaa acgcttccag caggcaggcc acaagccggt tgcgctggta     180
   ggcggcgcga cgggtctgat tggcgacccg agcttcaaag ctgccgagcg taagctgaac     240
   accgaagaaa ctgttcagga gtgggtggac aaaatccgta agcaggttgc cccgttcctc     300
   gatttcgact gtggagaaaa ctctgctatc gcggccaata attatgactg gttcggcaat     360
   atgaatgtgc tgaccttcct gcgcgatatt ggcaaacact tctccgttaa ccagatgatc     420
   aacaaagaag cggttaagca gcgtctcaac cgtgaagatc aggggatttc gttcactgag     480
   ttttcctaca acctgctgca gggttatacg atggcctgtg tgaacaaaca gtacggtgtg     540
   gtgctgcaaa ttggtggttc tgaccagtgg ggtaacatca cttctggtat cgacctgacc     600
   cgtcgtctgc atcagaatca ggtg                                            624
<210> 29
   <211> 624
   <212> DNA
   <213> Artificial
<220>
   <223> artificial synthetase
<400> 29
   cgggggctgg tagcccaggt gacggacgag gaagcgttag cagagcgact ggcgcaaggc      60
   ccgatcgcac tcgcgtgcgg cttcgatcct accgctgaca gcttgcattt ggggcatctt     120
   gttccattgt tatgcctgaa acgcttccag caggcgggcc acaagccggt tgcgctggta     180
   ggcggcgcga cgggtctgat tggcgacccg agcttcaagg ctgccgagcg taagctgaac     240
   accgaagaaa ctgttcagga gtgggtggac aaaatccgta agcaggttgc cccgttcctc     300
   gatttcgact gtggagaaaa ctctgctatc gcggccaata attatgactg gttcggcaat     360
   atgaatgtgc tgaccttcct gcgcgatatt ggcaaacact tctccgttaa ccagatgatc     420
   aacaaagaag cggttaagca gcgtctcaac cgtgaagatc aggggatttc gttcactgag     480
   ttttcctaca acctgctgca gggttattct tatgcctgtc ttaacaaaca gtacggtgtg     540
   gtgctgcaaa ttggtggttc tgaccagtgg ggtaacatca cttctggtat cgacctgacc     600
   cgtcgtctgc atcagaatca ggtg                                            624
<210> 30
   <211> 624
   <212> DNA
   <213> Artificial
<220>
   <223> artificial synthetase
<400> 30
   cgggggctgg tagcccaggt gacggacgag gaagcgttag cagagcgact ggcgcaaggc      60
   ccgatcgcac tcgcgtgtgg cttcgatcct accgctgaca gcttgcattt ggggcatctt     120
   gttccattgt tatgcctgaa acgcttccag caggcgggcc acaagccggt tgcgctggta     180
   ggcggcgcga cgggtctgat tggcgacccg agcttcaaag ctgccgagcg taagctgaac     240
   accgaagaaa ctgttcagga gtgggtggac aaaatccgta agcaggttgc cccgttcctc     300
   gatttcgact gtggagaaaa ctctgctatc gcggccaata attatgactg gttcggcaat     360
   atgaatgtgc tgaccttcct gcgcgatatt ggcaaacact tctccgttaa ccagatgatc     420
   aacaaagaag cggttaagca gcgtctcaac cgtgaagatc aggggatttc gttcactgag     480
   ttttcctaca acctgctgca gggttatacg atggcctgtt gtaacaaaca gtacggtgtg     540
   gtgctgcaaa ttggtggttc tgaccagtgg ggtaacatca cttctggtat cgacctgacc     600
   cgtcgtctgc atcagaatca ggtg                                            624
<210> 31
   <211> 624
   <212> DNA
   <213> Artificial
<220>
   <223> artificial synthetase
<400> 31
   cgggggctgg taccccaagt gacggacgag gaagcgttag cagagcgact ggcgcaaggc      60
   ccgatcgcac tcacgtgtgg cttcgatcct accgctgaca gcttgcattt ggggcatctt     120
   gttccattgt tatgcctgaa acgcttccag caggcgggcc acaagccggt tgcgctggta     180
   ggcggcgcga cgggtctgat tggcgacccg agcttcaaag ctgccgagcg taagctgaac     240
   accgaagaaa ctgttcagga gtgggtggac aaaatccgta agcaggttgc cccgttcctc     300
   gatttcgact gtggagaaaa ctctgctatc gcggccaata attatgactg gttcggcaat     360
   atgaatgtgc tgaccttcct gcgcgatatt ggcaaacact tctccgttaa ccagatgatc     420
   aacaaagaag cggttaagca gcgtctcaac cgtgaagatc aggggatttc gttcgctgag     480
   ttttcctaca acctgctgca gggttatacg tttgcctgta tgaacaaaca gtacggtgtg     540
   gtgctgcaaa ttggtggttc tgaccagtgg ggtaacatca cttctggtat cgacctgacc     600
   cgtcgtctgc atcagaatca ggtg                                            624
<210> 32
   <211> 606
   <212> DNA
   <213> Artificial
<220>
   <223> artificial synthetase
<400> 32
   gtgacggacg aggaagcgtt agcagagcga ctggcgcaag gcccgatcgc actcacgtgt     60
   ggcttcgatc ctaccgctga cagcttgcat ttggggcatc ttgttccatt gttatgcctg    120
   aaacgcttcc agcaggcggg ccacaagccg gttgcgctgg taggcggcgc gacgggtctg    180
   attggcgacc cgagcttcaa agctgccgag cgtaagctga acaccgaaga aactgttcag    240
   gagtgggtgg acaaaatccg taagcaggtt gccccgttcc tcgatttcga ctgtggagaa    300
   aactctgcta tcgcggccaa taattatgac tggttcggca atatgaatgt gctgaccttc    360
   ctgcgcgata ttggcaaaca cttctccgtt aaccagatga tcaacaaaga agcggttaag    420
   cagcgtctca accgtgaaga tcaggggatt tcgttcactg agttttccta caatctgctg    480
   cagggttatt cggctgcctg tcttaacaaa cagtacggtg tggtgctgca aattggtggt    540
   tctgaccagt ggggtaacat cacttctggt atcgacctga cccgtcgtct gcatcagaat    600
   caggtg                                                               606
<210> 33
   <211> 624
   <212> DNA
   <213> Artificial
<220>
   <223> artificial synthetase
<400> 33
   cgggggctgg tagcccaggt gacggacgag gaagcgttag cagagcgact ggcgcaaggc     60
   ccgatcgcac tcgtttgtgg cttcgatcct accgctgaca gcttgcattt ggggcatctt    120
   gttccattgt tatgcctgaa acgcttccag caggcgggcc acaagccggt tgcgctggta    180
   ggcggcgcga cgggtctgat tggcgacccg agcttcaaag ctgccgagcg taagctgaac    240
   accgaagaaa ctgttcagga gtgggtggac aaaatccgta agcaggttgc cccgttcctc    300
   gatttcgact gtggagaaaa ctctgctatc gcggccaata attatgactg gttcggcaat    360
   atgaatgtgc tgaccttcct gcgcgatatt ggcaaacact tctccgttaa ccagatgatc    420
   aacaaagaag cggttaagca gcgtctcaac cgtgaagatc aggggatttc gttcactgag    480
   ttttcctaca acctgctgca gggttattcg atggcctgta cgaacaaaca gtacggtgtg    540
   gtgctgcaaa ttggtggttc tgaccagtgg ggtaacatca cttctggtat cgacctgacc    600
   cgtcgtctgc atcagaatca ggtg                                           624
<210> 34
   <211> 624
   <212> DNA
   <213> Artificial
<220>
   <223> artificial synthetase
<220>
   <221> misc_feature
   <222> (13)..(13)
   <223> n is a, c, g, or t
<400> 34
   cgggggctgg tancccaagt gacggacggg gaagcgttag cagagcgact ggcgcaaggc      60
   ccgatcgcac tcagttgtgg cttcgatcct accgctgaca gcttgcattt ggggcatctt     120
   gttccattgt tatgcctgaa acgcttccag caggcgggcc acaagccggt tgcgctggta     180
   ggcggcgcga cgggtctgat tggcgacccg agcttcaaag ctgccgagcg taagctgaac     240
   accgaagaaa ctgttcagga gtgggtggac aaaatccgta agcaggttgc cccgttcctc     300
   gatctcgact gtggagaaaa ctctgctatc gcggccaata attatgactg gttcggcaat     360
   atgaatgtgc tgaccttcct gcgcgatatt ggcaaacact tctccgttaa ccagatgatc     420
   aacaaagaag cggttaagca gcgtctcaac cgtgaagatc aggggatttc gttcactgag     480
   ttttcctaca acctgctgca gggttatagt tttgcctgtc tgaacaaaca gtacggtgtg     540
   gtgctgcaaa ttggtggttc tgaccagtgg ggtaacatca cttctggtat cgacctgacc     600
   cgtcgtctgc atcagaatca ggtg                                            624
<210> 35
   <211> 624
   <212> DNA
   <213> Artificial
<220>
   <223> artificial synthetase
<400> 35
   cgggggctgg tagcccaggt gacggacgag gaagcgttag cagagcgact ggcgcaaggc     60
   ccgatcgcac tcacgtgtgg cttcgatcct accgctgaca gcttgcattt ggggcatctt    120
   gttccattgt tatgcctgaa acgcttccag caggcgggcc acaagccggt tgcgctggta    180
   ggcggcgcga cgggtctgat tggcgacccg agcttcaaag ctgccgagcg taagctgaac    240
   accgaagaaa ctgttcagga gtgggtggac aaaatccgta agcaggttgc cccgttcctc    300
   gatttcgact gtggagaaaa ctctgctatc gcggccaata attatgactg gttcggcaat    360
   atgaatgtgc tgaccttcct gcgcgatatt ggcaaacact tctccgttaa ccagatgatc    420
   aacaaagaag cggttaagca gcgtctcaac cgtgaagatc aggggatttc gttcactgag    480
   ttttcctaca acctgctgca gggttatacg tttgcctgta ctaacaaaca gtacggtgtg    540
   gtgctgcaaa ttggtggttc tgaccagtgg ggtaacatca cttctggtat cgacctgacc    600
   cgtcgtctgc atcagaatca ggtg                                           624
<210> 36
   <211> 424
   <212> PRT
   <213> Artificial
<220>
   <223> artificial synthetase
<400> 36
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
               20                  25                  30
   Pro Ile Ala Leu Val Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Ser Tyr Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                 320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
           355                 360                 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 37
   <211> 424
   <212> PRT
   <213> Artificial
<220>
   <223> artificial synthetase
<400> 37
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Ile Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Met Ala Cys Leu Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 38
   <211> 424
   <212> PRT
   <213> Artificial
<220>
   <223> artificial synthetase
<400> 38
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
               20                  25                  30
   Pro Ile Ala Leu Val Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Ser Met Ala Cys Ala Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 39
   <211> 424
   <212> PRT
   <213> Artificial
<220>
   <223> artificial synthetase
<400> 39
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
                   20                  25                  30
   Pro Ile Ala Leu Val Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Ser Met Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 40
   <211> 424
   <212> PRT
   <213> Artificial
<220>
   <223> artificial synthetase
<400> 40
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Thr Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Thr Met Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                 320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 41
   <211> 424
   <212> PRT
   <213> Artificial
<220>
   <223> artificial synthetase
<400> 41
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Thr Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Thr Tyr Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                 320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
           355                 360                 365
   Gln Pro Ser Arg Gly Gln Ala Arg Lys Thr Ile Ala Ser Asn Ala Ile
       370                 375                 380
   Thr Ile Asn Gly Glu Lys Gln Ser Asp Pro Glu Tyr Phe Phe Lys Glu
   385                 390                 395                 400
   Glu Asp Arg Leu Phe Gly Arg Phe Thr Leu Leu Arg Arg Gly Lys Lys
                   405                 410                 415
   Asn Tyr Cys Leu Ile Cys Trp Lys
   420
<210> 42
   <211> 424
   <212> PRT
   <213> Artificial
<220>
   <223> artificial synthetase
<400> 42
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Leu Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Met Ala Cys Ser Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 43
   <211> 424
   <212> PRT
   <213> Artificial
<220>
   <223> artificial synthetase
<400> 43
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Leu Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Met Ala Cys Ala Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 44
   <211> 424
   <212> PRT
   <213> Artificial
<220>
   <223> artificial synthetase
<400> 44
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Thr Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Arg Met Ala Cys Leu Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 45
   <211> 424
   <212> PRT
   <213> Artificial
<220>
   <223> artificial synthetase
<400> 45
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Ile Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Gly Met Ala Cys Ala Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                 320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
           355                 360                 365
   Gln Pro Ser Arg Gly Gln Ala Arg Lys Thr Ile Ala Ser Asn Ala Ile
       370                 375                 380
   Thr Ile Asn Gly Glu Lys Gln Ser Asp Pro Glu Tyr Phe Phe Lys Glu
   385                 390                 395                 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 46
   <211> 424
   <212> PRT
   <213> Artificial
<220>
   <223> artificial synthetase
<400> 46
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Gly Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Gly Phe Ala Cys Ala Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 47
   <211> 424
   <212> PRT
   <213> Artificial
<220>
   <223> artificial synthetase
<400> 47
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Gly Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Gly Tyr Ala Cys Met Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 48
   <211> 424
   <212> PRT
   <213> Artificial
<220>
   <223> artificial synthetase
<400> 48
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Leu Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Met Ala Cys Ala Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 49
   <211> 424
   <212> PRT
   <213> Artificial
<220>
   <223> artificial synthetase
<400> 49
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Val Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Ala Ala Cys Ala Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 50
   <211> 424
   <212> PRT
   <213> Artificial
<220>
   <223> artificial synthetase
<400> 50
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Leu Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Ala Ala Cys Ala Asn Lys Gln Tyr Gly Val
   180 185 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                 320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
           355                 360                 365
   Gln Pro Ser Arg Gly Gln Ala Arg Lys Thr Ile Ala Ser Asn Ala Ile
       370                 375                 380
   Thr Ile Asn Gly Glu Lys Gln Ser Asp Pro Glu Tyr Phe Phe Lys Glu
   385                 390                 395                 400
   Glu Asp Arg Leu Phe Gly Arg Phe Thr Leu Leu Arg Arg Gly Lys Lys
                   405                 410                 415
   Asn Tyr Cys Leu Ile Cys Trp Lys
               420
<210> 51
   <211> 424
   <212> PRT
   <213> Artificial
<220>
   <223> artificial synthetase
<400> 51
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
               20                  25                  30
   Pro Ile Ala Leu Val Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Ser Ala Ala Cys Val Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 52
   <211> 424
   <212> PRT
   <213> Artificial
<220>
   <223> artificial synthetase
<400> 52
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Ile Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asp Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Asn Phe Ala Cys Val Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 53
   <211> 424
   <212> PRT
   <213> Artificial
<220>
   <223> artificial synthetase
<400> 53
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Thr Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Ser Ala Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                 320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
           355                 360                 365
   Gln Pro Ser Arg Gly Gln Ala Arg Lys Thr Ile Ala Ser Asn Ala Ile
       370                 375                 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 54
   <211> 424
   <212> PRT
   <213> Artificial
<220>
   <223> artificial synthetase
<400> 54
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Gly Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Met Ala Cys Leu Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 55
   <211> 424
   <212> PRT
   <213> Artificial
<220>
   <223> artificial synthetase
<400> 55
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
               20                  25                  30
   Pro Ile Ala Leu Thr Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Ser Ala Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 56
   <211> 424
   <212> PRT
   <213> Artificial
<220>
   <223> artificial synthetase
<400> 56
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Ser Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Thr Met Ala Cys Val Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 57
   <211> 424
   <212> PRT
   <213> Artificial
<220>
   <223> artificial synthetase
<400> 57
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Ala Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Tyr Ala Cys Leu Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 58
   <211> 424
   <212> PRT
   <213> Artificial
<220>
   <223> artificial synthetase
<400> 58
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Ala Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
                   165                 170                 175
   Leu Leu Gln Gly Tyr Thr Met Ala Cys Cys Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                     240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
       290                 295                 300
   Glu Gln Val Thr Arg Leu Val His Gly Glu Glu Gly Leu Gln Ala Ala
   305                 310                 315                     320
   Lys Arg Ile Thr Glu Cys Leu Phe Ser Gly Ser Leu Ser Ala Leu Ser
                   325                 330                 335
   Glu Ala Asp Phe Glu Gln Leu Ala Gln Asp Gly Val Pro Met Val Glu
               340                 345                 350
   Met Glu Lys Gly Ala Asp Leu Met Gln Ala Leu Val Asp Ser Glu Leu
           355                 360                 365
   Gln Pro Ser Arg Gly Gln Ala Arg Lys Thr Ile Ala Ser Asn Ala Ile
       370                 375                 380
   Thr Ile Asn Gly Glu Lys Gln Ser Asp Pro Glu Tyr Phe Phe Lys Glu
   385                 390                 395                     400
   Glu Asp Arg Leu Phe Gly Arg Phe Thr Leu Leu Arg Arg Gly Lys Lys
                   405                 410                 415
   Asn Tyr Cys Leu Ile Cys Trp Lys
               420
<210> 59
   <211> 424
   <212> PRT
   <213> Artificial
<220>
   <223> artificial synthetase
<400> 59
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
               20                  25                  30
   Pro Ile Ala Leu Thr Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Thr Phe Ala Cys Met Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 60
   <211> 424
   <212> PRT
   <213> Artificial
<220>
   <223> artificial synthetase
<400> 60
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Thr Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Ser Val Ala Cys Leu Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225                 230                 235                 240
   Gly Gly Ala Val Trp Leu Asp Pro Lys Lys Thr Ser Pro Tyr Lys Phe
                   245                 250                 255
   Tyr Gln Phe Trp Ile Asn Thr Ala Asp Ala Asp Val Tyr Arg Phe Leu
               260                 265                 270
   Lys Phe Phe Thr Phe Met Ser Ile Glu Glu Ile Asn Ala Leu Glu Glu
           275                 280                 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 61
   <211> 424
   <212> PRT
   <213> Artificial
<220>
   <223> artificial synthetase
<400> 61
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Val Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Met Ala Cys Thr Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 62
   <211> 424
   <212> PRT
   <213> Artificial
<220>
   <223> artificial synthetase
<400> 62
   Met Ala Ser Ser Asn Leu Ile Lys Gln Leu Gln Glu Arg Gly Leu Val
   1 5 10 15
   Ala Gln Val Thr Asp Glu Glu Ala Leu Ala Glu Arg Leu Ala Gln Gly
   20 25 30
   Pro Ile Ala Leu Ser Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Asp Gln Gly Ile Ser Phe Thr Glu Phe Ser Tyr Asn
   165 170 175
   Leu Leu Gln Gly Tyr Ser Phe Ala Cys Leu Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 63
   <211> 424
   <212> PRT
   <213> Artificial
<220>
   <223> artificial synthetase
<400> 63
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
               20                  25                  30
   Pro Ile Ala Leu Thr Cys Gly Phe Asp Pro Thr Ala Asp Ser Leu His
           35                  40                  45
   Leu Gly His Leu Val Pro Leu Leu Cys Leu Lys Arg Phe Gln Gln Ala
       50                  55                  60
   Gly His Lys Pro Val Ala Leu Val Gly Gly Ala Thr Gly Leu Ile Gly
   65                  70                  75                  80
   Asp Pro Ser Phe Lys Ala Ala Glu Arg Lys Leu Asn Thr Glu Glu Thr
                   85                  90                  95
   Val Gln Glu Trp Val Asp Lys Ile Arg Lys Gln Val Ala Pro Phe Leu
               100                 105                 110
   Asp Phe Asp Cys Gly Glu Asn Ser Ala Ile Ala Ala Asn Asn Tyr Asp
           115                 120                 125
   Trp Phe Gly Asn Met Asn Val Leu Thr Phe Leu Arg Asp Ile Gly Lys
       130                 135                 140
   His Phe Ser Val Asn Gln Met Ile Asn Lys Glu Ala Val Lys Gln Arg
   145                 150                 155                 160
   Leu Asn Arg Glu Asp Gln Gly Ile Ser Phe Thr Glu Phe Ser Tyr Asn
                   165                 170                 175
   Leu Leu Gln Gly Tyr Thr Phe Ala Cys Thr Asn Lys Gln Tyr Gly Val
               180                 185                 190
   Val Leu Gln Ile Gly Gly Ser Asp Gln Trp Gly Asn Ile Thr Ser Gly
           195                 200                 205
   Ile Asp Leu Thr Arg Arg Leu His Gln Asn Gln Val Phe Gly Leu Thr
       210                 215                 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 64
   <211> 129
   <212> DNA
   <213> Escherichia coli
<400> 64
   agcttcccga taagggagca ggccagtaaa aagcattacc ccgtggtggg gttcccgagc 60
   ggccaaaggg agcagactct aaatctgccg tcatcgacct cgaaggttcg aatccttccc 120
   ccaccacca 129
<210> 65
   <211> 129
   <212> RNA
   <213> Escherichia coli
<400> 65
   agcuucccga uaagggagca ggccaguaaa aagcauuacc ccgugguggg guucccgagc 60
   ggccaaaggg agcagacucu aaaucugccg ucaucgaccu cgaagguucg aauccuuccc 120
   ccaccacca 129
<210> 66
   <211> 34
   <212> DNA
   <213> Artificial
<220>
   <223> oligonucleotide primer
<400> 66
   atgaagtagc tgtcttctat cgaacaagca tgcg 34
<210> 67
   <211> 34
   <212> DNA
   <213> Artificial
<220>
   <223> oligonucleotide primer
<400> 67
   cgaacaagca tgcgattagt gccgacttaa aaag 34
<210> 68
   <211> 33
   <212> DNA
   <213> Artificial
<220>
   <223> oligonucleotide primer
<400> 68
   cgctactctc ccaaatagaa aaggtctccg ctg 33
<210> 69
   <211> 32
   <212> DNA
   <213> Artificial
<220>
   <223> oligonucleotide primer
<400> 69
   ctggaacagc tatagctact gatttttcct cg 32
<210> 70
   <211> 34
   <212> DNA
   <213> Artificial
<220>
   <223> oligonucleotide primer
<400> 70
   gccgtcacag attagttggc ttcagtggag actg 34
<210> 71
   <211> 33
   <212> DNA
   <213> Artificial
<220>
   <223> oligonucleotide primer
<400> 71
   gattggcttc ataggagact gatatgctct aac 33
<210> 72
   <211> 33
   <212> DNA
   <213> Artificial
<220>
   <223> oligonucleotide primer
<400> 72
   gcctctatag ttgagacagc atagaataat gcg 33
<210> 73
   <211> 35
   <212> DNA
   <213> Artificial
<220>
   <223> oligonucleotide primer
<400> 73
   gagacagcat agatagagtg cgacatcatc atcgg 35
<210> 74
   <211> 37
   <212> DNA
   <213> Artificial
<220>
   <223> oligonucleotide primer
<400> 74
   gaataagtgc gacatagtca tcggaagaga gtagtag 37
<210> 75
   <211> 35
   <212> DNA
   <213> Artificial
<220>
   <223> oligonucleotide primer
<400> 75
   ggtcaaagac agttgtaggt atcgattgac tcggc 35
<210> 76
<211> 34
   <212> DNA
   <213> Artificial
<220>
   <223> oligonucleotide primer
<400> 76
   cgctactctc cccaaattta aaaggtctcc gctg 34
<210> 77
   <211> 34
   <212> DNA
   <213> Artificial
<220>
   <223> oligonucleotide primer
<400> 77
   cgctactctc cccaaatata aaaggtctcc gctg 34
<210> 78
   <211> 34
   <212> DNA
   <213> Artificial
<220>
   <223> oligonucleotide primer
<400> 78
   cgctactctc cccaaatgga aaaggtctcc gctg 34
<210> 79
   <211> 34
   <212> DNA
   <213> Artificial
<220>
   <223> oligonucleotide primer
<400> 79
   cgctactctc cccaaagata aaaggtctcc gctg 34
<210> 80
   <211> 34
   <212> DNA
   <213> Artificial
<220>
   <223> oligonucleotide primer
<400> 80
   cgctactctc cccaaaaaaa aaaggtctcc gctg 34
<210> 81
   <211> 34
   <212> DNA
   <213> Artificial
<220>
   <223> oligonucleotide primer
<400> 81
   gccgtcacag attttttggc ttcagtggag actg 34
<210> 82
   <211> 34
   <212> DNA
   <213> Artificial
<220>
   <223> oligonucleotide primer
<400> 82
   gccgtcacag attatttggc ttcagtggag actg 34
<210> 83
   <211> 34
   <212> DNA
   <213> Artificial
<220>
   <223> oligonucleotide primer
<400> 83
   gccgtcacag attggttggc ttcagtggag actg 34
<210> 84
   <211> 34
   <212> DNA
   <213> Artificial
<220>
   <223> oligonucleotide primer
<400> 84
   gccgtcacag atgatttggc ttcagtggag actg 34
<210> 85
   <211> 34
   <212> DNA
   <213> Artificial
<220>
   <223> oligonucleotide primer
<400> 85
   gccgtcacag ataaattggc ttcagtggag actg 34
<210> 86
   <211> 424
   <212> PRT
   <213> Artificial
<220>
   <223> artificial synthetase
<400> 86
   Met Ala Ser Ser Asn Leu Ile Lys Gln Leu Gln Glu Arg Gly Leu Val
   1               5                   10                  15
   Ala Gln Val Thr Asp Glu Glu Ala Leu Ala Glu Arg Leu Ala Gln Gly
   20 25 30
   Pro Ile Ala Leu Ile Cys Gly Phe Asp Pro Thr Ala Asp Ser Leu His
   35 40 45
   Leu Gly His Leu Val Pro Leu Leu Cys Leu Lys Arg Phe Gln Gln Ala
   50 55 60
   Gly His Lys Pro Val Ala Leu Val Gly Gly Ala Thr Gly Leu Ile Gly
   65 70 75 80
   Asp Pro Ser Phe Lys Ala Ala Glu Arg Lys Leu Asn Thr Glu Glu Thr
   85 90 95
   Val Gln Glu Trp Val Asp Lys Ile Arg Lys Gln Val Ala Pro Phe Leu
   100 105 110
   Asp Phe Asp Cys Gly Glu Asn Ser Ala Ile Ala Ala Asn Asn Tyr Asp
   115 120 125
   Trp Phe Gly Asn Met Asn Val Leu Thr Phe Leu Arg Asp Ile Gly Lys
   130 135 140
   His Phe Ser Val Asn Gln Met Ile Asn Lys Glu Ala Val Lys Gln Arg
   145 150 155 160
   Leu Asn Arg Glu Gly Gln Gly Ile Ser Phe Thr Glu Phe Ser Tyr Asn
   165 170 175
   Leu Leu Gln Gly Tyr Gly Met Ala Cys Ala Asn Lys Gln Tyr Gly Val
   180 185 190
   Val Leu Gln Ile Gly Gly Ser Asp Gln Trp Gly Asn Ile Thr Ser Gly
   195 200 205
   Ile Asp Leu Thr Arg Arg Leu His Gln Asn Gln Val Phe Gly Leu Thr
   210 215 220
   Val Pro Leu Ile Thr Lys Ala Asp Gly Thr Lys Phe Gly Lys Thr Glu
   225 230 235 240
   Gly Gly Ala Val Trp Leu Asp Pro Lys Lys Thr Ser Pro Tyr Lys Phe
   245 250 255
   Tyr Gln Phe Trp Ile Asn Thr Ala Asp Ala Asp Val Tyr Arg Phe Leu
   260 265 270
   Lys Phe Phe Thr Phe Met Ser Ile Glu Glu Ile Asn Ala Leu Glu Glu
   275 280 285
   Glu Asp Lys Asn Ser Gly Lys Ala Pro Arg Ala Gln Tyr Val Leu Ala
   290 295 300
   Glu Gln Val Thr Arg Leu Val His Gly Glu Glu Gly Leu Gln Ala Ala
   305 310 315 320
   Lys Arg Ile Thr Glu Cys Leu Phe Ser Gly Ser Leu Ser Ala Leu Ser
   325 330 335
   Glu Ala Asp Phe Glu Gln Leu Ala Gln Asp Gly Val Pro Met Val Glu
   340 345 350
   Met Glu Lys Gly Ala Asp Leu Met Gln Ala Leu Val Asp Ser Glu Leu
   355 360 365
   Gln Pro Ser Arg Gly Gln Ala Arg Lys Thr Ile Ala Ser Asn Ala Ile
   370 375 380
   Thr Ile Asn Gly Glu Lys Gln Ser Asp Pro Glu Tyr Phe Phe Lys Glu
   385 390 395 400
   Glu Asp Arg Leu Phe Gly Arg Phe Thr Leu Leu Arg Arg Gly Lys Lys
   405 410 415
   Asn Tyr Cys Leu Ile Cys Trp Lys
   420
<210> 87
   <211> 11
   <212> DNA
   <213> Artificial
<220>
   <223> B box sequence
<220>
   <221> misc-feature
   <222> (8)..(8)
   <223> n is a, c, g, or t
<400> 87
   ggttcgantc c 11
<210> 88
   <211> 95
   <212> DNA
   <213> Artificial
<220>
   <223> B. stearothermophilus tRNA expression insert
<400> 88
   ggattacgca tgctcagtgc aatcttcggt tgcctggact agcgctccgg tttttctgtg 60
   ctgaacctca ggggacgccg acacacgtac acgtc 95
<210> 89
   <211> 42
   <212> DNA
   <213> Artificial
<220>
   <223> B. stearothermophilus tRNA amber suppression mutant
<400> 89
   gacaagtgcg gtttttttct ccagctcccg atgacttatg gc 42
<210> 90
   <211> 80
   <212> DNA
   <213> Artificial
<220>
   <223> FTam 73: forward primer
<400> 90
   gtacgaattc ccgagatctg gattacgcat gctcagtgca atcttcggtt gcctggacta 60
   gcgctccggt ttttctgtgc 80
<210> 91
   <211> 88
   <212> DNA
   <213> Artificial
<220>
   <223> FTam 74: Reverse primer
<400> 91
   agtccgccgc gtttagccac ttcgctaccc ctccgacgtg tacgtgtgtc ggcgtcccct 60
   gaggttcagc acagaaaaac cggagcgc 88
<210> 92
   <211> 75
   <212> DNA
   <213> Artificial
<220>
   <223> FTam 75: Forward primer
<400> 92
   gatgcaagct tgatggatcc gccataagtc atcgggagct ggagaaaaaa accgcacttg 60
   tctggagggg gacgg 75
<210> 93
   <211> 75
   <212> DNA
   <213> Artificial
<220>
   <223> FTam 76: Reverse primer
<400> 93
   gatgcaagct tgatggatcc gccataagtc atcgggagct ggagaaaaaa accgcacttg 60
   tctggagggg gacgg 75
<210> 94
   <211> 790
   <212> DNA
   <213> Artificial
<220>
   <223> hIgG1-Fc2 DNA
<400> 94
   ctgagatcac cggcgaagga gggccaccat gtacaggatg caactcctgt cttgcattgc 60
   actaagtctt gcacttgtca cgaattcgat atcggccatg gttagatctg acaaaactca 120
   cacatgccca ccgtgcccag cacctgaact cctgggggga ccgtcagtct tcctcttccc 180
   cccaaaaccc aaggacaccc tcatgatctc ccggacccct gaggtcacat gcgtggtggt 240
   ggacgtgagc cacgaagacc ctgaggtcaa gttcaactgg tacgtggacg gcgtggaggt 300
   gcataatgcc aagacaaagc cgcgggagga gcagtacaac agcacgtacc gtgtggtcag 360
   cgtcctcacc gtcctgcacc aggactggct gaatggcaag gagtacaagt gcaaggtctc 420
   caacaaagcc ctcccagccc ccatcgagaa aaccatctcc aaagccaaag ggcagccccg 480
   agaaccacag gtgtacaccc tgcccccatc ccgggaggag atgaccaaga accaggtcag 540
   cctgacctgc ctggtcaaag gcttctatcc cagcgacatc gccgtggagt gggagagcaa 600
   tgggcagccg gagaacaact acaagaccac gcctcccgtg ctggactccg acggctcctt 660
   cttcctctac agcaagctca ccgtggacaa gagcaggtgg cagcagggga acgtcttctc 720
   atgctccgtg atgcatgagg gtctgcacaa ccactacacg cagaagagcc tctccctgtc 780
   tccgggtaaa 790
<210> 95
   <211> 41
   <212> DNA
   <213> Artificial
<220>
   <223> 5' IL2 signal sequence
<400> 95
   atgtacagga tgcaactcct gtcttgcatt gcactaagtc t 41
<210> 96
   <211> 254
   <212> PRT
   <213> Artificial
<220>
   <223> hIgG1-Fc2 protein sequence
<400> 96
   Met Tyr Arg Met Gln Leu Leu Ser Cys Ile Ala Leu Ser Leu Ala Leu
   1               5                   10                  15
   Val Thr Asn Ser Ile Ser Ala Met Val Arg Ser Asp Lys Thr His Thr
   20 25 30
   Cys Pro Pro Cys Pro Ala Pro Glu Leu Leu Gly Gly Pro Ser Val Phe
   35 40 45
   Leu Phe Pro Pro Lys Pro Lys Asp Thr Leu Met Ile Ser Arg Thr Pro
   50 55 60
   Glu Val Thr Cys Val Val Val Asp Val Ser His Glu Asp Pro Glu Val
   65 70 75 80
   Lys Phe Asn Trp Tyr Val Asp Gly Val Glu Val His Asn Ala Lys Thr
   85 90 95
   Lys Pro Arg Glu Glu Gln Tyr Asn Ser Thr Tyr Arg Val Val Ser Val
   100 105 110
   Leu Thr Val Leu His Gln Asp Trp Leu Asn Gly Lys Glu Tyr Lys Cys
   115 120 125
   Lys Val Ser Asn Lys Ala Leu Pro Ala Pro Ile Glu Lys Thr Ile Ser
   130 135 140
   Lys Ala Lys Gly Gln Pro Arg Glu Pro Gln Val Tyr Thr Leu Pro Pro
   145 150 155 160
   Ser Arg Glu Glu Met Thr Lys Asn Gln Val Ser Leu Thr Cys Leu Val
   165 170 175
   Lys Gly Phe Tyr Pro Ser Asp Ile Ala Val Glu Trp Glu Ser Asn Gly
   180 185 190
   Gln Pro Glu Asn Asn Tyr Lys Thr Thr Pro Pro Val Leu Asp Ser Asp
   195 200 205
   Gly Ser Phe Phe Leu Tyr Ser Lys Leu Thr Val Asp Lys Ser Arg Trp
   210 215 220
   Gln Gln Gly Asn Val Phe Ser Cys Ser Val Met His Glu Gly Leu His
   225 230 235 240
   Asn His Tyr Thr Gln Lys Ser Leu Ser Leu Ser Pro Gly Lys
   245 250
<210> 97
   <211> 20
   <212> PRT
   <213> Artificial
<220>
   <223> IL2 signal sequence protein
<400> 97
   Met Tyr Arg Met Gln Leu Leu Ser Cys Ile Ala Leu Ser Leu Ala Leu
   1               5                   10                  15
   Val Thr Asn Ser
               20